# EUROPÄISCHE PATENTSCHRIFT

(11) **EP 1 181 280 B1**
(45) Veröffentlichungstag und Bekanntmachung des Hinweises auf die Patenterteilung: **01.12.2004**
(21) Anmeldenummer: 00927048.9
(22) Anmeldetag: 26.04.2000
(51) Int. Cl.: C07D 233/32, C07D 233/42, C07D 239/10, C07D 249/08, A01N 43/50, A01N 43/54, A01N 43/653

(54) **SUBSTITUIERTE BENZOYLKETONE, VERFAHREN ZU IHRER HERSTELLUNG UND IHRE VERWENDUNG ALS HERBIZIDE**
SUBSTITUTED BENZOYL KETONES, METHODS FOR PRODUCING THEM AND THEIR USE AS HERBICIDES
CETONES DE BENZOYLE SUBSTITUEES, LEUR PROCEDE DE PREPARATION ET LEUR UTILISATION COMME HERBICIDES

(30) Priorität: 08.05.1999 DE 19921424
(43) Veröffentlichungstag der Anmeldung: 27.02.2002
(73) Patentinhaber: Bayer CropScience AG, 40789 Monheim (DE)
(72) Erfinder: LEHR, Stefan, D-40764 Langenfeld (DE); SCHALLNER, Otto, D-40789 Monheim (DE); SCHWARZ, Hans-Georg, D-40764 Langenfeld (DE); WROBLOWSKY, Heinz-Jürgen, D-40764 Langenfeld (DE); DREWES, Mark, Wilhelm, D-40764 Langenfeld (DE); FEUCHT, Dieter, D-40789 Monheim (DE); PONTZEN, Rolf, D-42799 Leichlingen (DE); WETCHOLOWSKY, Ingo, CEP-13280-000 Vinhedo, SP (BR); MULLER, Klaus-Helmut, D-40593 Dusseldorf (DE)
(86) Internationale Anmeldenummer: PCT/EP2000/003712
(87) Internationale Veröffentlichungsnummer: WO 2000/068204

(56) Entgegenhaltungen:
- WO-A-95/04716
- WO-A-97/27187
- WO-A-97/28122
- WO-A-97/28136
- WO-A-98/51153
- WO-A-99/03856
- US-A- 5 846 906

## Beschreibung

Die Erfindung betrifft neue substituierte Benzoylketone, Verfahren zu ihrer Herstellung und ihre Verwendung als Herbizide.

Es ist bereits bekannt, dass bestimmte substituierte Benzoylketone herbizide Eigenschaften aufweisen (vgl. EP-A-625505, EP-A-625508, US-A-5804532, US-A-5846906, WO-A-96/26193). Die Wirkung dieser Verbindungen ist jedoch nicht in allen Belangen zufriedenstellend.

Es wurden nun die neuen substituierten Benzoylketone der Formel (1), 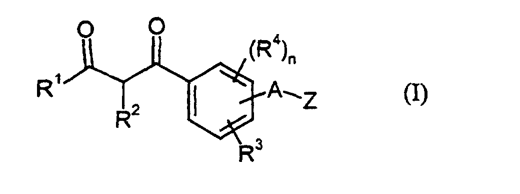
- einschließlich aller möglichen tautomeren Formen der Verbindungen der allgemeinen Formel (I) und der möglichen Salze der Verbindungen der allgemeinen Formel (I) - gefunden.

In den Definitionen sind die Kohlenwasserstoffketten, wie Alkyl oder Alkandiyl - auch in Verbindung mit Heteroatomen, wie in Alkoxy - jeweils geradkettig oder verzweigt.
- n: steht für 0, 1 oder 2.
- A: steht für eine Einfachbindung oder für Alkandiyl (Alkylen) mit 1 bis 4 Kohlenstoffatomen.
- R¹: steht für Wasserstoff, für gegebenenfalls durch Cyano, Carboxy, Carbamoyl, Halogen, C₁-C₄-Alkoxy, C₁-C₄-Alkylthio, C₁-C₄-Alkylsulfinyl oder C₁-C₄-Alkylsulfonyl substituiertes Alkyl mit 1 bis 6 Kohlenstoffatomen, oder für gegebenenfalls durch Cyano, Carboxy, Carbamoyl, Halogen, C₁-C₄-Alkyl oder C₁-C₄-Alkoxy-carbonyl substituiertes Cycloalkyl mit 3 bis 6 Kohlenstoffatomen.
- R²: steht für Wasserstoff, Cyano, Carbamoyl, Halogen, für jeweils gegebenenfalls durch Cyano, Carbamoyl, Halogen oder C₁-C₄-Alkoxy substituiertes Alkyl, Alkoxy oder Alkoxycarbonyl mit jeweils bis zu 6 Kohlenstoffatomen, oder für jeweils gegebenenfalls durch Halogen substituiertes Alkylthio, Alkylsulfinyl oder Alkylsulfonyl mit jeweils 1 bis 6 Kohlenstoffatomen.
- R³: steht für Wasserstoff, Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen.
- R⁴: steht für Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen.
- Z: steht für eine der nachstehenden heterocyclischen Gruppierungen 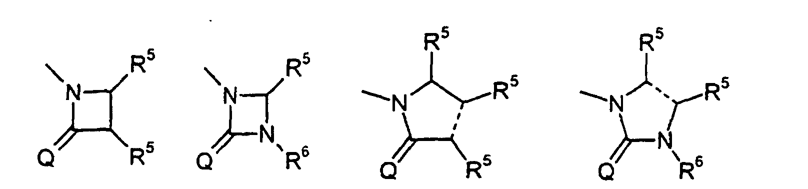 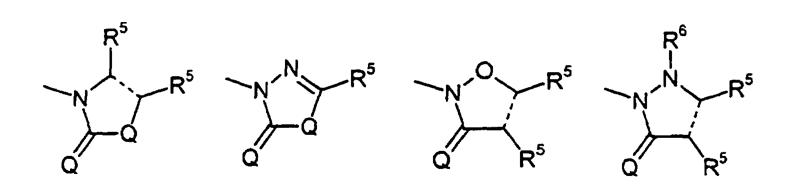 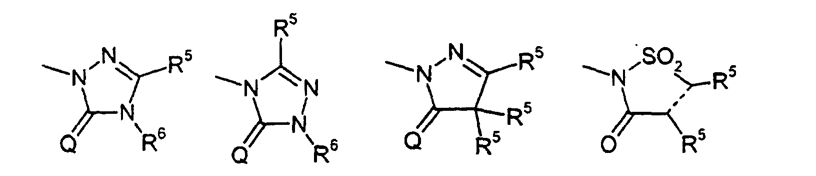 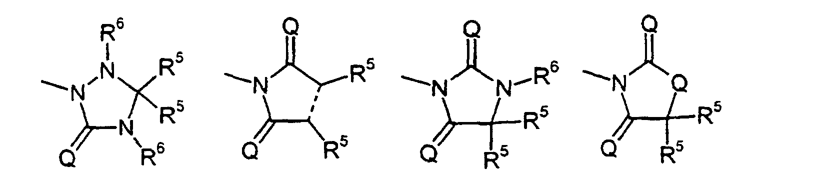 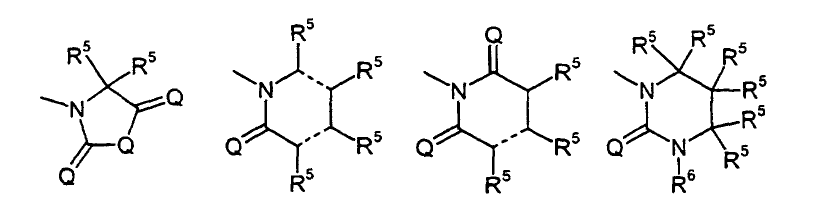 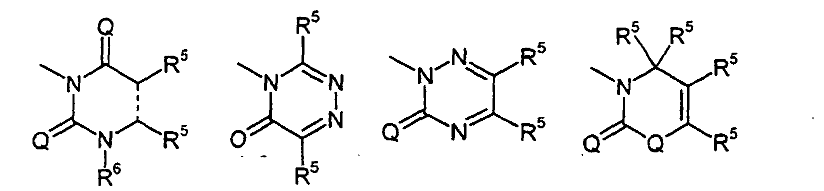 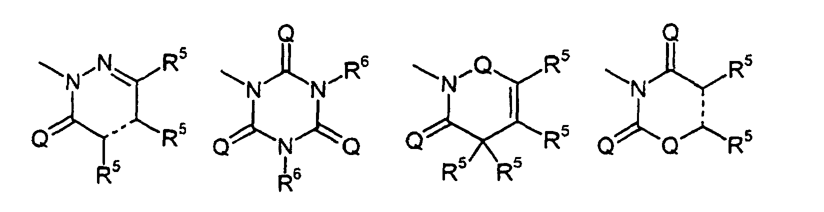 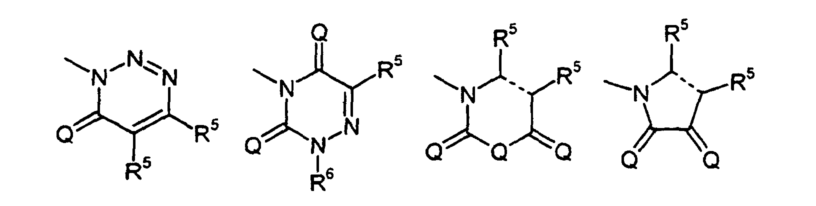 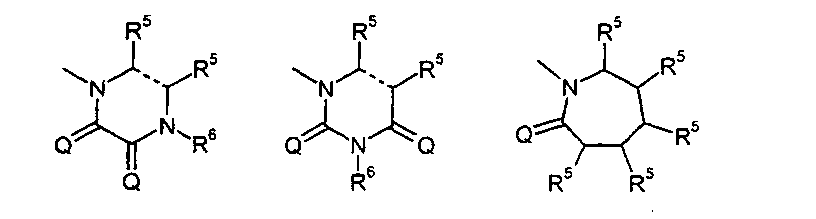 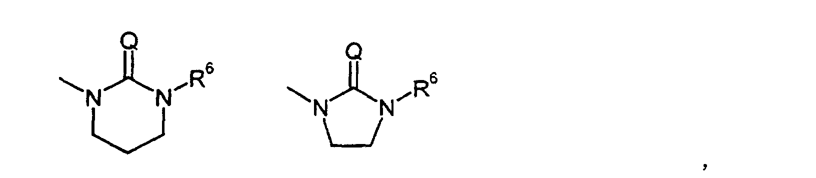 worin jeweils die gestrichelt gezeichnete Bindung eine Einfachbindung oder eine Doppelbindung ist, und jede heterozyclische Gruppierung vorzugsweise nur zwei Substituenten der Definition R⁵ und/oder R⁶ trägt,
- Q: für Sauerstoff oder Schwefel steht,
- R⁵: für Wasserstoff, Hydroxy, Mercapto, Cyano, Halogen, für jeweils gegebenenfalls durch Cyano, Halogen, C₁-C₄-Alkoxy, C₁-C₄-Alkylthio, C₁-C₄-Alkylsulfinyl oder C₁-C₄-Alkylsulfonyl substituiertes Alkyl, Alkylcarbonyl, Alkoxy, Alkoxycarbonyl, Alkylthio, Alkylsulfinyl oder Alkylsulfonyl mit jeweils bis zu 6 Kohlenstoffatomen in den Alkylgruppen, für jeweils gegebenenfalls durch Halogen substituiertes Alkylamino oder Dialkylamino mit jeweils bis zu 6 Kohlenstoffatomen in den Alkylgruppen, für jeweils gegebenenfalls durch Halogen substituiertes Alkenyl, Alkinyl, Alkenyloxy, Alkenylthio oder Alkenylamino mit jeweils bis zu 6 Kohlenstoffatomen in den Alkenyl- bzw. Alkinylgruppen, für jeweils gegebenenfalls durch Halogen substituiertes Cycloalkyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in den Cycloalkylgruppen und gegebenenfalls bis zu 4 Kohlenstoffatomen im Alkylteil, oder für jeweils gegebenenfalls durch Halogen, C₁-C₄-Alkyl oder C₁-C₄-Alkoxy substituiertes Phenyl, Phenyloxy, Phenylthio, Phenylamino, Benzyl, Benzyloxy, Benzylthio oder Benzylamino steht, oder - für den Fall, dass zwei benachbarte Reste R⁵ und R⁵ sich an einer Doppelbindung befinden - zusammen mit dem benachbarten Rest R⁵ auch für eine Benzogruppierung steht, und
- R⁶: für Wasserstoff, Hydroxy, Amino, Alkylidenamino mit bis zu 4 Kohlenstoffatomen, für jeweils gegebenenfalls durch Halogen oder C₁-C₄-Alkoxy substituiertes Alkyl, Alkoxy, Alkylamino, Dialkylamino oder Alkanoylamino mit jeweils bis zu 6 Kohlenstoffatomen in den Alkylgruppen, für jeweils gegebenenfalls durch Halogen substituiertes Alkenyl, Alkinyl oder Alkenyloxy mit jeweils bis zu 6 Kohlenstoffatomen in den Alkenyl- bzw. Alkinylgruppen, für jeweils gegebenenfalls durch Halogen substituiertes Cycloalkyl, Cycloalkylalkyl oder Cycloalkylamino mit jeweils 3 bis 6 Kohlenstoffatomen in den Cycloalkylgruppen und gegebenenfalls bis zu 3 Kohlenstoffatomen im Alkylteil, oder für jeweils gegebenenfalls durch Halogen, C₁-C₄-Alkyl oder C₁-C₄-Alkoxy substituiertes Phenyl oder Benzyl steht, oder zusammen mit einem benachbarten Rest R⁵ oder R⁶ für gegebenenfalls durch Halogen oder C₁-C₄-Alkyl substituiertes Alkandiyl mit 3 bis 5 Kohlenstoffatomen steht,
wobei die einzelnen Reste R⁵ und R⁶ - soweit mehrere davon an gleiche heterocyclische Gruppierungen gebunden sind, gleiche oder verschiedene Bedeutungen im Rahmen der obigen Definition haben können.
- Q: steht für Sauerstoff.
- R⁵: steht für Wasserstoff, Hydroxy, Mercapto, Cyano, Fluor, Chlor, Brom, Iod, für jeweils gegebenenfalls durch Fluor, Chlor, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n-oder i-Propylthio, n-, i-, s- oder t-Butylthio, Methylsulfinyl, Ethylsulfinyl, n-oder i-Propylsulfinyl, Methylsulfonyl, Ethylsulfonyl, n- oder i-Propylsulfonyl, für Methylamino, Ethylamino, n- oder i-Propylamino, n-, i-, s- oder t-Butylamino, Dimethylamino, Diethylamino, Di-n-propylamino oder Di-i-propylamino, für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, Butenylthio, Propenylamino oder Butenylamino, für jeweils gegebenenfalls durch Fluor und/oder Chlor substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Ethyl, n- oder i-Propyl, n-, i-, s- oder t-Butyl, Methoxy, Ethoxy, n-oder i-Propoxy substituiertes Phenyl, Phenyloxy, Phenylthio, Phenylamino, Benzyl, Benzyloxy, Benzylthio oder Benzylamino, oder - für den Fall, dass zwei benachbarte Reste R⁵ und R⁵ sich an einer Doppelbindung befinden - zusammen mit dem benachbarten Rest R⁵ auch für eine Benzogruppierung.
- R⁶: steht für Wasserstoff, Hydroxy, Amino, für jeweils gegebenenfalls durch Fluor und/oder Chlor, Methoxy oder Ethoxy substituiertes Methyl, Ethyl, n- oder i-Propyl, n-, i- oder s-Butyl, Methoxy, Ethoxy, n- oder i-Propoxy, Methylamino, Ethylamino oder Dimethylamino, für jeweils gegebenenfalls durch Fluor und/oder Chlor substituiertes Ethenyl, Propenyl, Ethinyl, Propinyl oder Propenyloxy, für jeweils gegebenenfalls durch Fluor und/oder Chlor substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Methyl, Ethyl, n- oder i-Propyl, n-, i-, s- oder t-Butyl, Methoxy, Ethoxy, n-oder i-Propoxy substituiertes Phenyl oder Benzyl, oder zusammen mit einem benachbarten Rest R⁵ oder R⁶ für jeweils gegebenenfalls durch Methyl und/oder Ethyl substituiertes Propan-1,3-diyl (Trimethylen), Butan-1,4-diyl (Tetramethylen) oder Pentan-1,5-diyl (Pentamethylen).
- n: steht bevorzugt für 0 oder 1.
- A: steht bevorzugt für Methylen, Ethyliden (Ethan-1,1-diyl) oder Dimethylen (Ethan-1,2-diyl).
- R¹: steht bevorzugt für Wasserstoff, für jeweils gegebenenfalls durch Cyano, Carboxy, Carbamoyl, Fluor, Chlor, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, oder für jeweils gegebenenfalls durch Cyano, Carboxy, Carbamoyl, Fluor, Chlor, Methyl, Ethyl, Methoxycarbonyl oder Ethoxycarbonyl substituiertes Cyclopropyl, Cyclobutyl, Cyclopentyl oder Cyclohexyl.
- R²: steht bevorzugt für Wasserstoff, Cyano, Carbamoyl, Fluor, Chlor, Brom, für jeweils gegebenenfalls durch Cyano, Carbamoyl, Fluor, Chlor, Methoxy oder Ethoxy substituiertes Methyl, Ethyl, n- oder i-Propyl, Methoxy, Ethoxy, n- oder i-Propoxy, Methoxycarbonyl, Ethoxycarbonyl, n-oder i-Propoxycarbonyl, oder für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl.
- R³: steht bevorzugt für Wasserstoff, Nitro, Cyano, Carboxy, Carbamoyl, Thiocarbamoyl, Fluor, Chlor, Brom, Iod, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl.
- R⁴: steht bevorzugt für Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl.
- R⁵: steht bevorzugt für Wasserstoff, Hydroxy, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlonnethyl, Fluorethyl, Chlorethyl, Difluorethyl, Dichlorethyl, Fluor-n-propyl, Fluor-i-propyl, Chlor-n-propyl, Chlor-i-propyl, Methoxymethyl, Ethoxymethyl, Methoxyethyl, Ethoxyethyl, Methoxy, Ethoxy, n- oder i-Propoxy, n-, i-, s-oder t-Butoxy, Fluorethoxy, Chlorethoxy, Difluorethoxy, Dichlorethoxy, Trifluorethoxy, Trichlorethoxy, Chlorfluorethoxy, Chlordifluorethoxy, Fluordichlorethoxy, Methylthio, Ethylthio, n- oder i-Propylthio, Fluorethylthio, Chlorethylthio, Difluorethylthio, Dichlorethylthio, Chlorfluorethylthio, Chlordifluorethylthio, Fluordichlorethylthio, Methylsulfinyl, Ethylsulfinyl, n-oder i-Propylsulfinyl, Methylsulfonyl, Ethylsulfonyl, n- oder i-Propylsulfonyl, Methylamino, Dimethylamino, Propenylthio, Butenylthio, Propinylthio, Butinylthio, Cyclopropyl, Cyclopropylmethyl, Cyclopropylmethoxy, Phenyl oder Phenoxy.
- R⁶: steht bevorzugt für Amino, Methyl, Ethyl, n- oder i-Propyl, n-, i-, s- oder t-Butyl, Methoxy, Ethoxy, Methylamino, Dimethylamino, Cyclopropyl oder Cyclopropylmethyl, oder zusammen mit R⁵ für Propan-1,3-diyl (Trimethylen), Butan-1,4-diyl (Tetramethylen) oder Pentan-1,5-diyl (Pentamethylen).
- Z: steht bevorzugt für die nachstehenden Gruppierungen 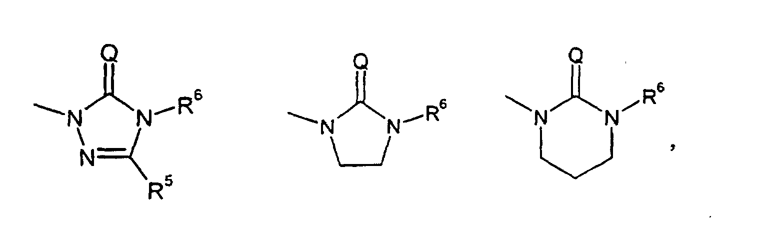 wobei Q, R⁵, und R⁶ die vorausgehend angegebene Bedeutung haben.
A steht besonders bevorzugt für Methylen.
R¹ steht besonders bevorzugt für jeweils gegebenenfalls durch Cyano, Fluor, Chlor, Methoxy, Ethoxy, n- oder i-Propoxy, Methylthio, Ethylthio, n-oder o-Propylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, oder für jeweils gegebenenfalls durch Cyano, Fluor, Chor, Methyl oder Ethyl substituiertes Cyclopropyl, Cyclobutyl, Cyclopentyl oder Cyclohexyl.
R² steht besonders bevorzugt für Wasserstoff, Cyano, Carbamoyl, Fluor, Chlor, Brom, für jeweils gegebenenfalls durch Cyano, Carbamoyl, Fluor, Chlor, Methoxy oder Ethoxy substituiertes Methyl, Ethyl, n- oder i-Propyl, oder für jeweils gegebenenfalls durch Fluor und/oder Chlor substituiertes Methylthio, Ethylthio, n- oder i-Propylthio, Methylsulfinyl, Ethylsulfinyl, n-oder i-Propylsulfinyl, Methylsulfonyl, Ethylsulfonyl, n- oder i-Propylsulfonyl.
R3 steht besonders bevorzugt für Wasserstoff, Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl.
R⁴ steht besonders bevorzugt für Methylsulfonyl, Chlor, Methoxy, Nitro, Trifluormethyl oder Methyl.
R⁵ steht besonders bevorzugt für Wasserstoff, Brom, Chlor, Methyl, Ethyl, Trifluormethyl, Cyclopropyl, Difluorethyl, Methylthio, Ethylthio, Methoxy, Ethoxy, n- oder i-Propoxy, Trifluorethoxy, Methylamino oder Dimethylamino.
R⁶ steht besonders bevorzugt für Wasserstoff, Amino, Methyl, Ethyl, Cyclopropyl, Dimethylamino, Methoxy oder Ethoxy steht.
R¹ steht am meisten bevorzugt für Cyclopropyl.
R² steht am meisten bevorzugt für Wasserstoff oder Cyano.
R3 steht am meisten bevorzugt für Wasserstoff Nitro, Cyano, Fluor, Chlor, Brom, Methyl, Trifluormethyl oder Methoxy.
R⁵ steht am meisten bevorzugt für Brom, Methyl, Ethyl, Methoxy, Methylthio, Ethoxy, Methylsulfonyl oder Dimethylamino.
R6 steht am meisten bevorzugt für Amino, Methyl, Ethyl, Cyclopropyl, Dimethylamino, Methoxy oder Ethoxy.

Erfindungsgemäß bevorzugt sind die Verbindungen der Formel (I), in welchen eine Kombination der vorstehend als bevorzugt aufgeführten Bedeutungen vorliegt.

Erfindungsgemäß besonders bevorzugt sind die Verbindungen der Formel (I), in welchen eine Kombination der vorstehend als besonders bevorzugt aufgeführten Bedeutungen vorliegt.

Erfindungsgemäß ganz besonders bevorzugt sind die Verbindungen der Formel (I), in welchen eine Kombination der vorstehend als ganz besonders bevorzugt aufgeführten Bedeutungen vorliegt.

Erfindungsgemäß am meisten bevorzugt sind die Verbindungen der Formel (I), in welchen eine Kombination der vorstehend als am meisten bevorzugt aufgeführten Bedeutungen vorliegt.

Die Verbindungen der Formel (IA), (IB) und (IC) sind insbesondere Gegenstand der vorliegenden Erfindung: 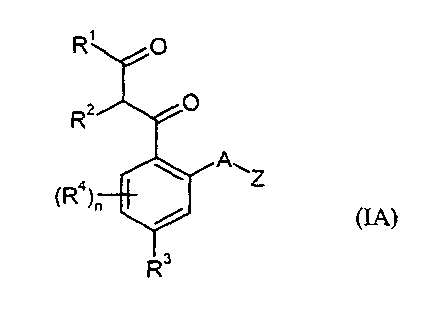 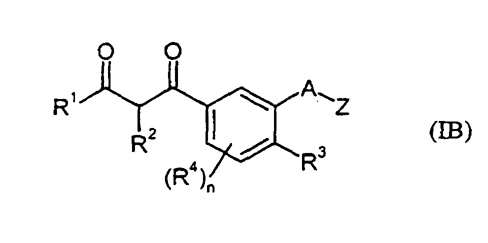 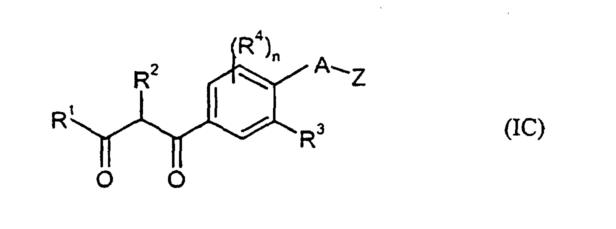 in welchen
Z für die nachstehenden Gruppierungen steht 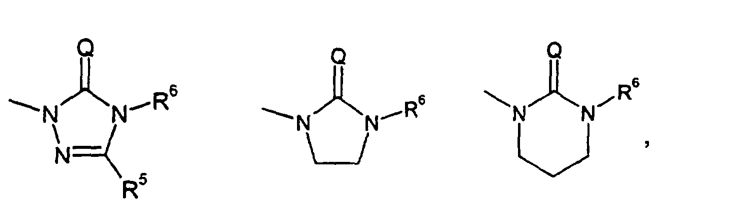 sowie n, A, Q, R¹, R², R³, R⁴, R⁵ und R⁶ die vorausgehend angegebenen Bedeutungen haben.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der allgemeinen Formel (I) sind in den nachstehenden Gruppen aufgeführt. 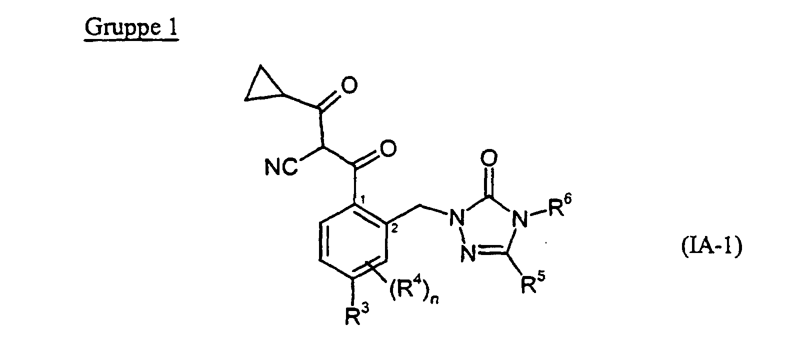 R³, (R⁴)ₙ, R⁵ und R⁶ haben hierbei beispielhaft die in der nachstehenden Tabelle angegebenen Bedeutungen:

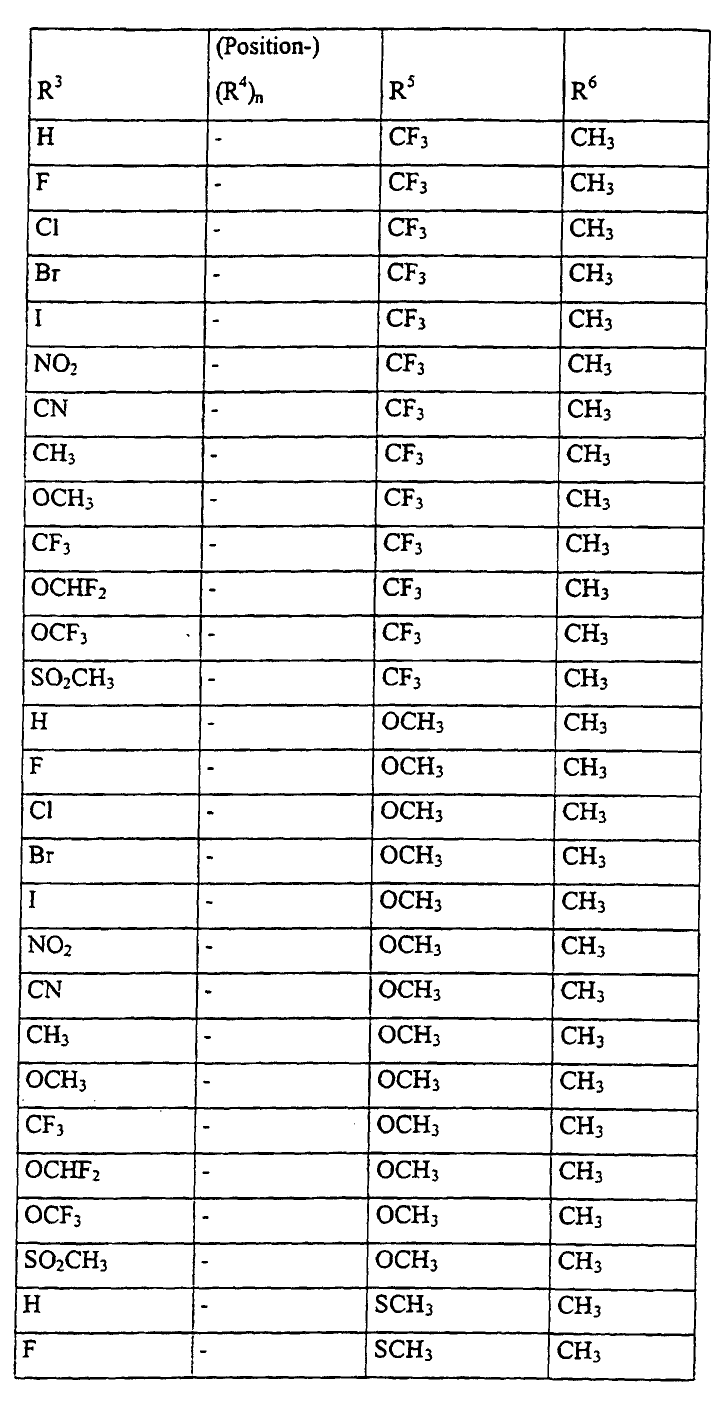

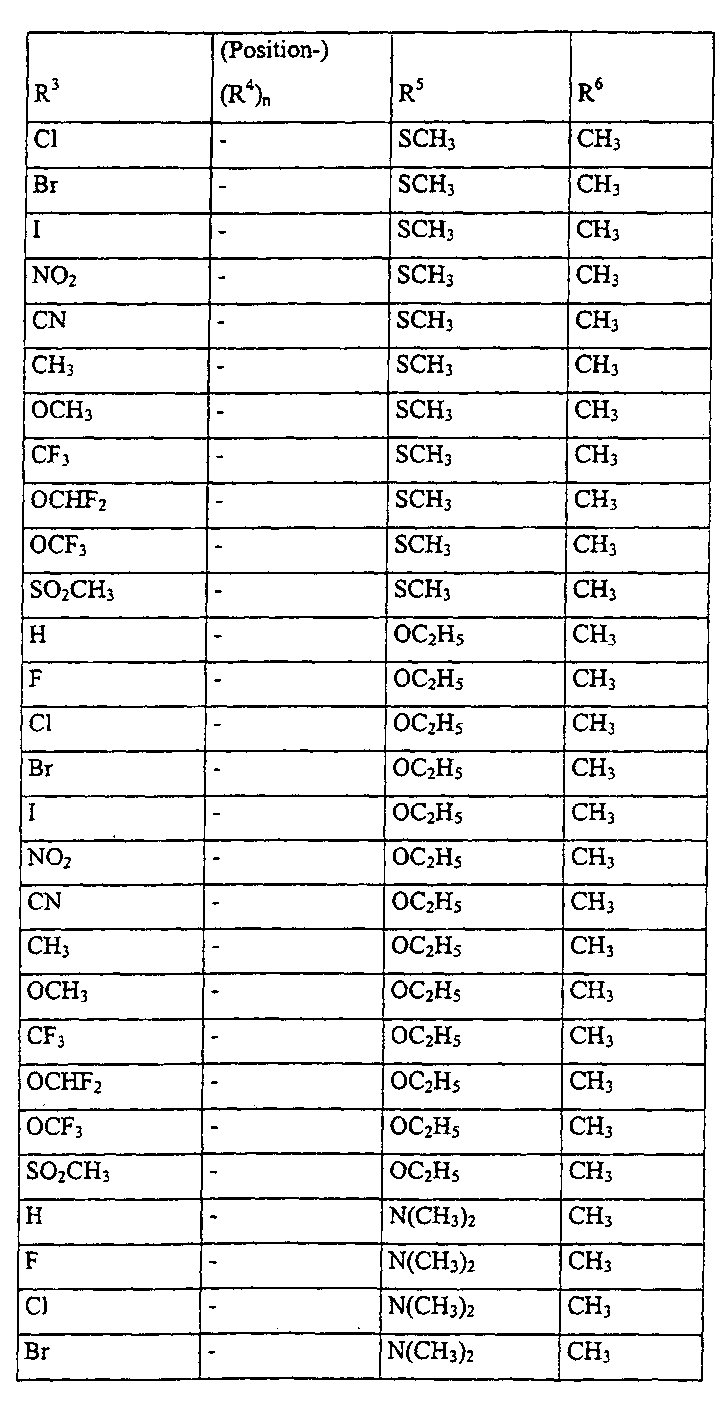

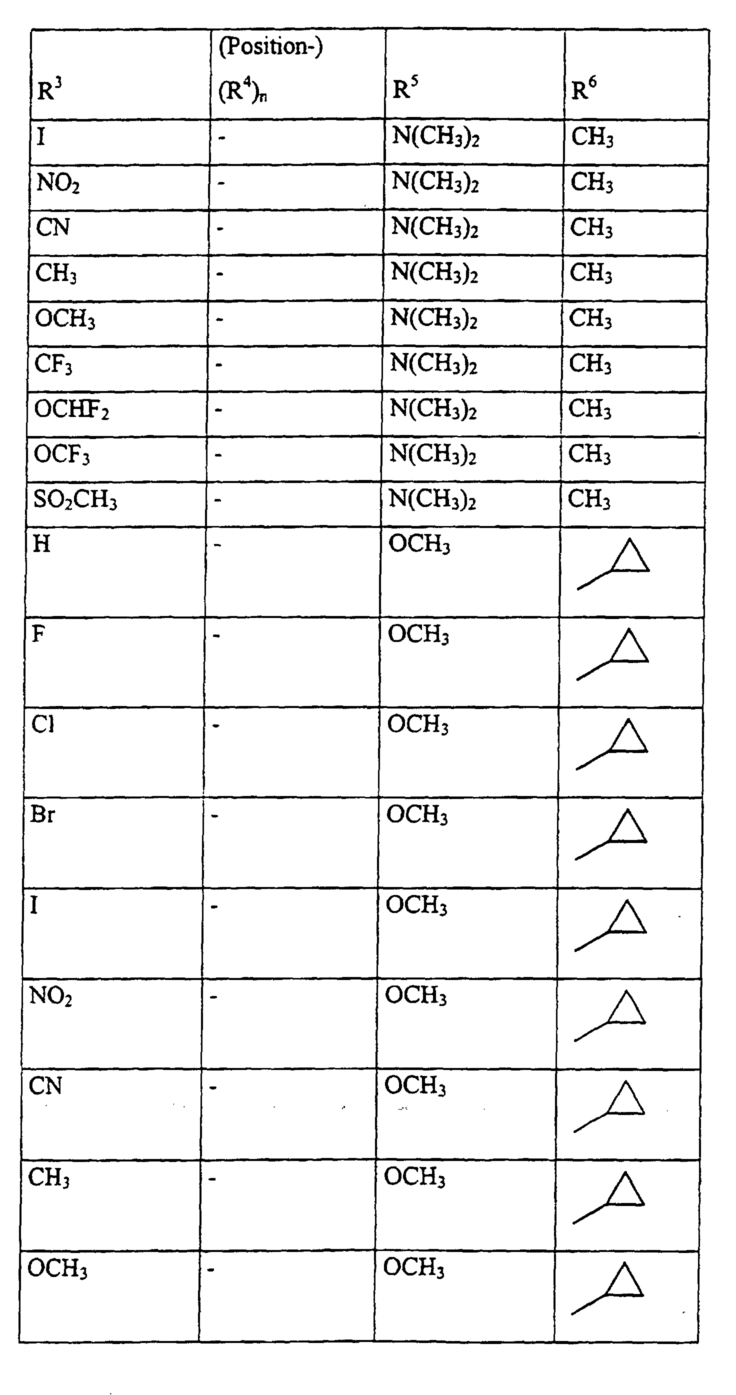

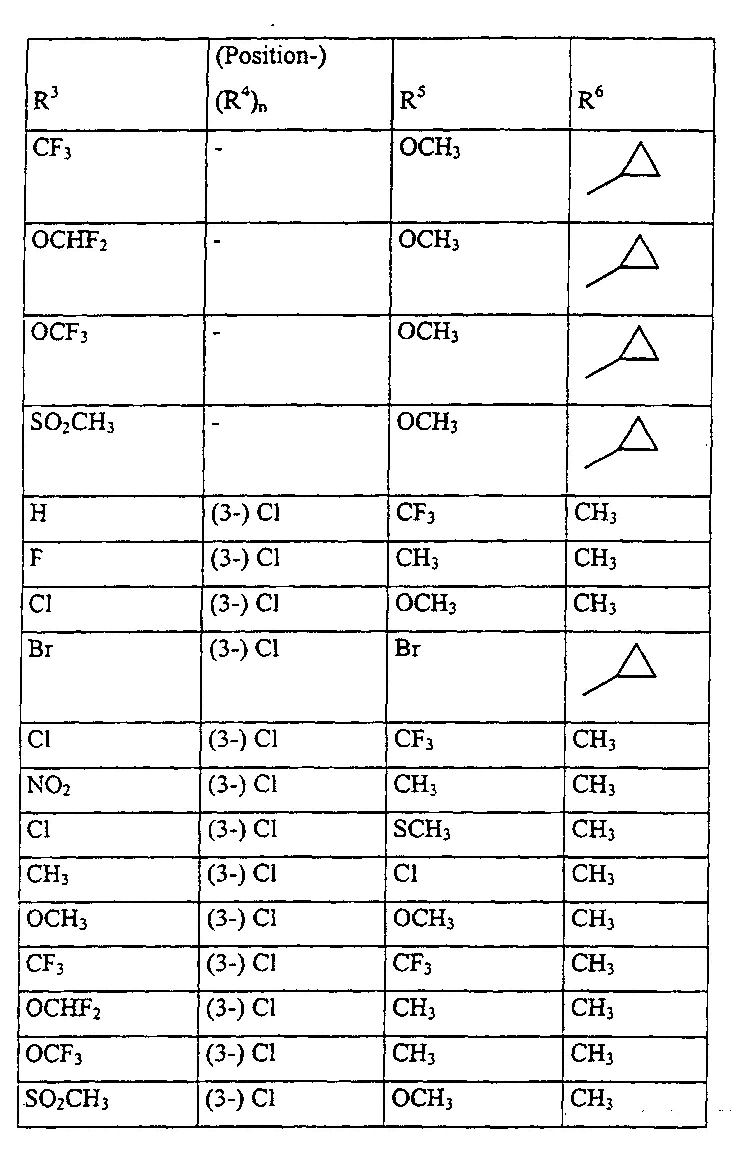

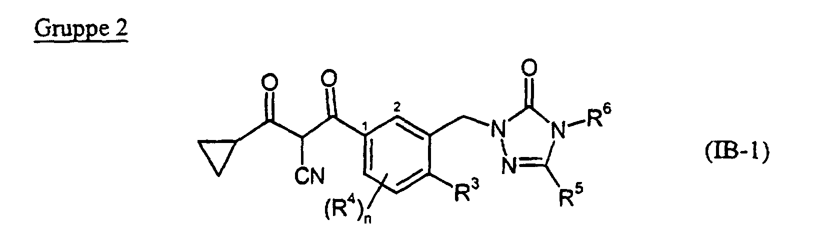

R³, (R⁴)ₙ, R⁵ und R⁶ haben hierbei beispielhaft die in der nachstehenden Tabelle angegebenen Bedeutungen:

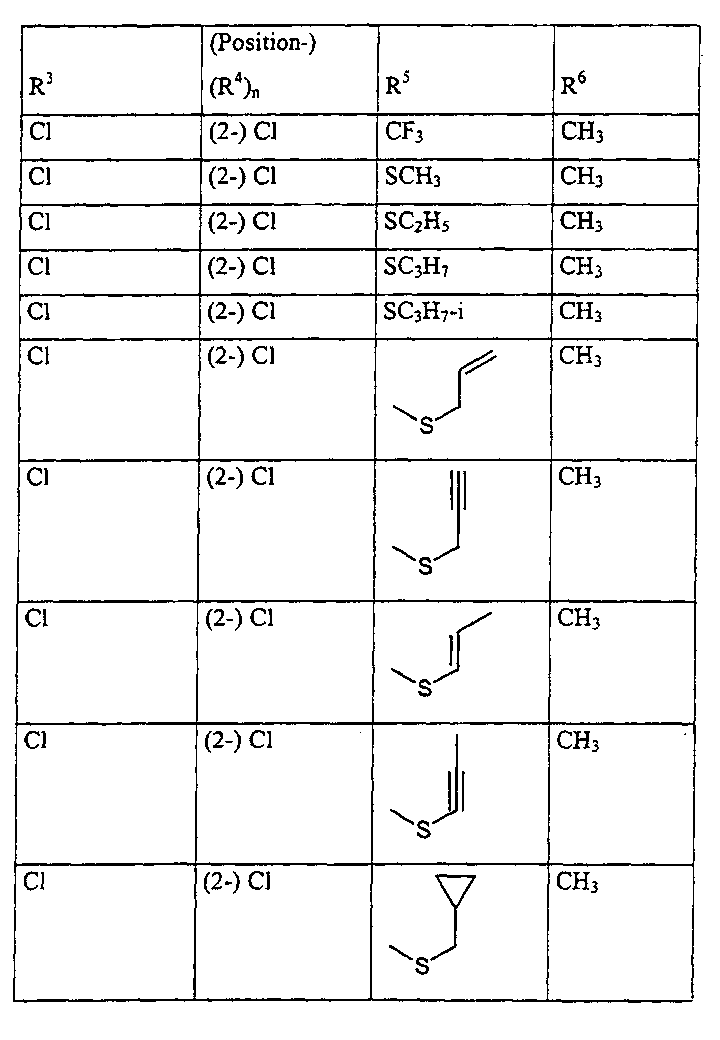

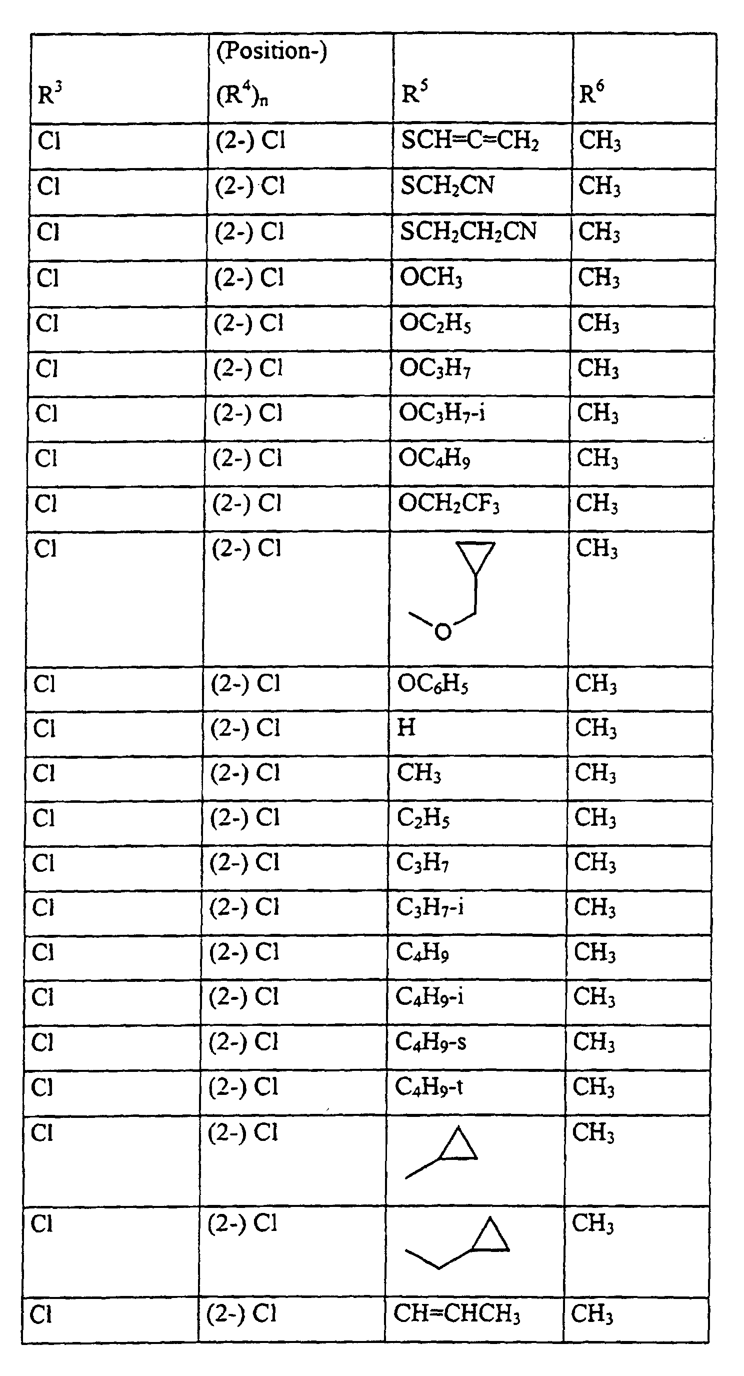

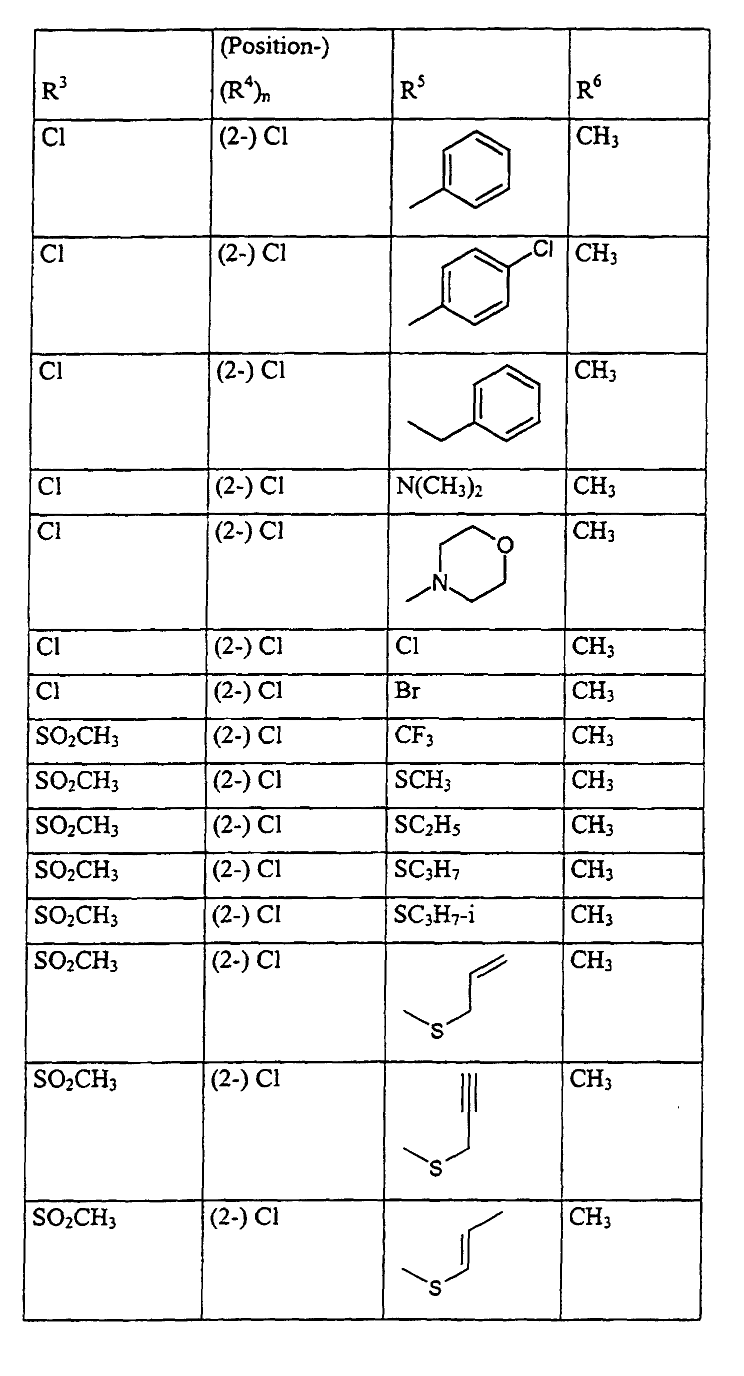

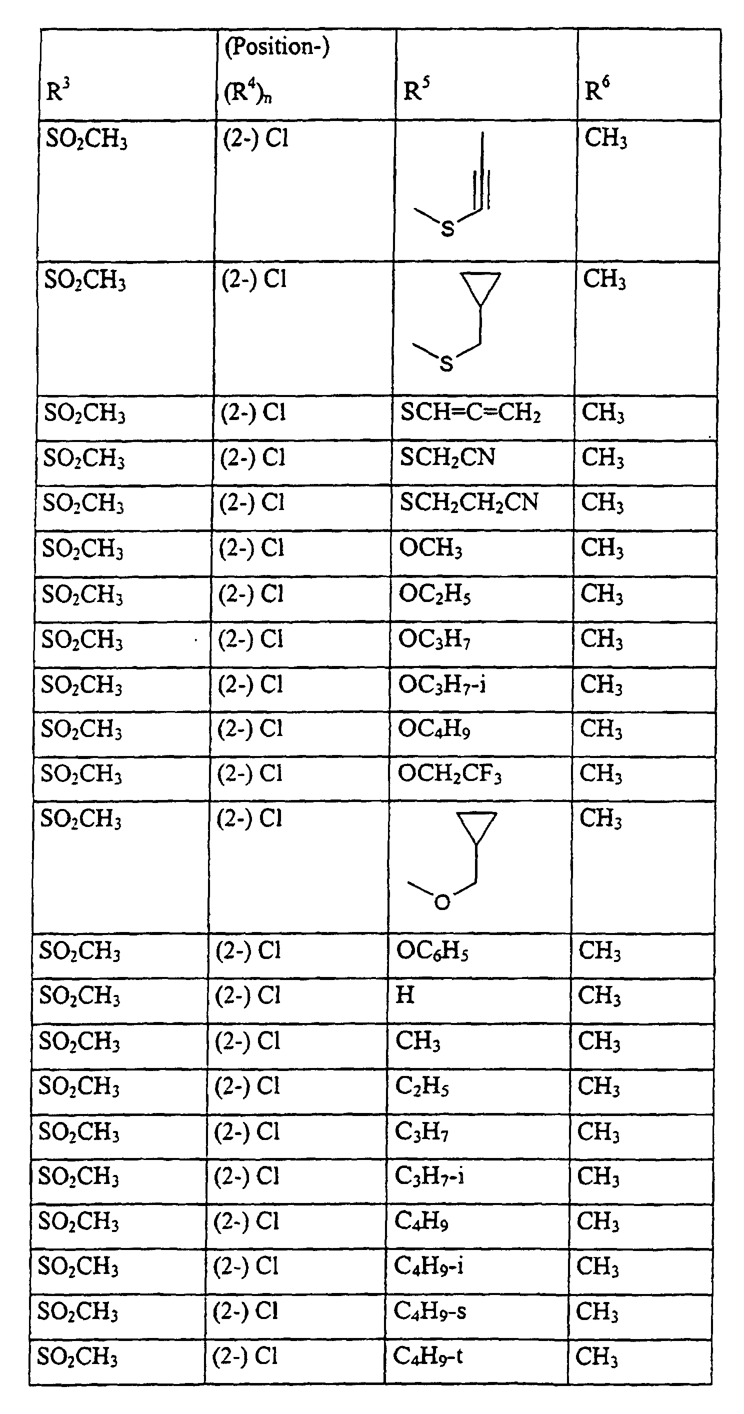

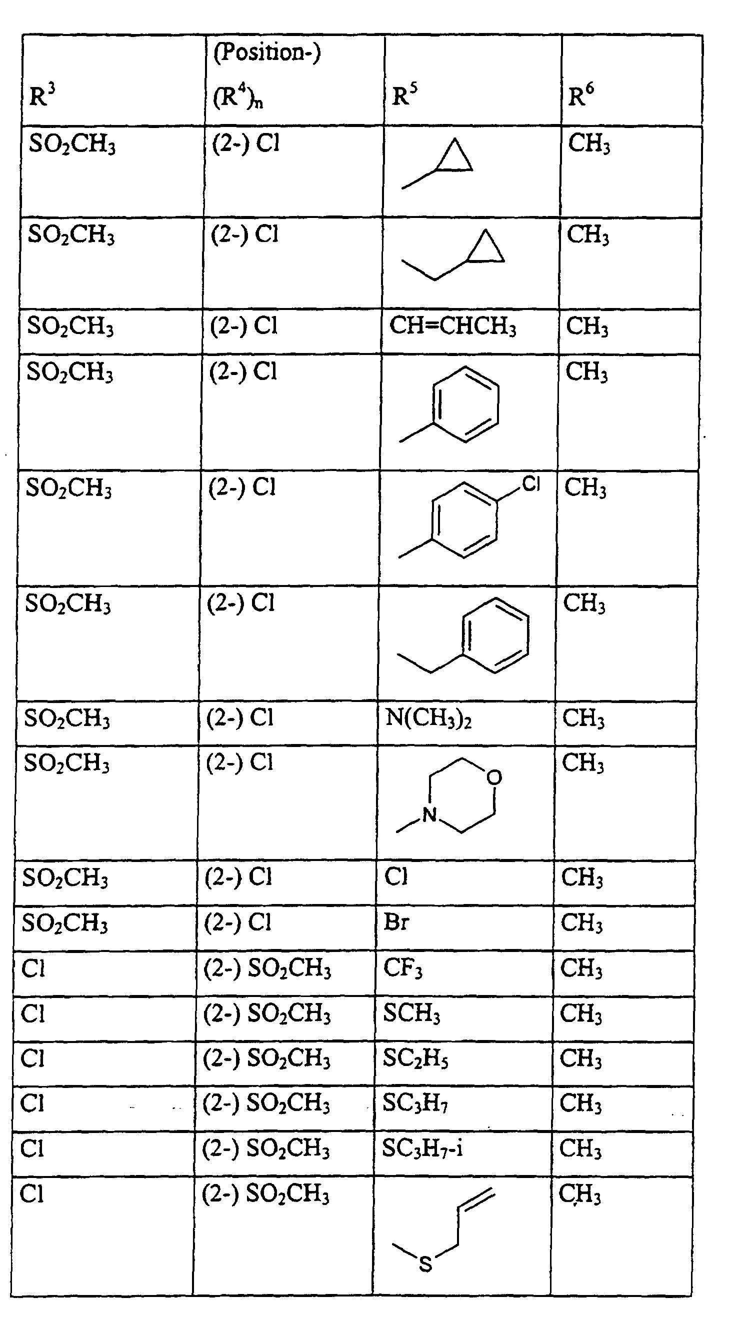

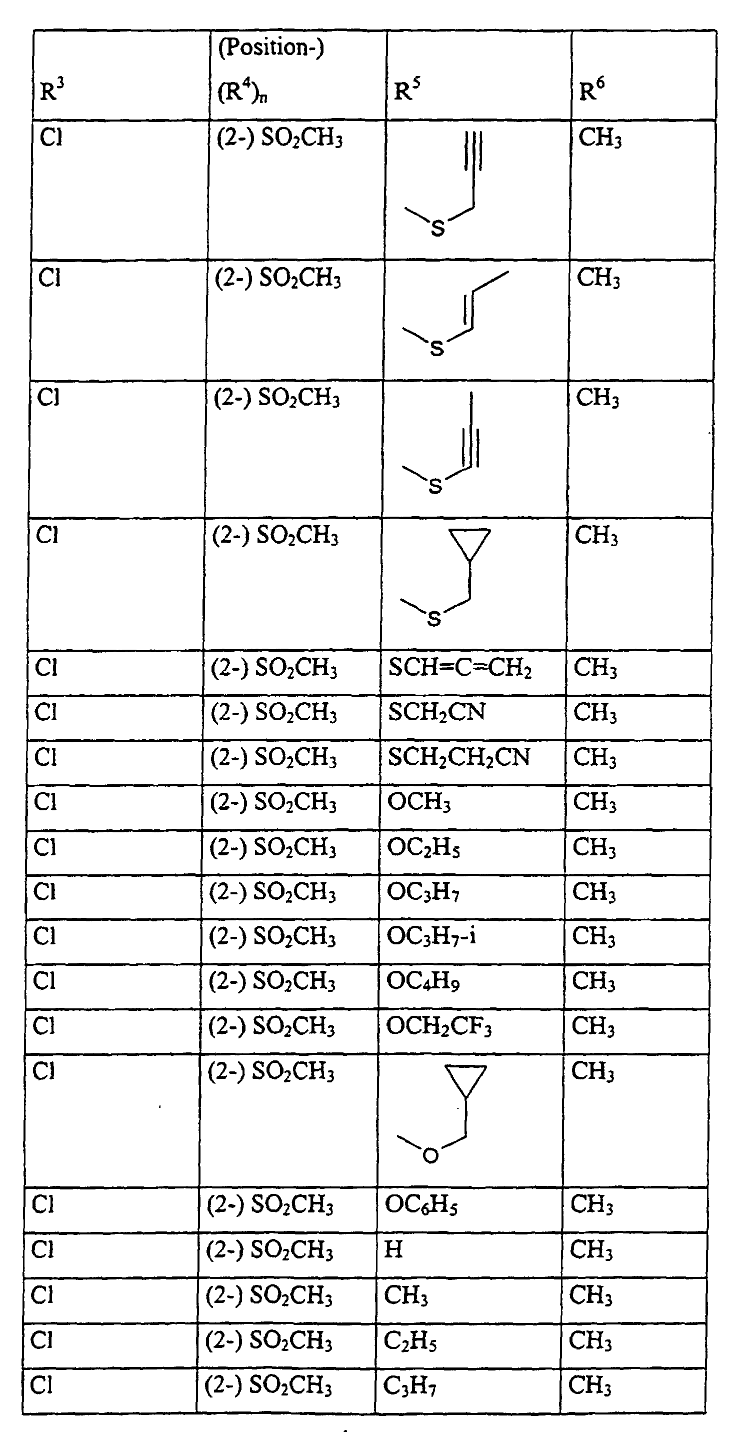

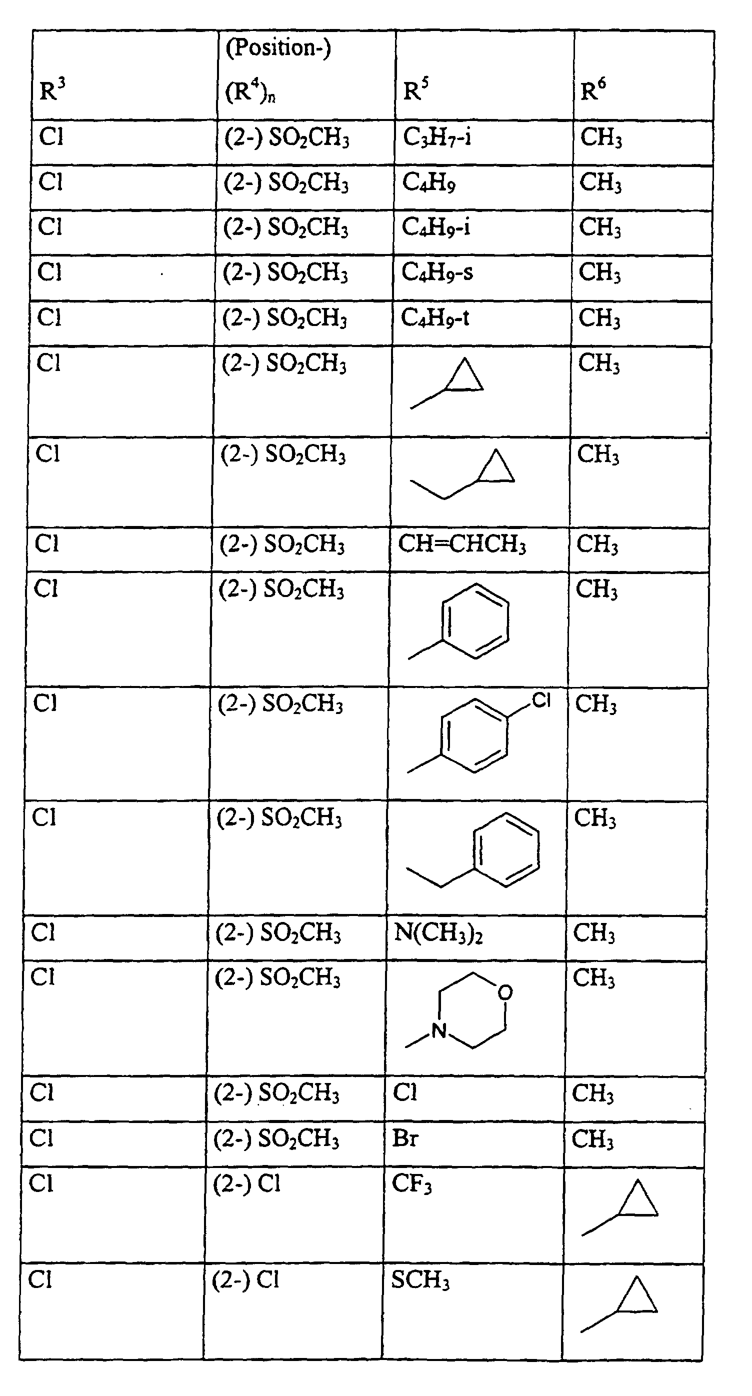

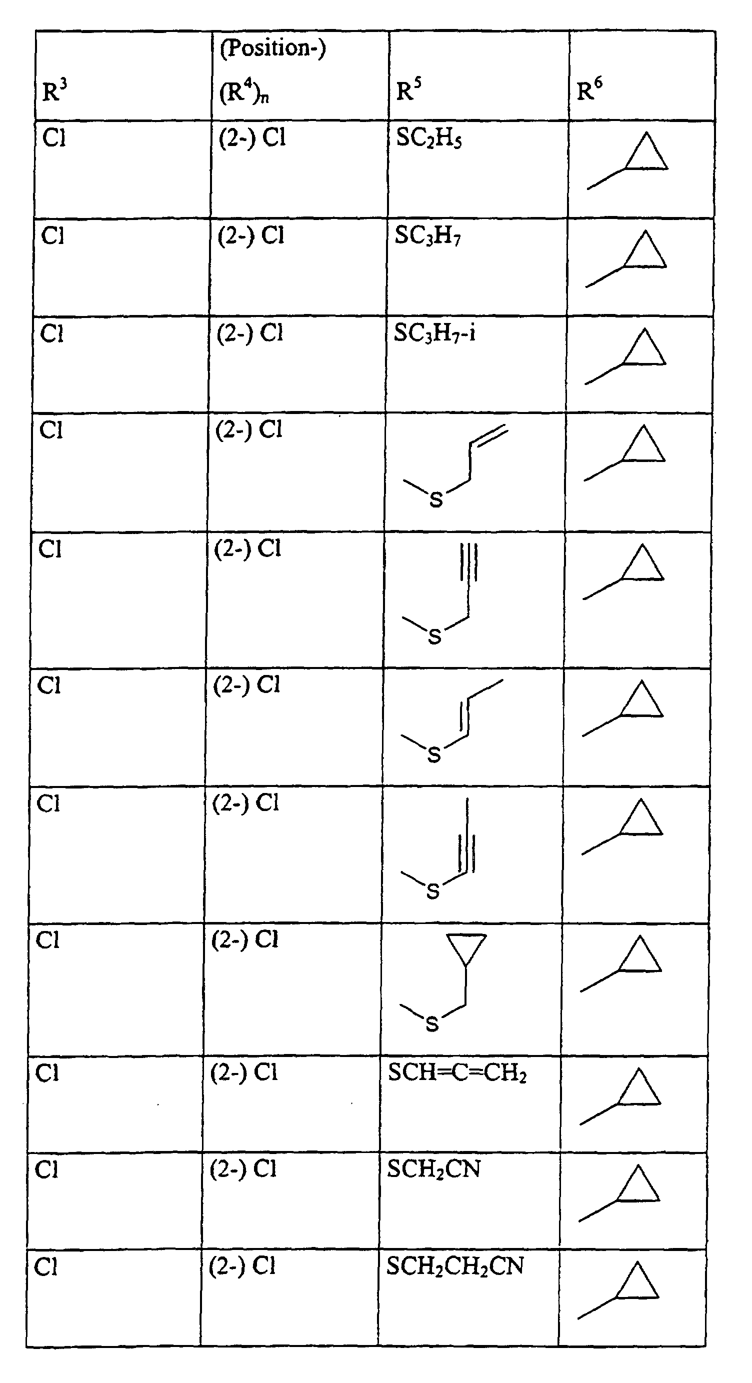

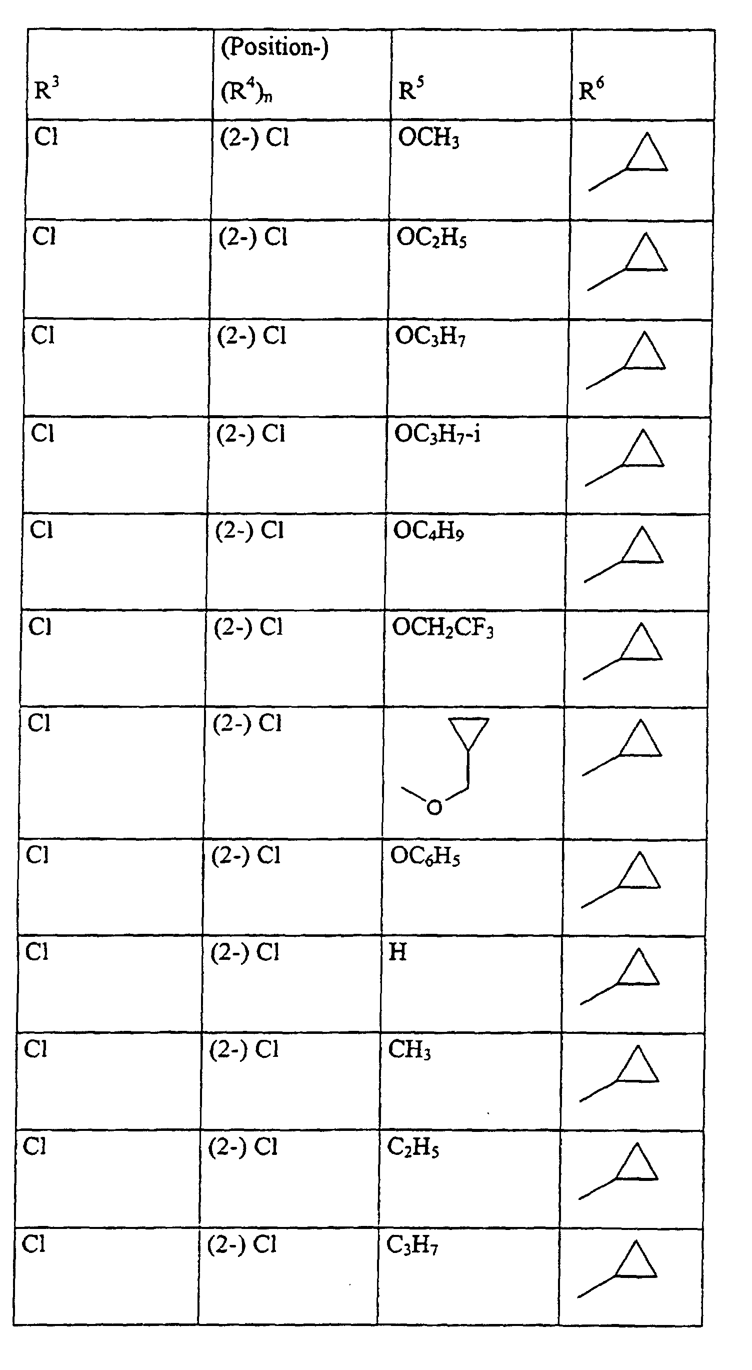

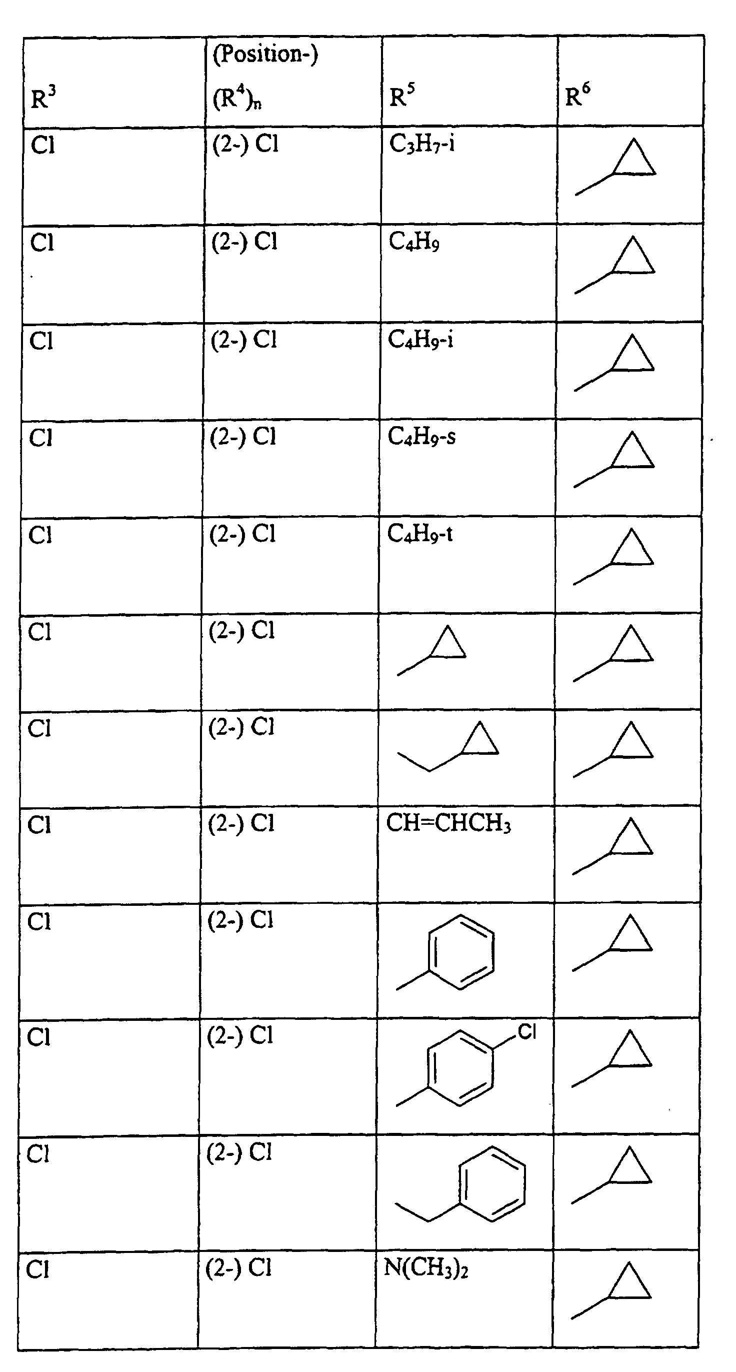

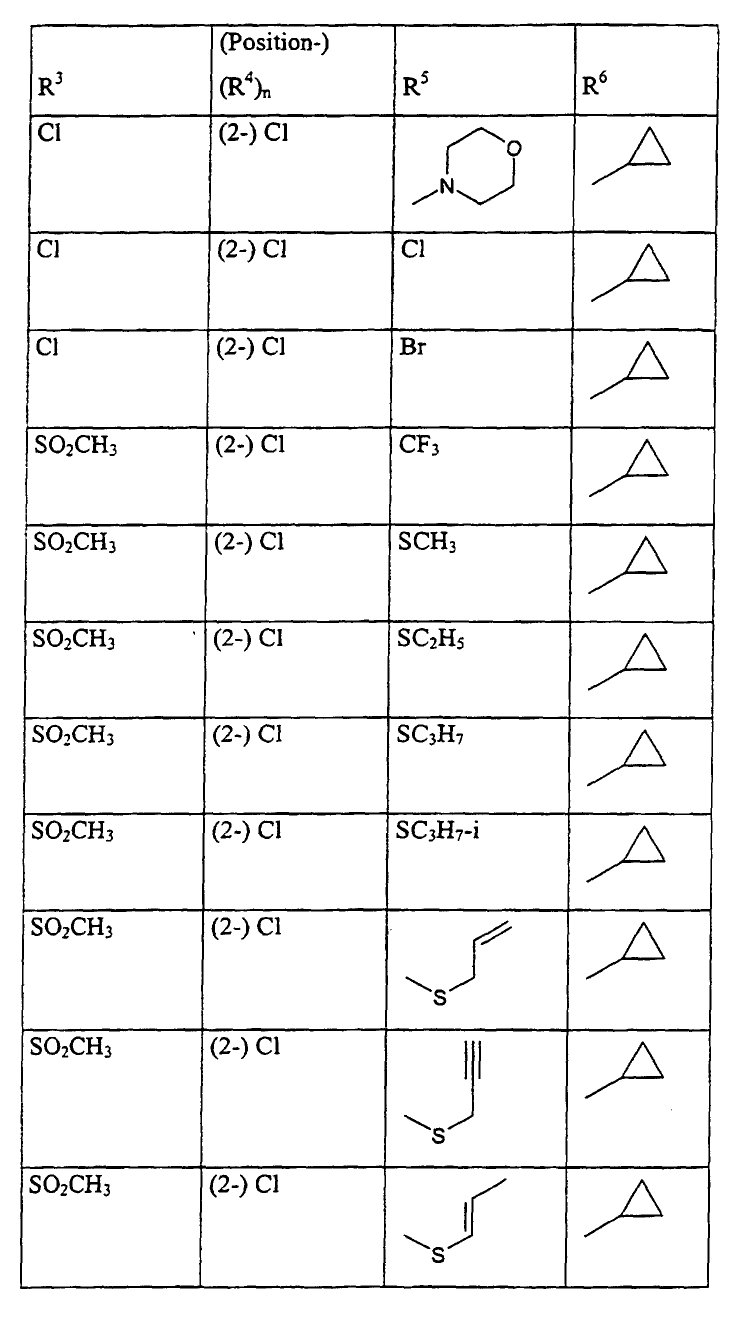

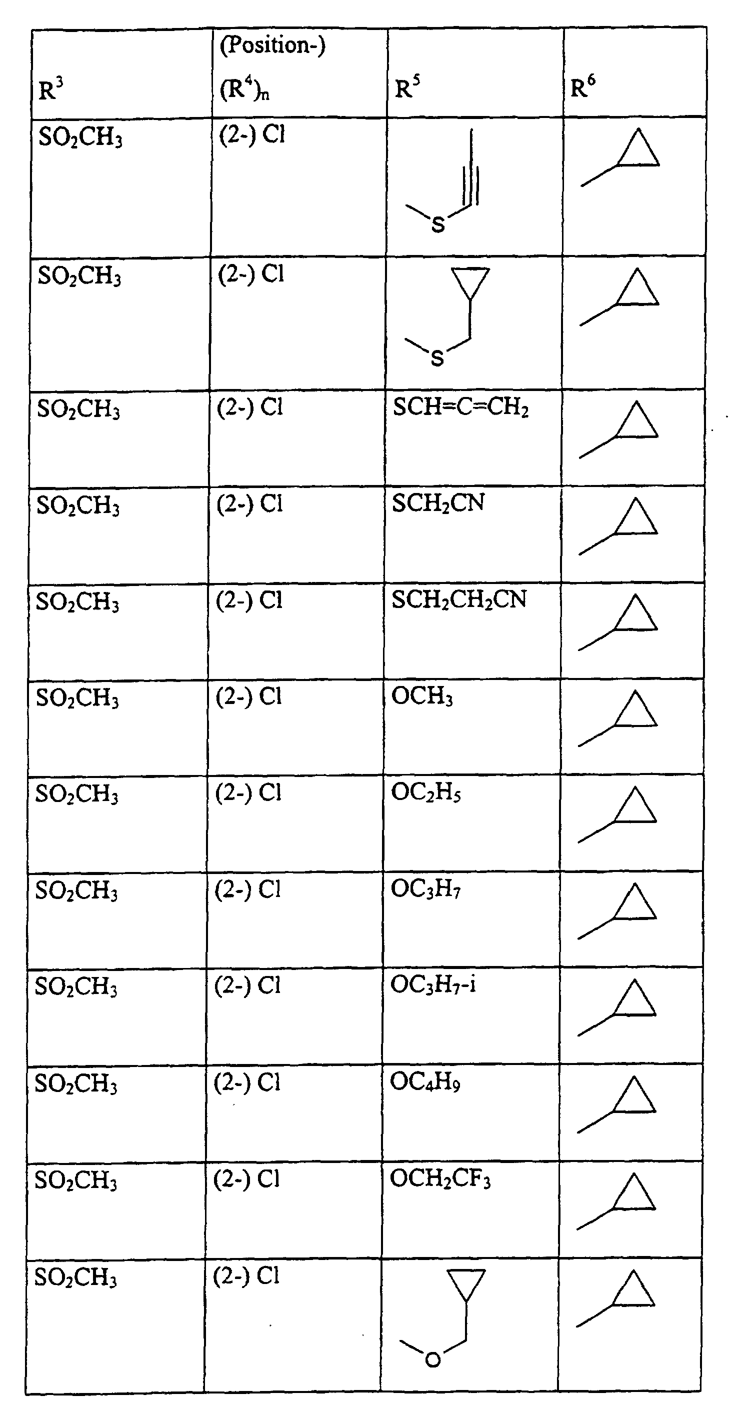

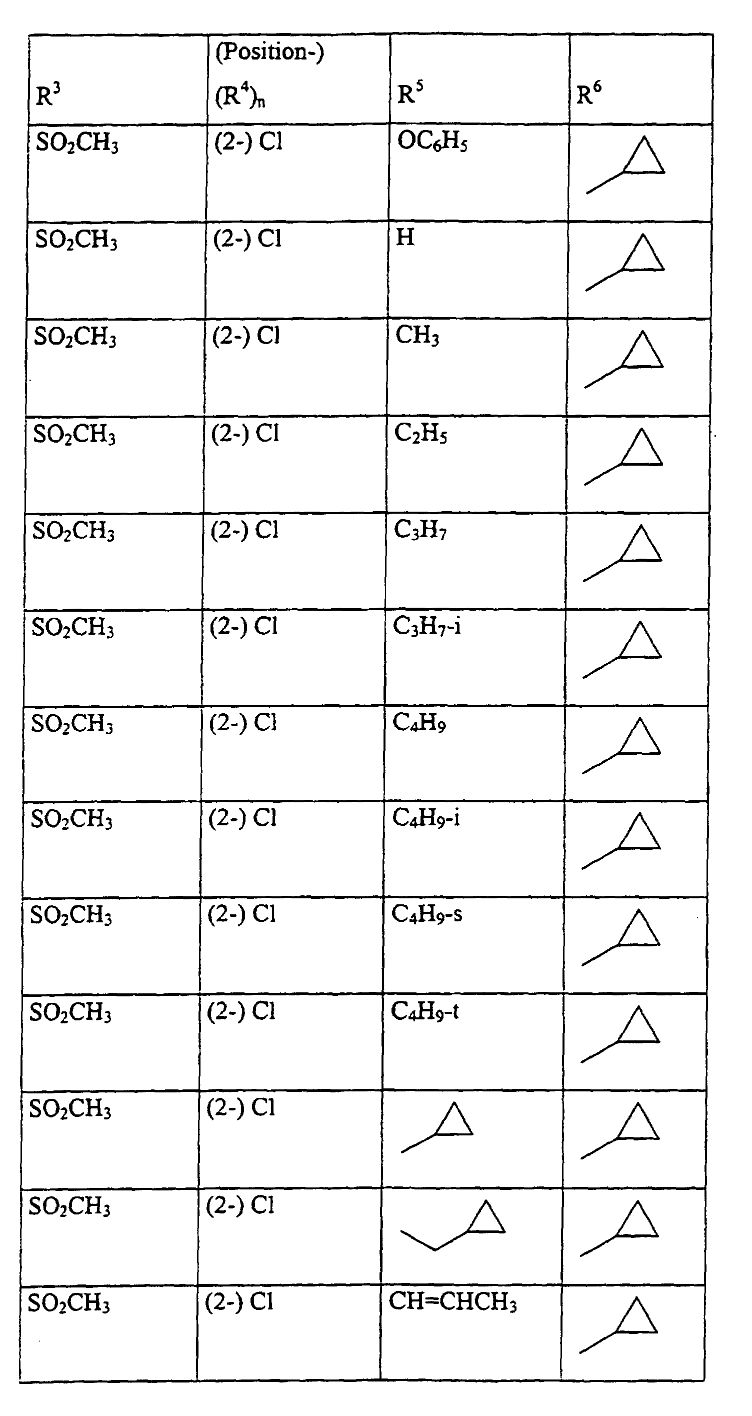

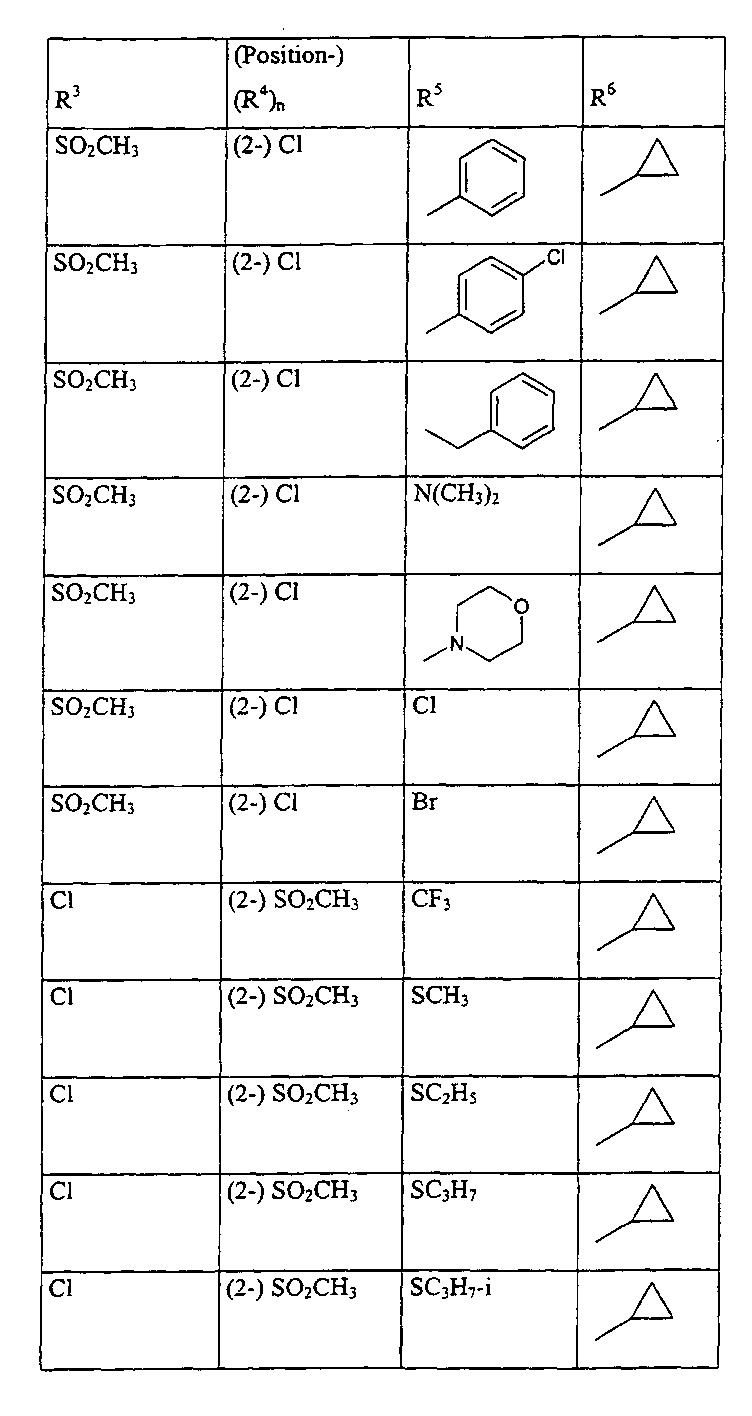

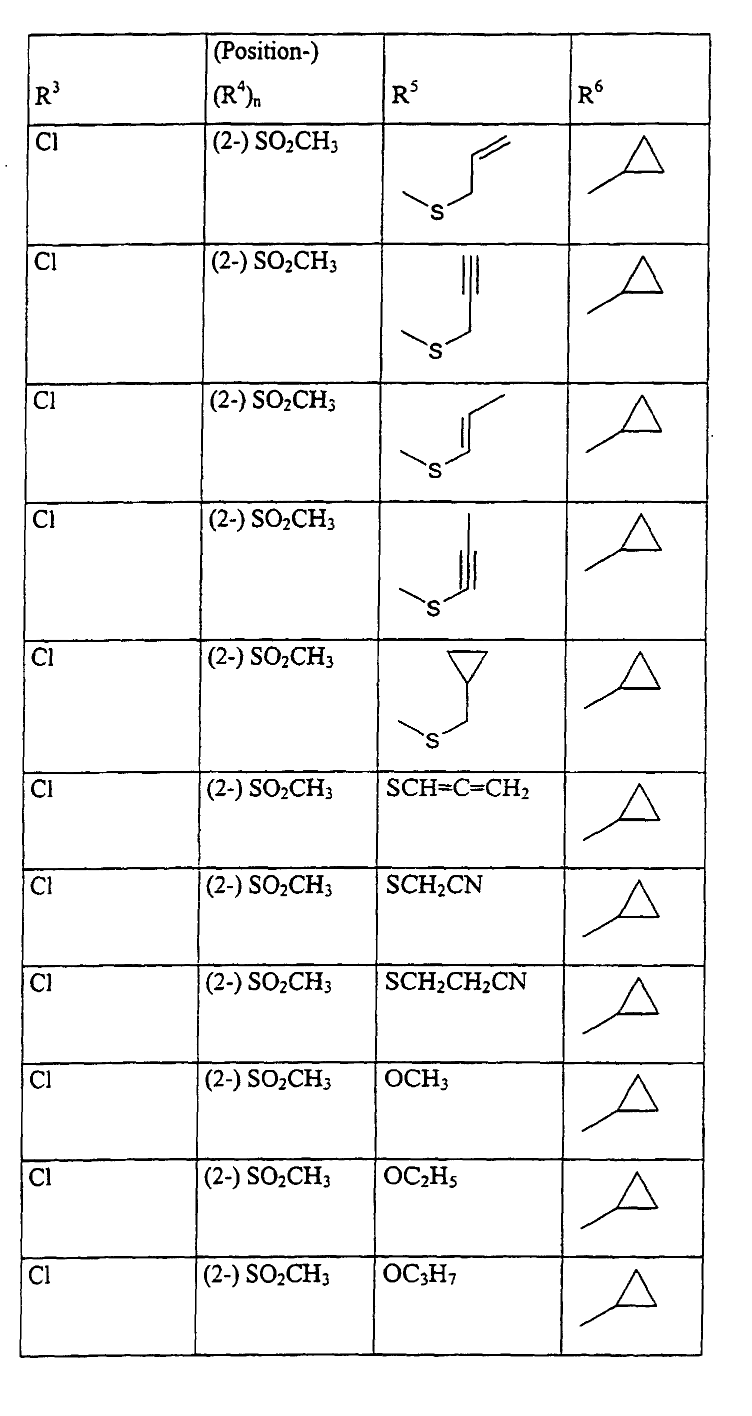

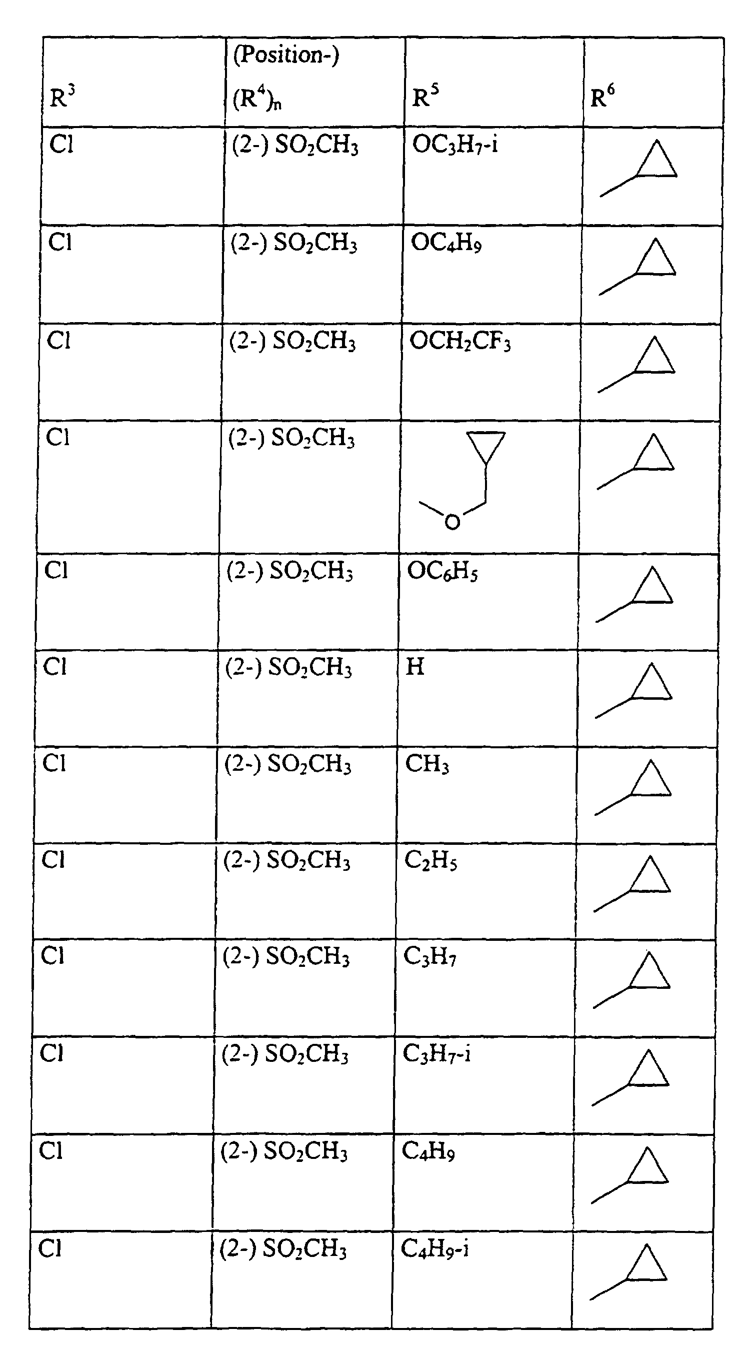

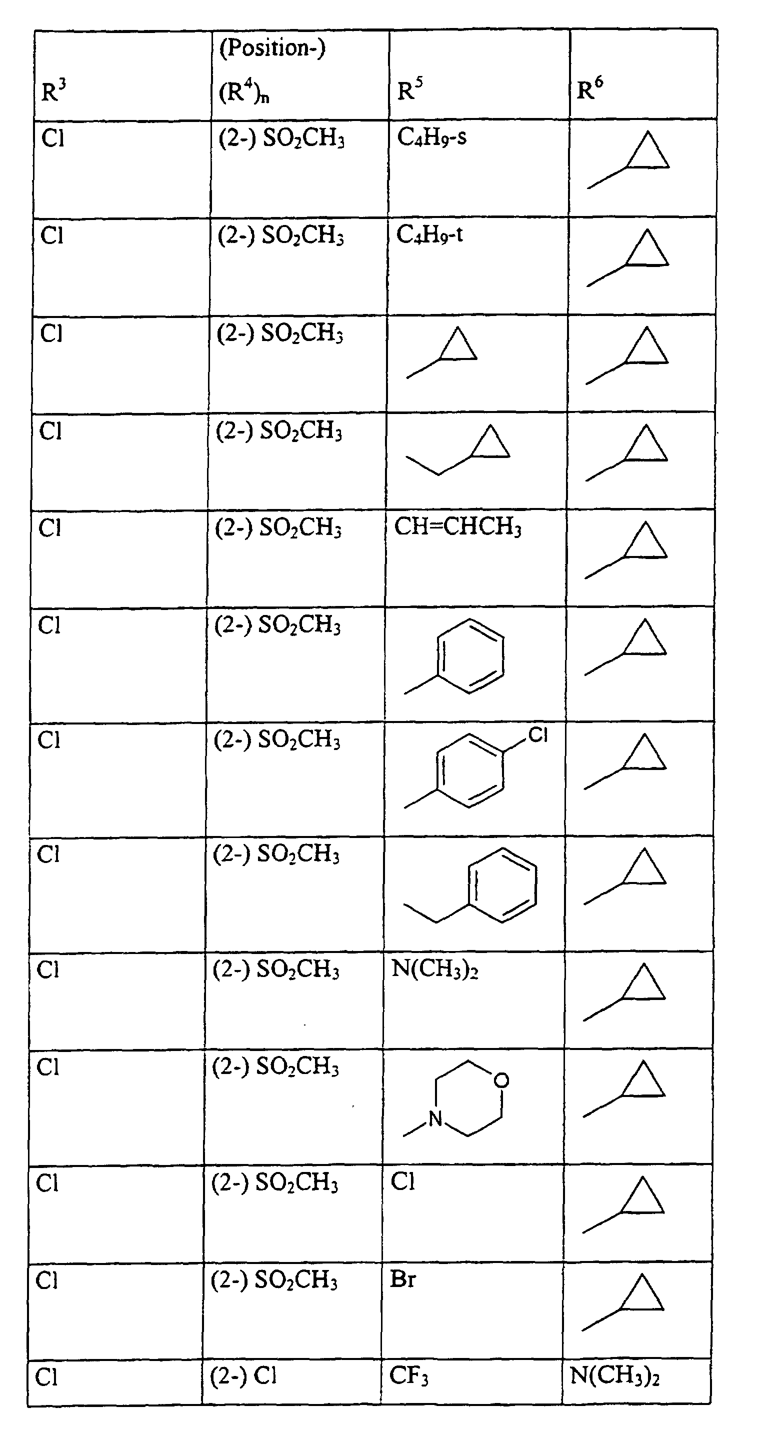

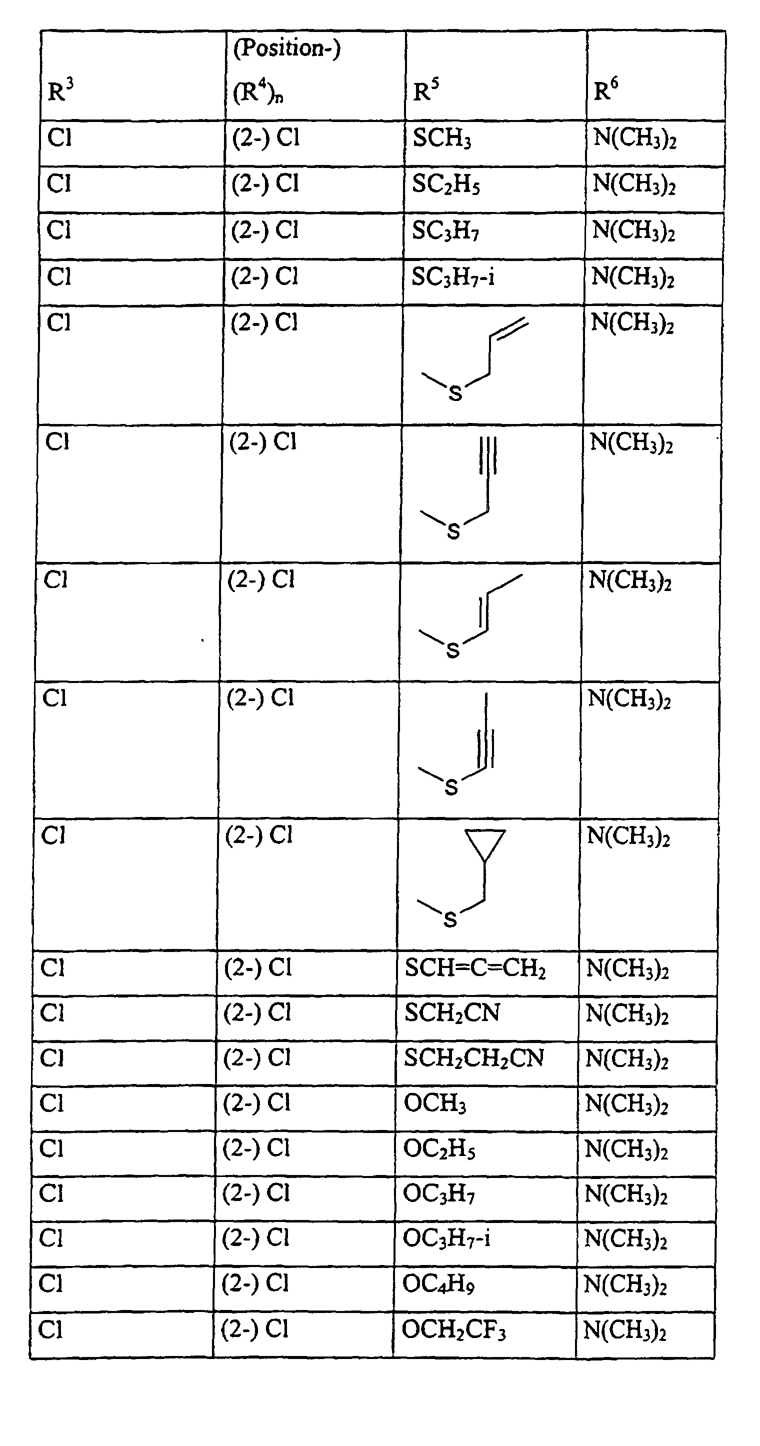

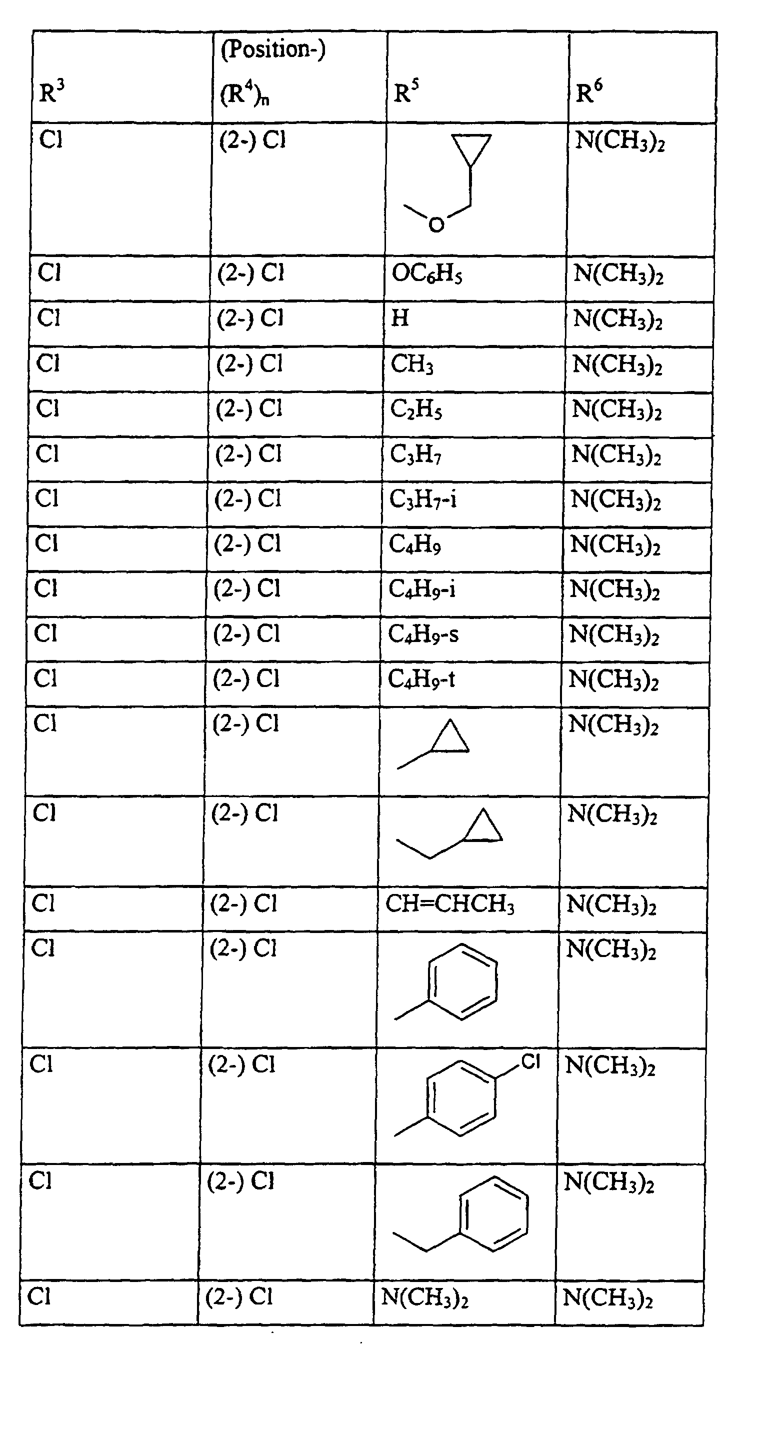

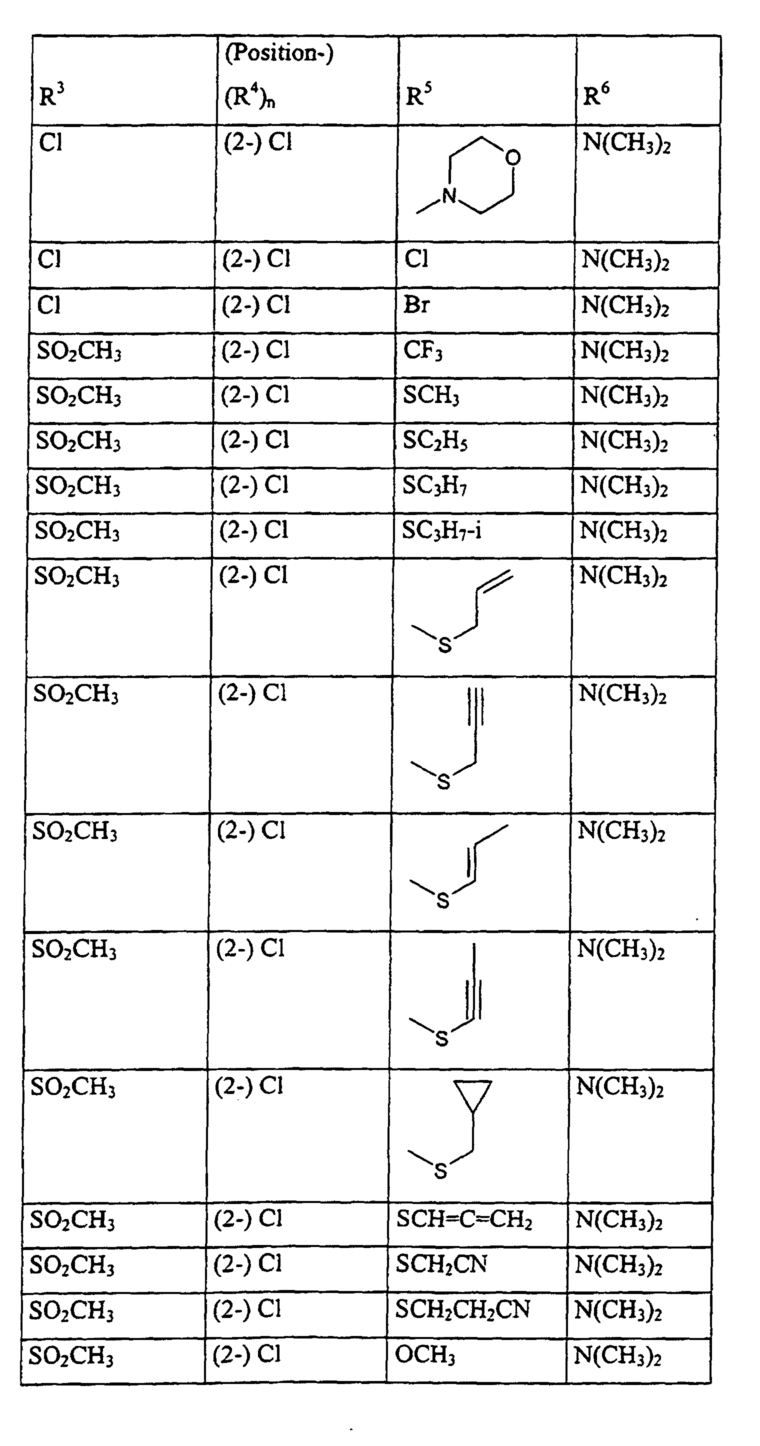

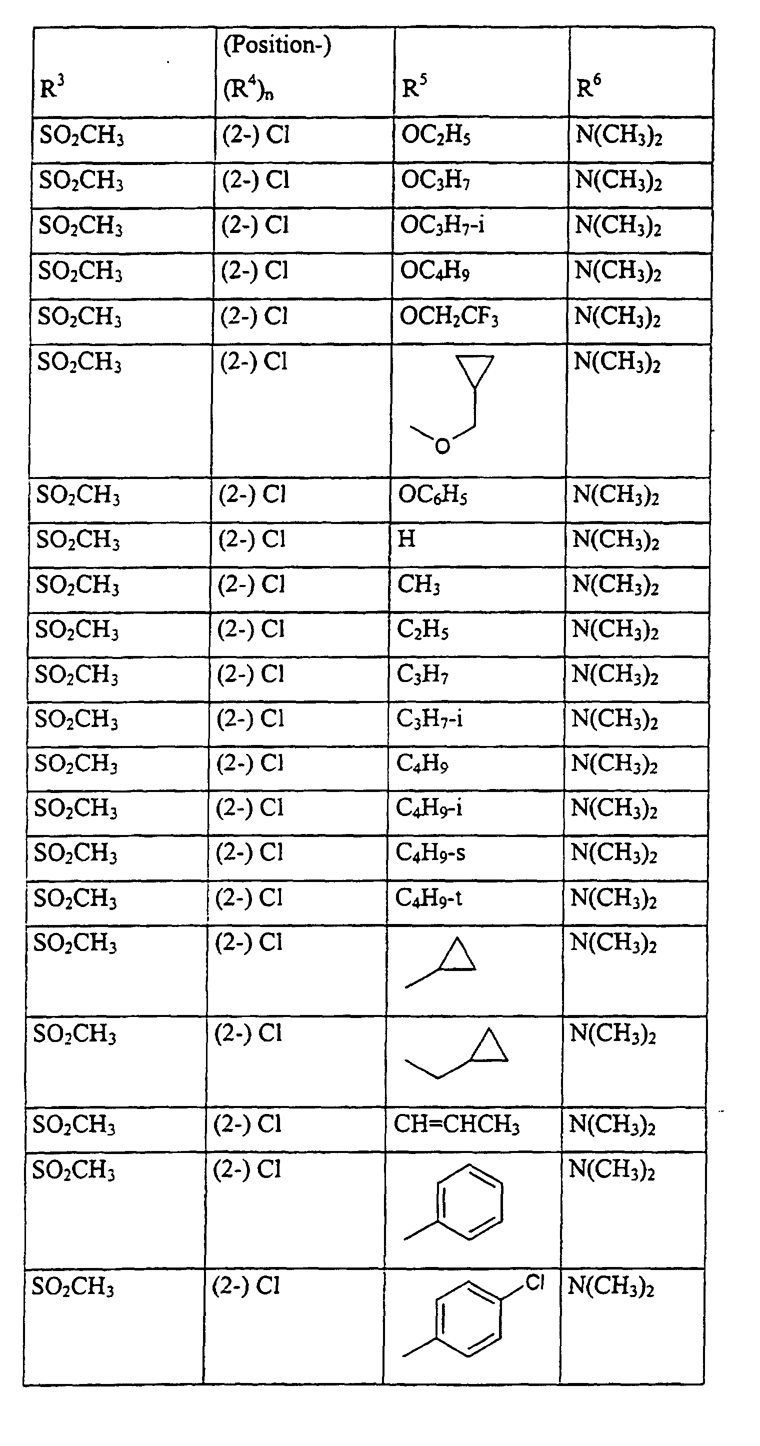

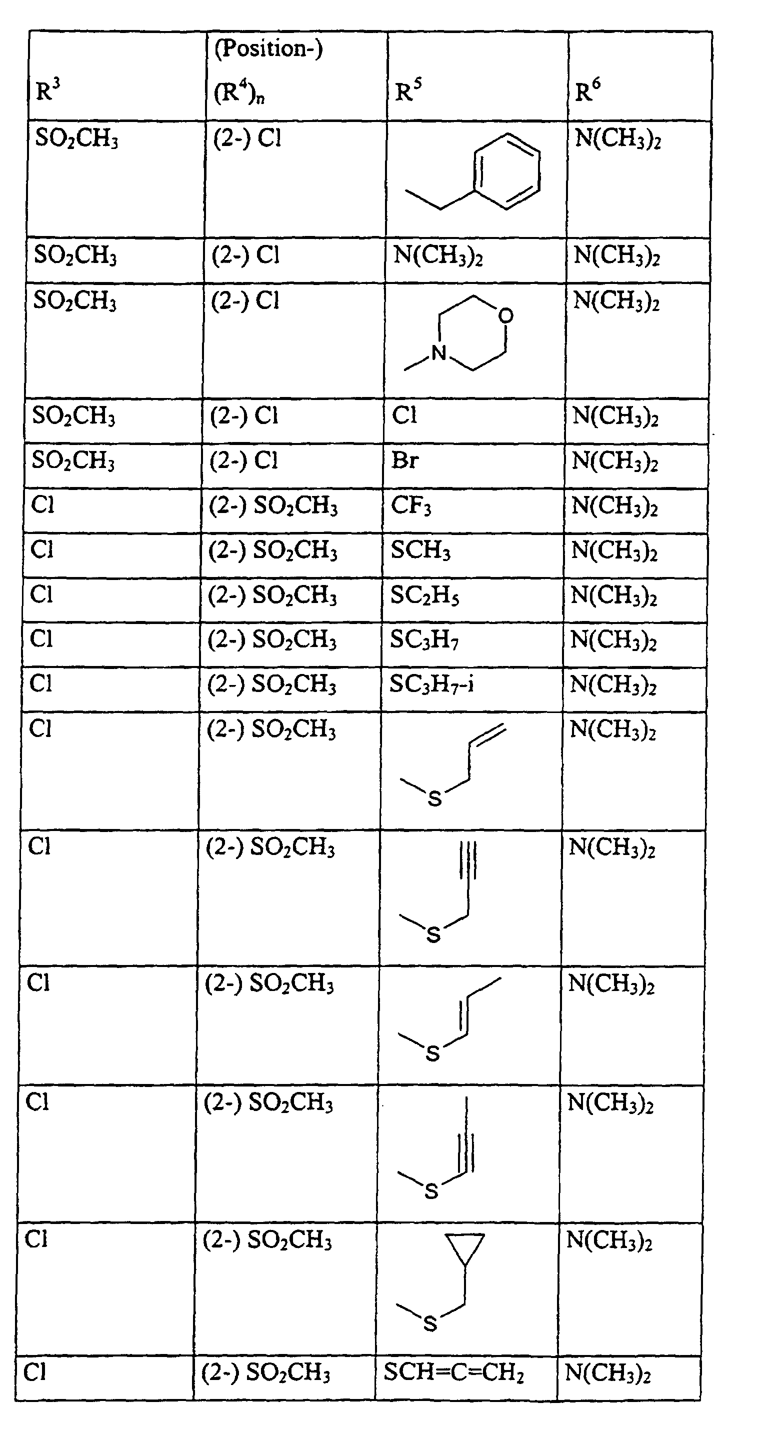

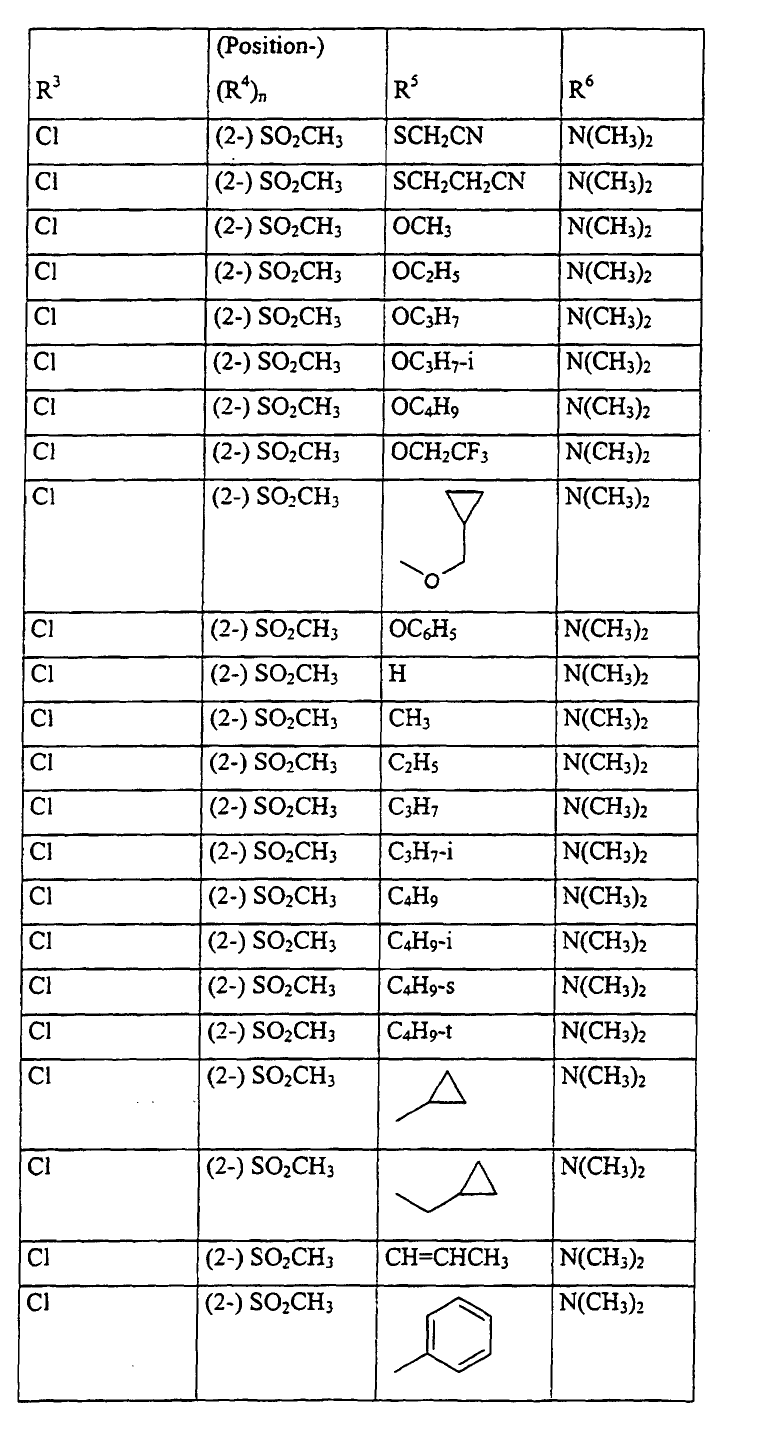

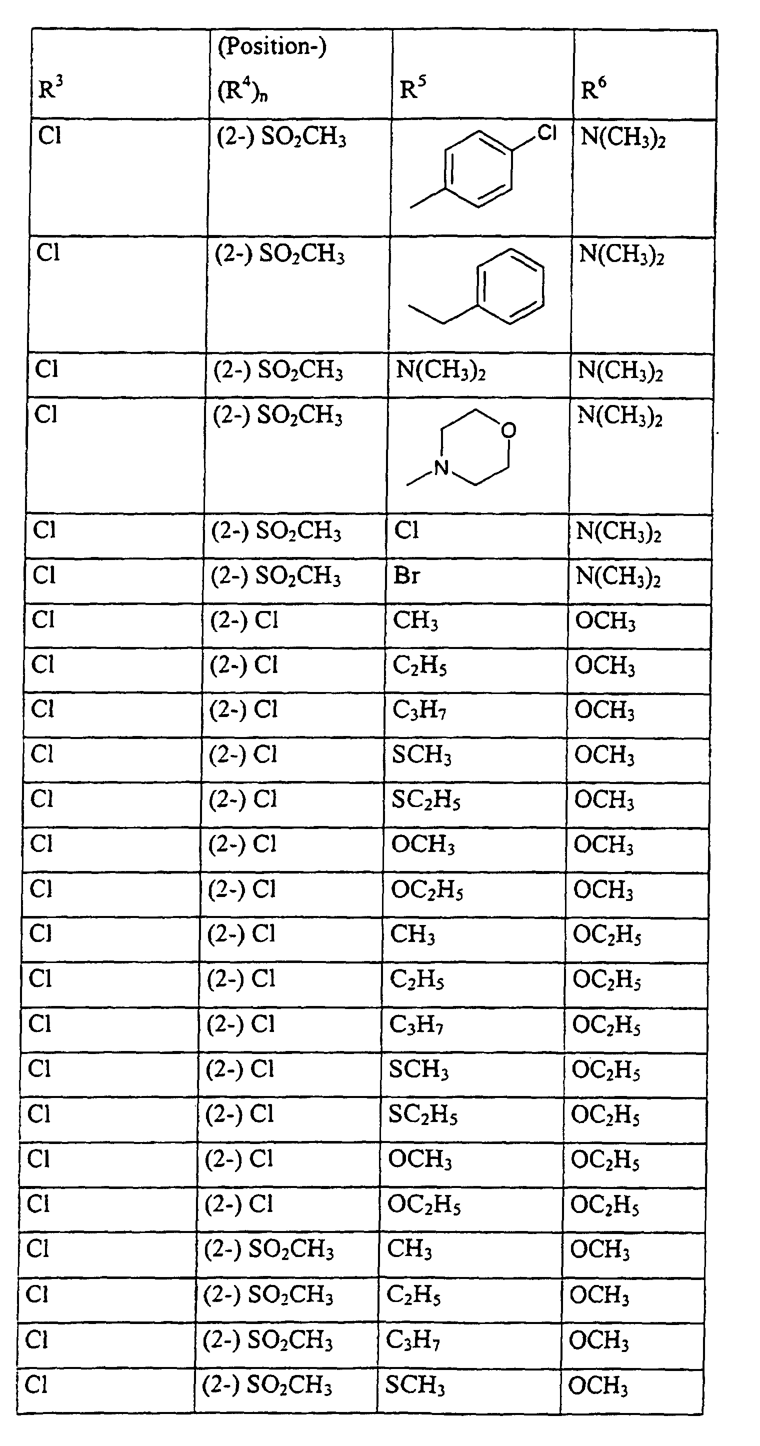

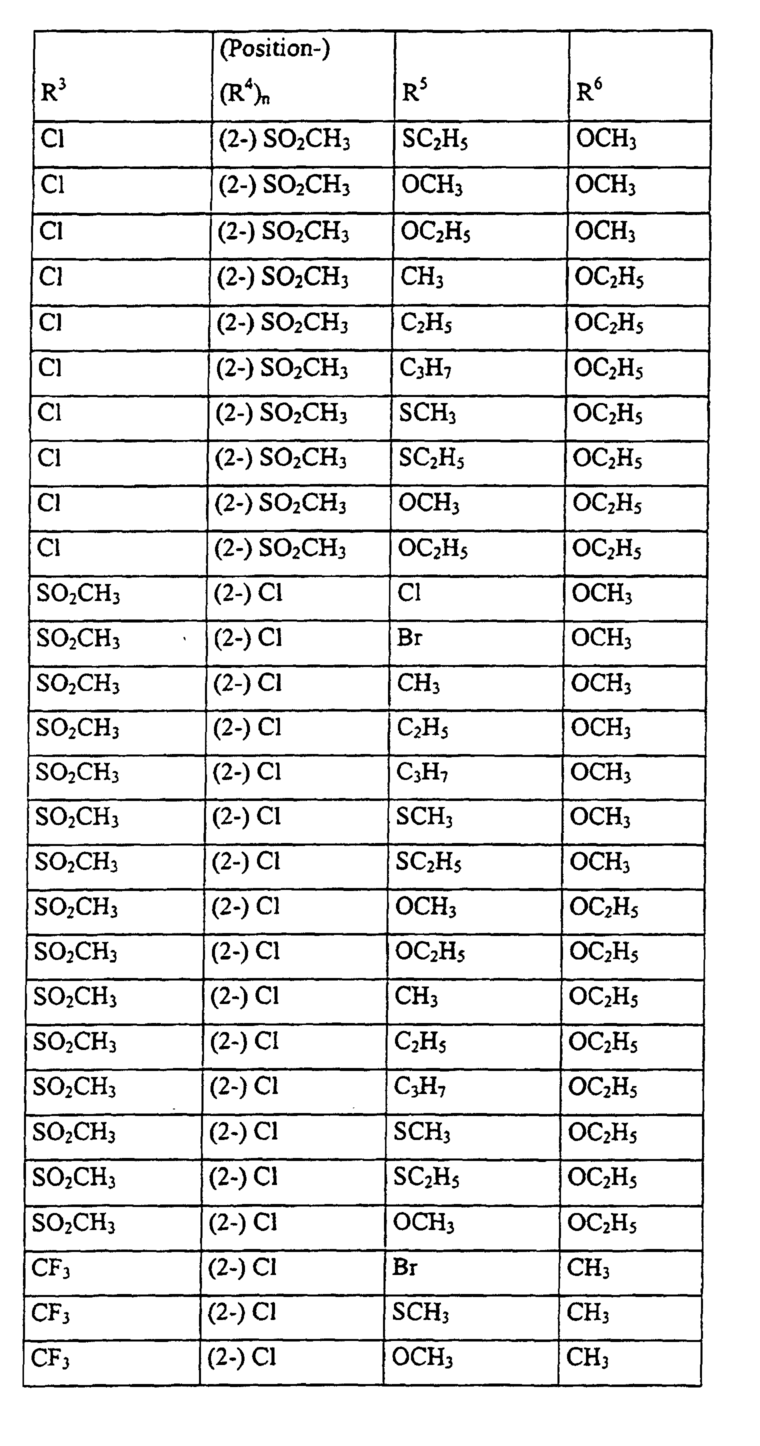

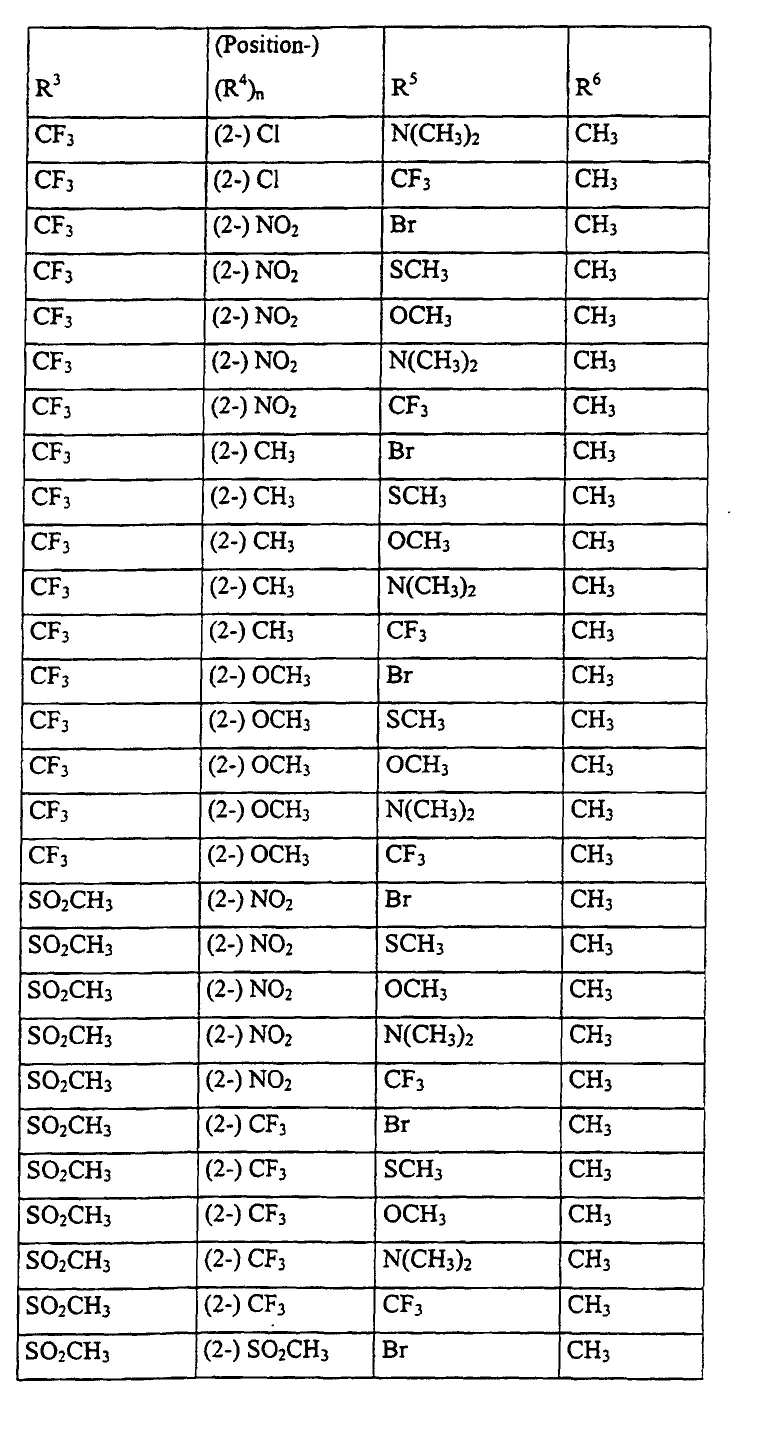

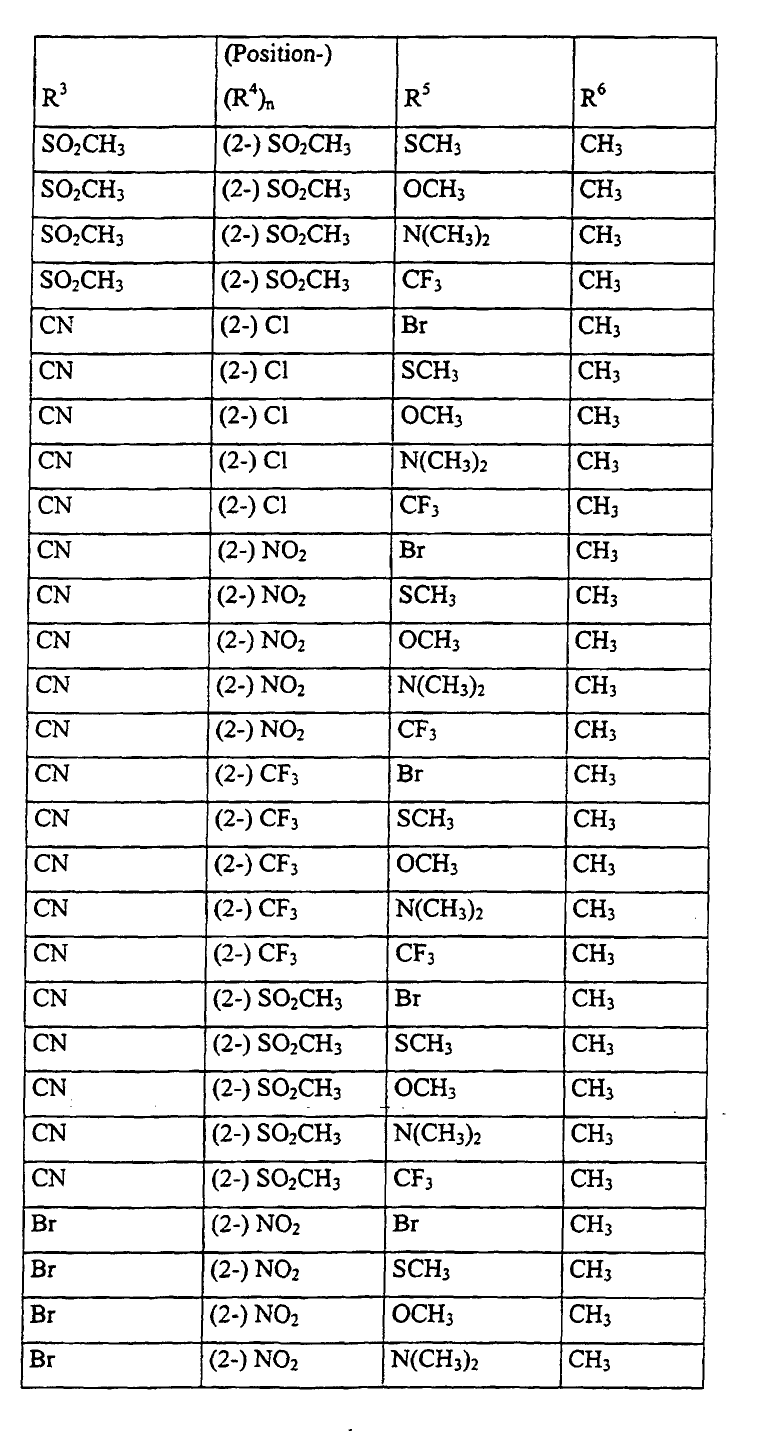

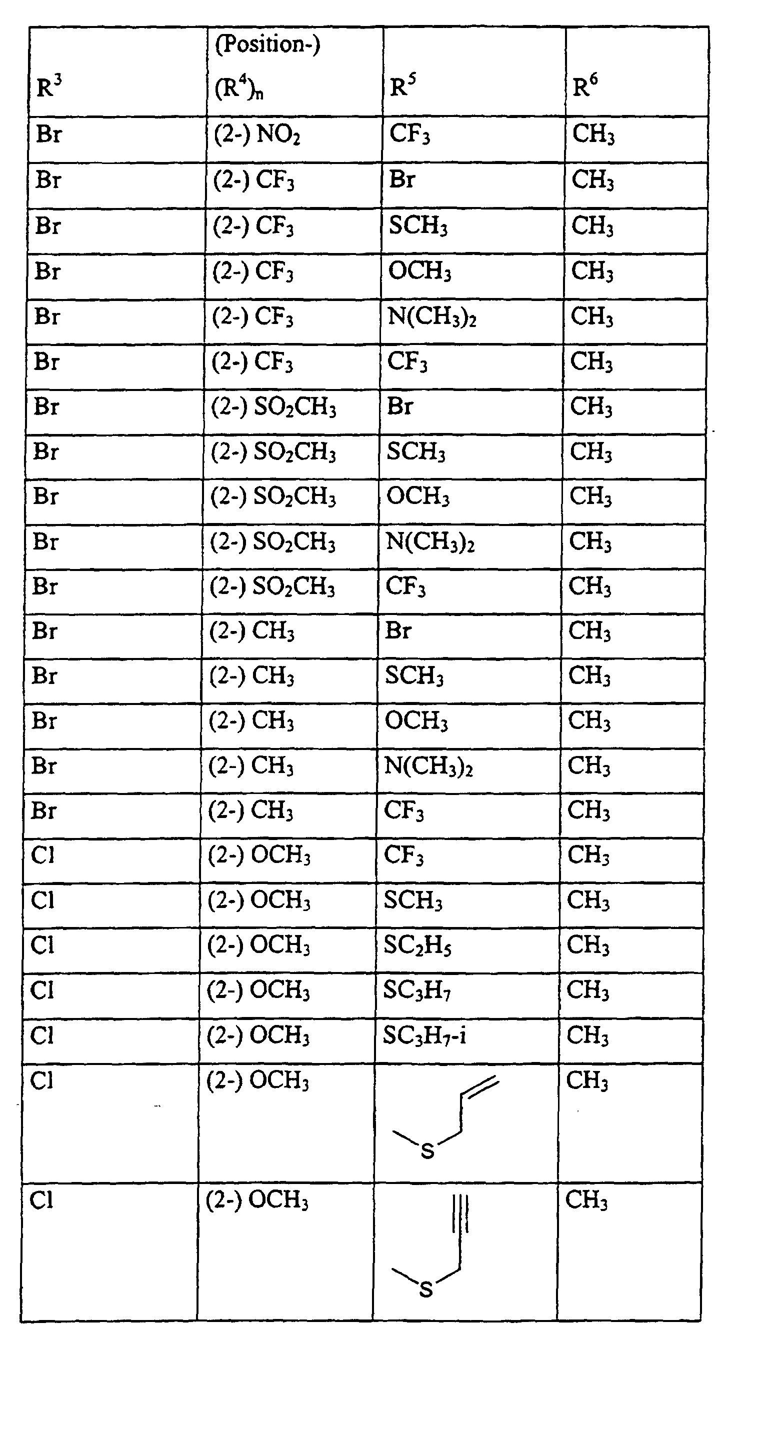

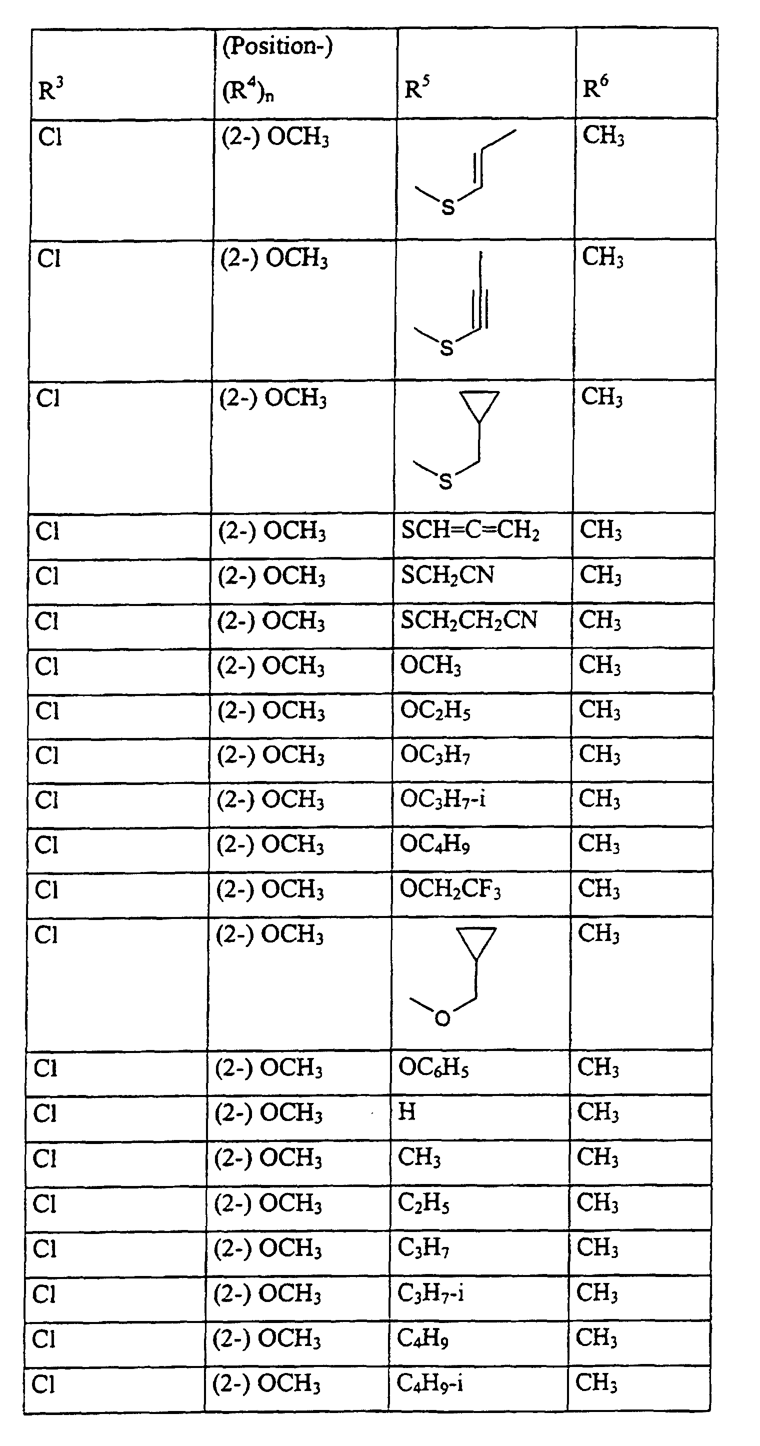

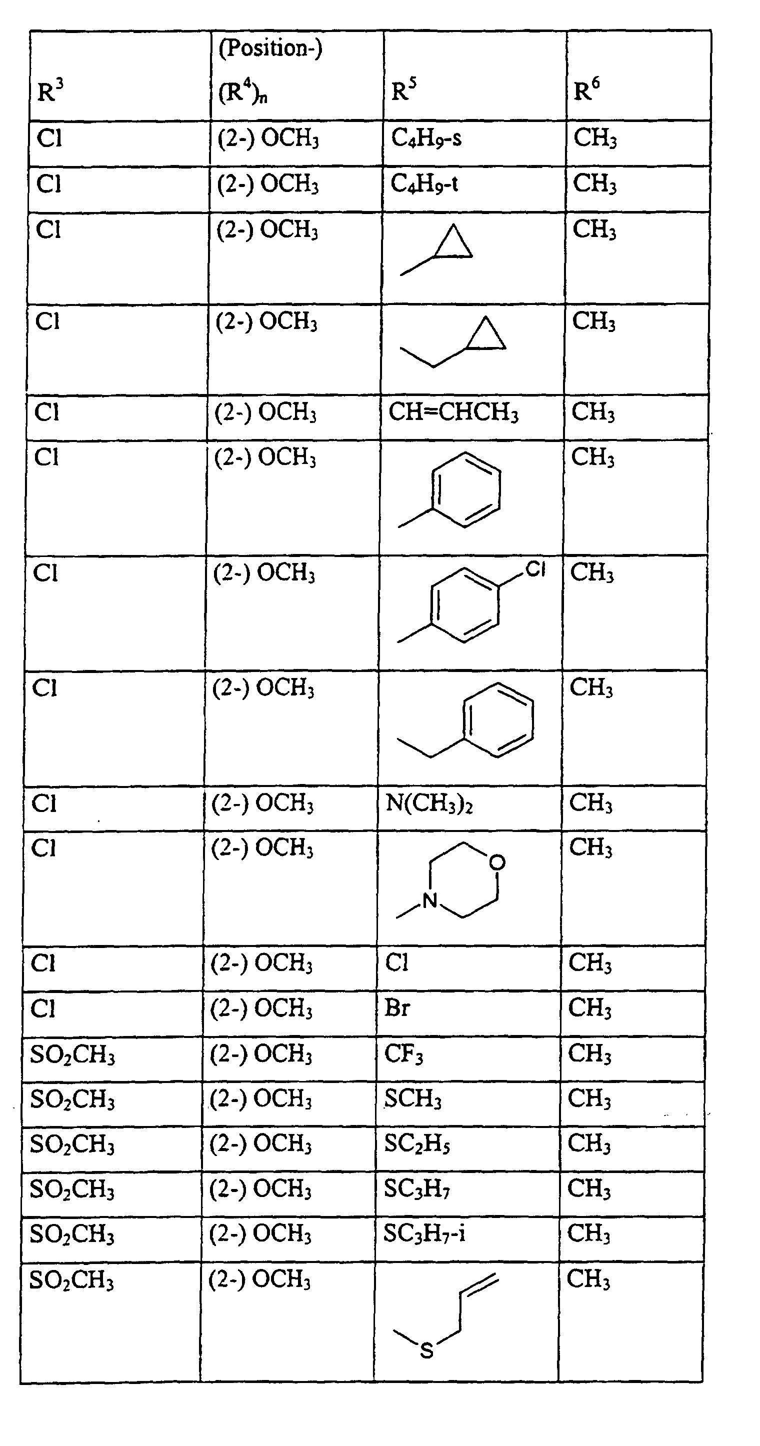

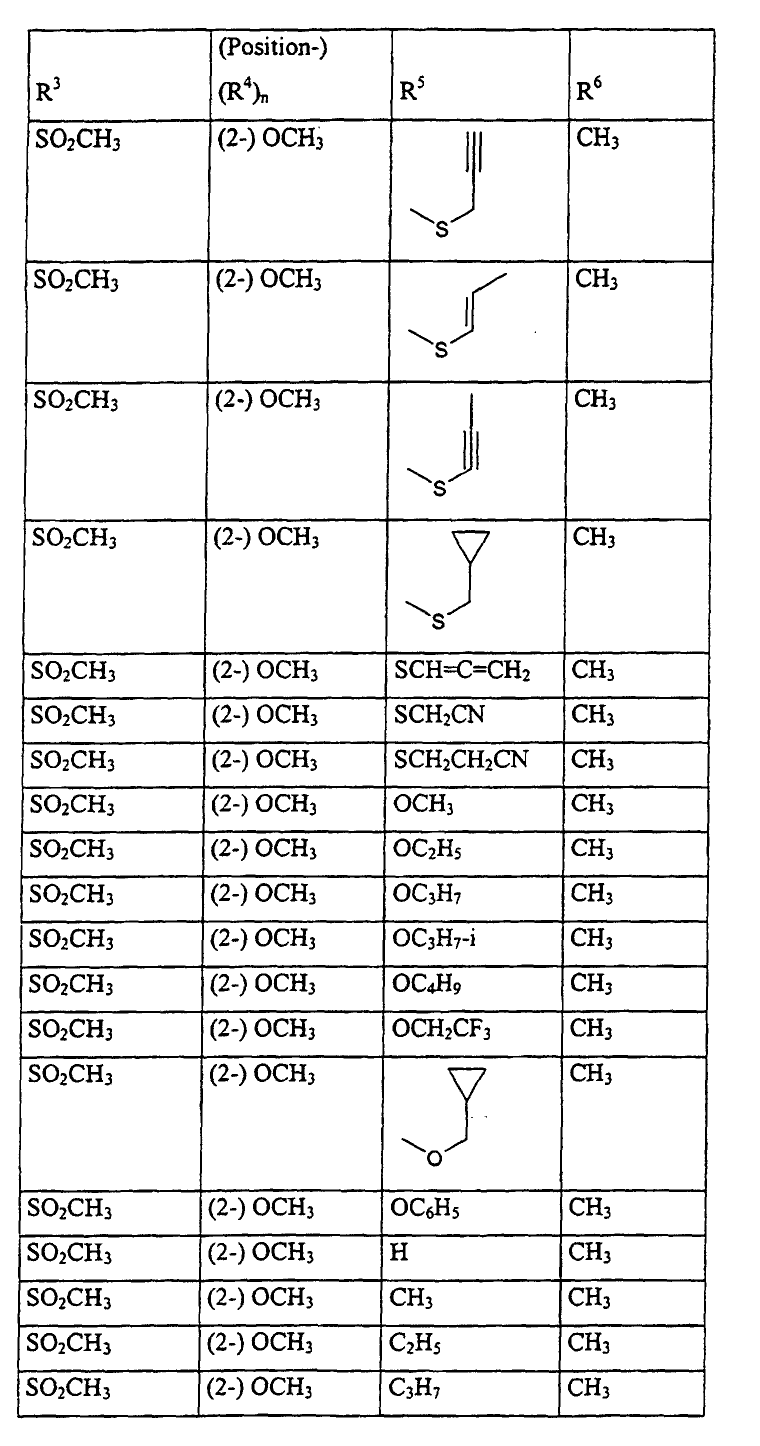

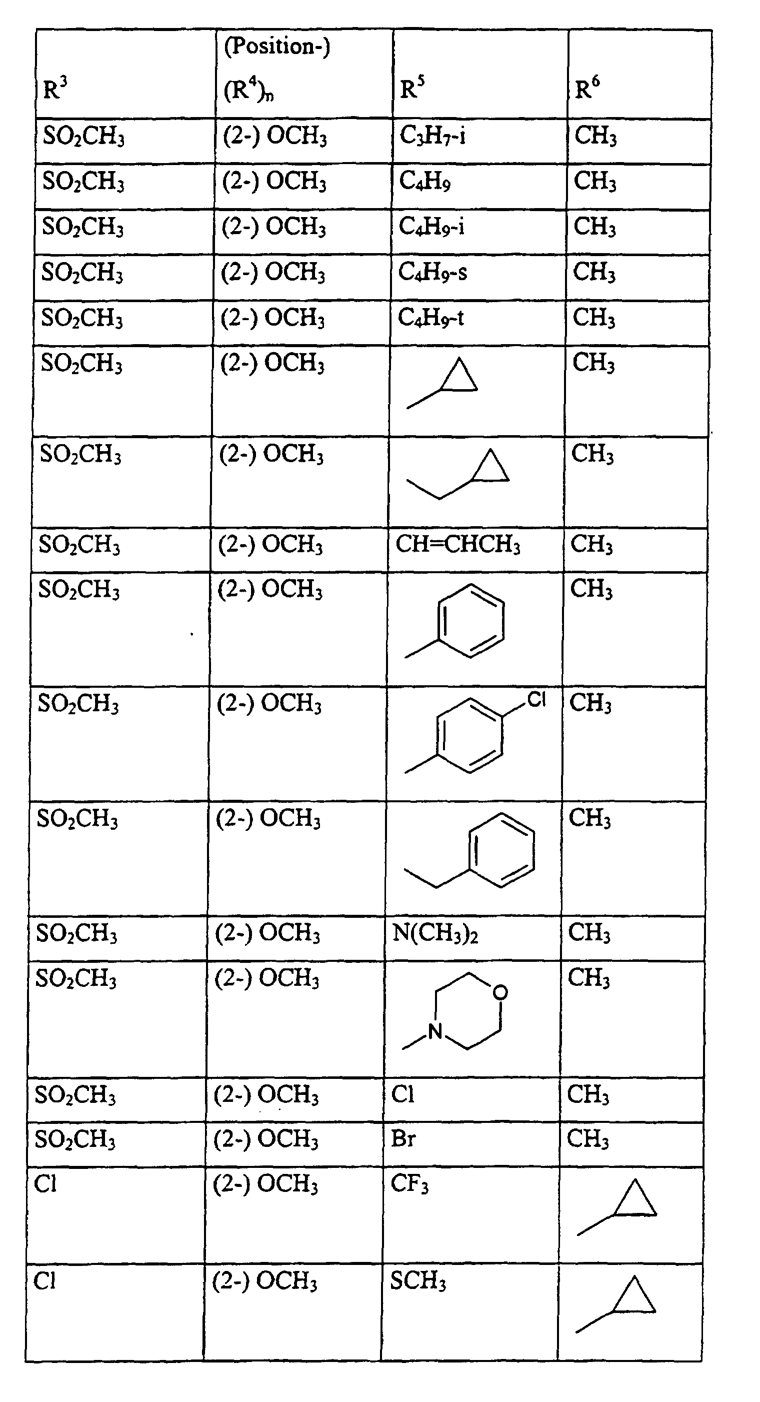

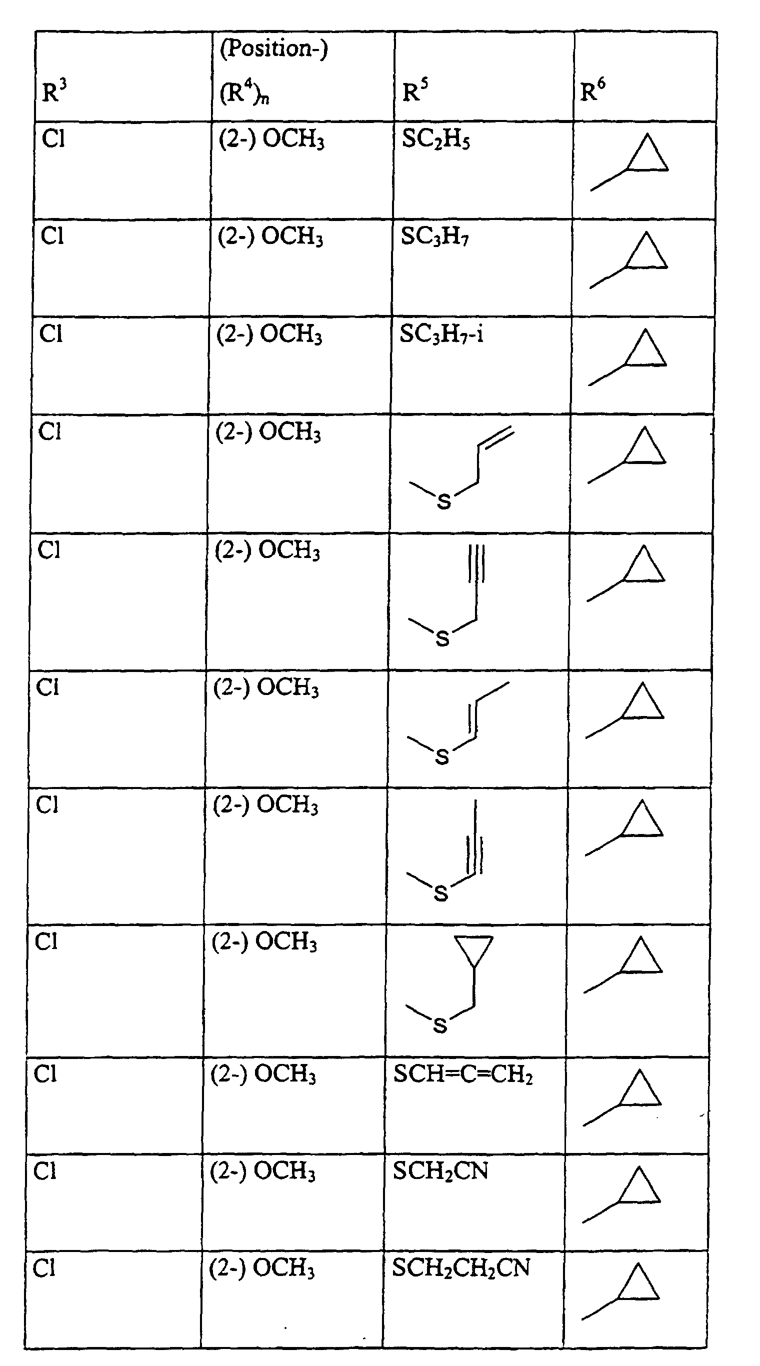

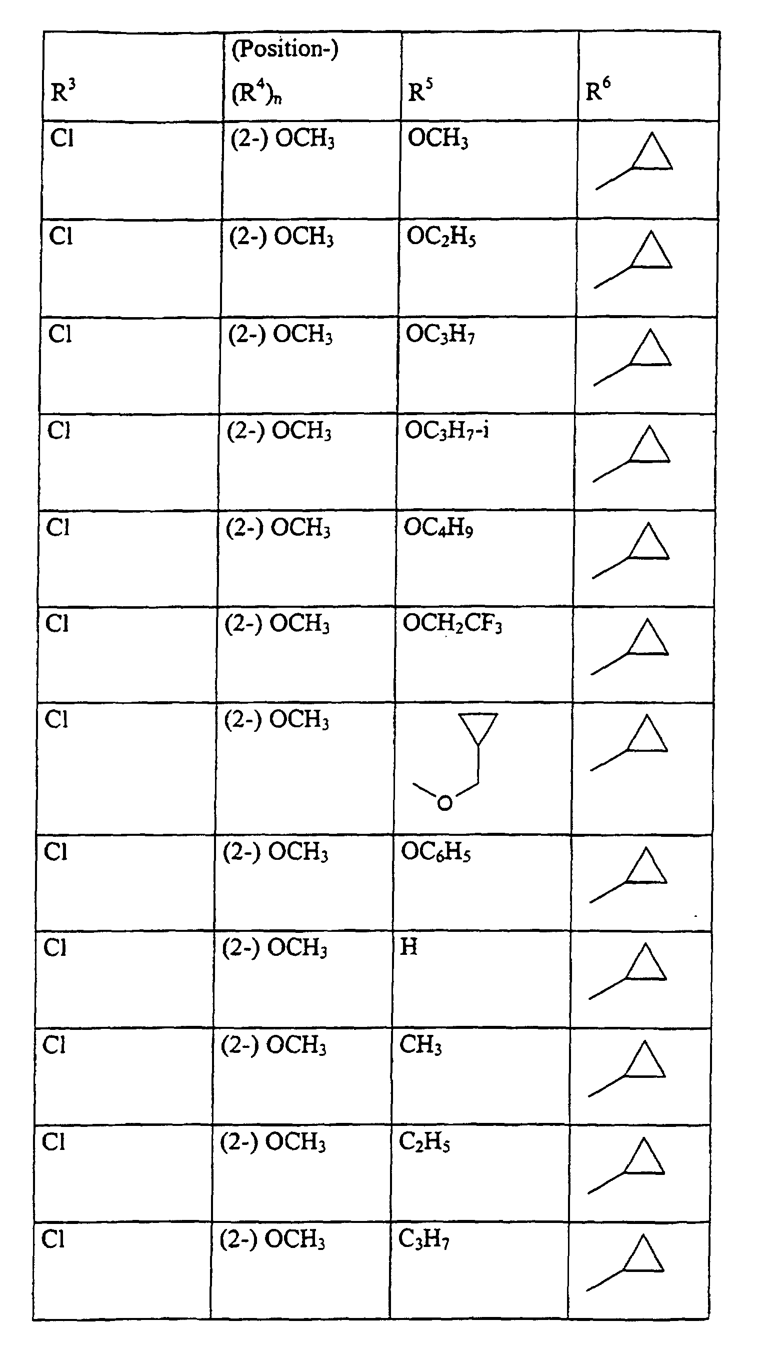

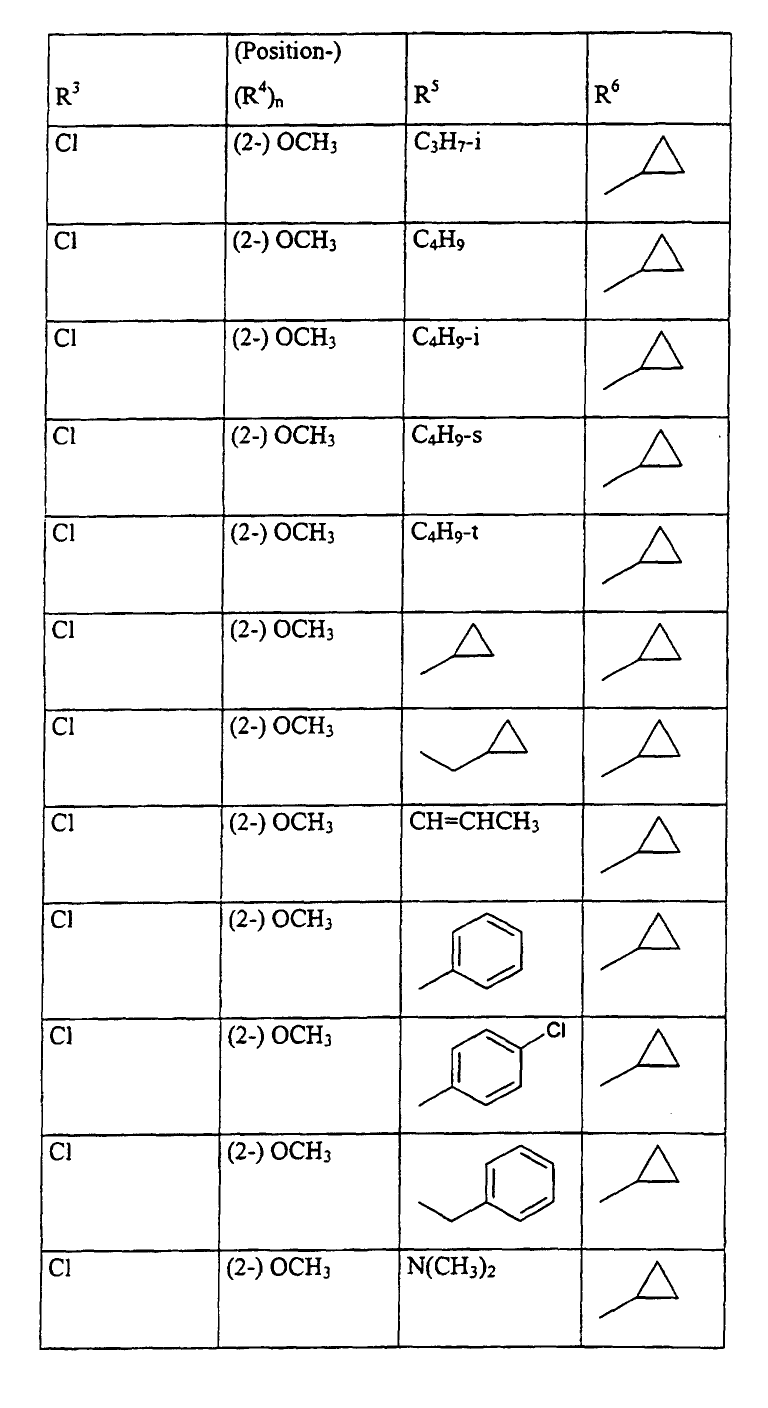

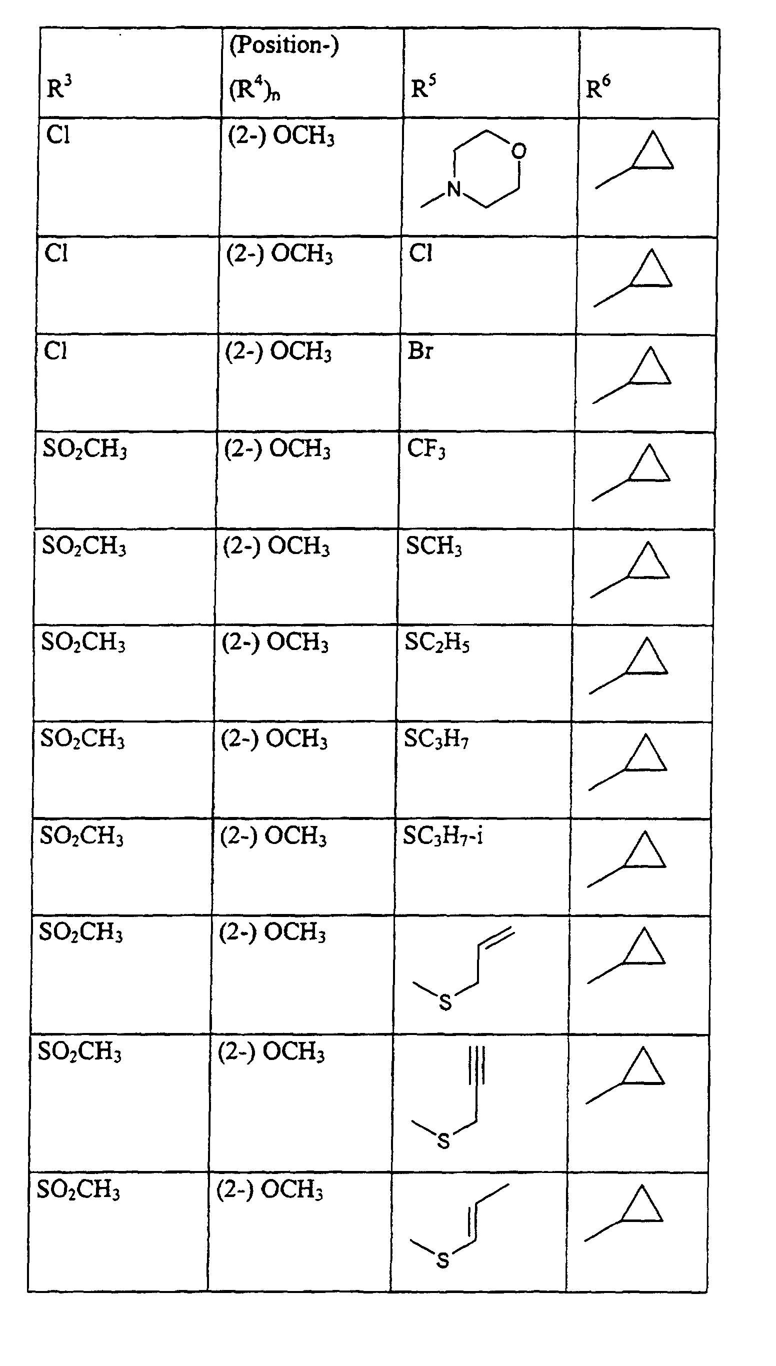

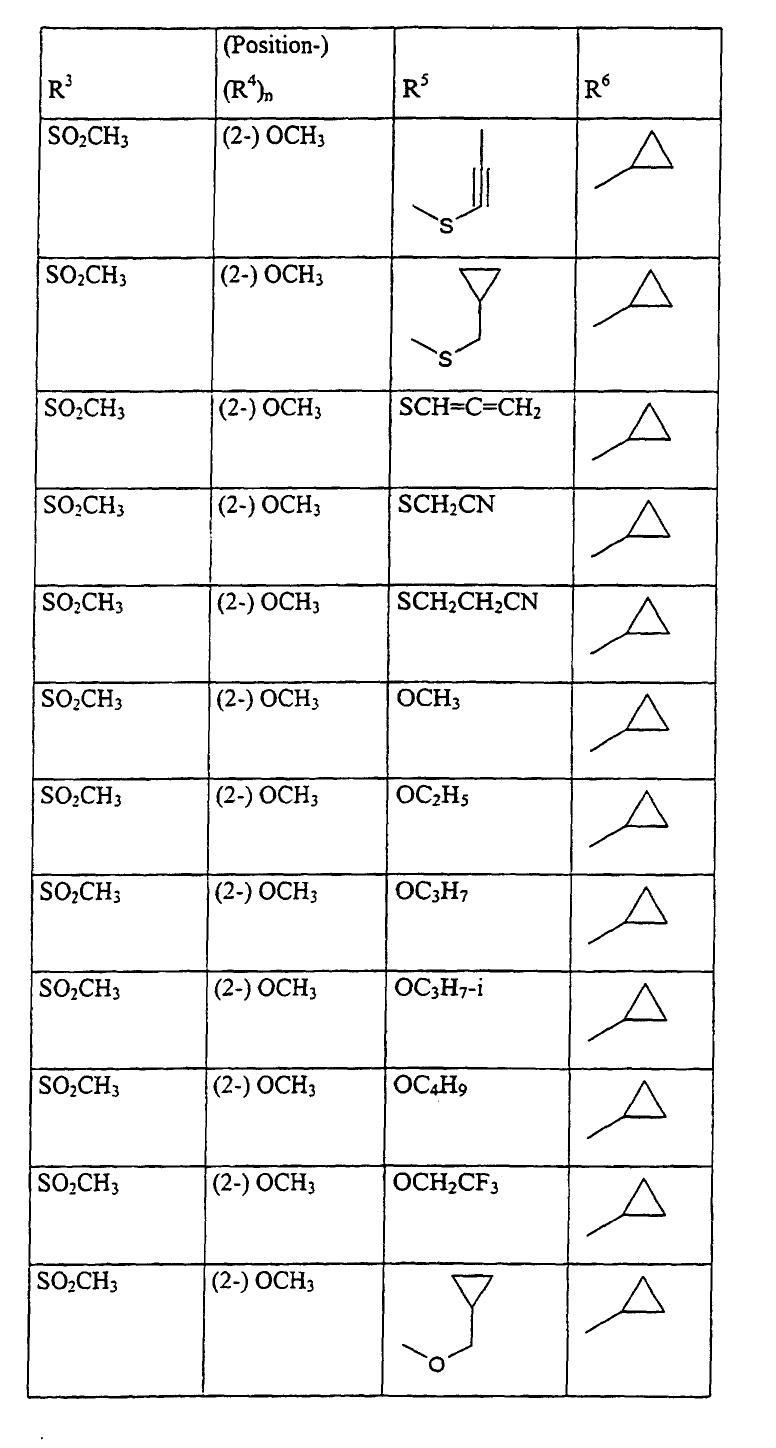

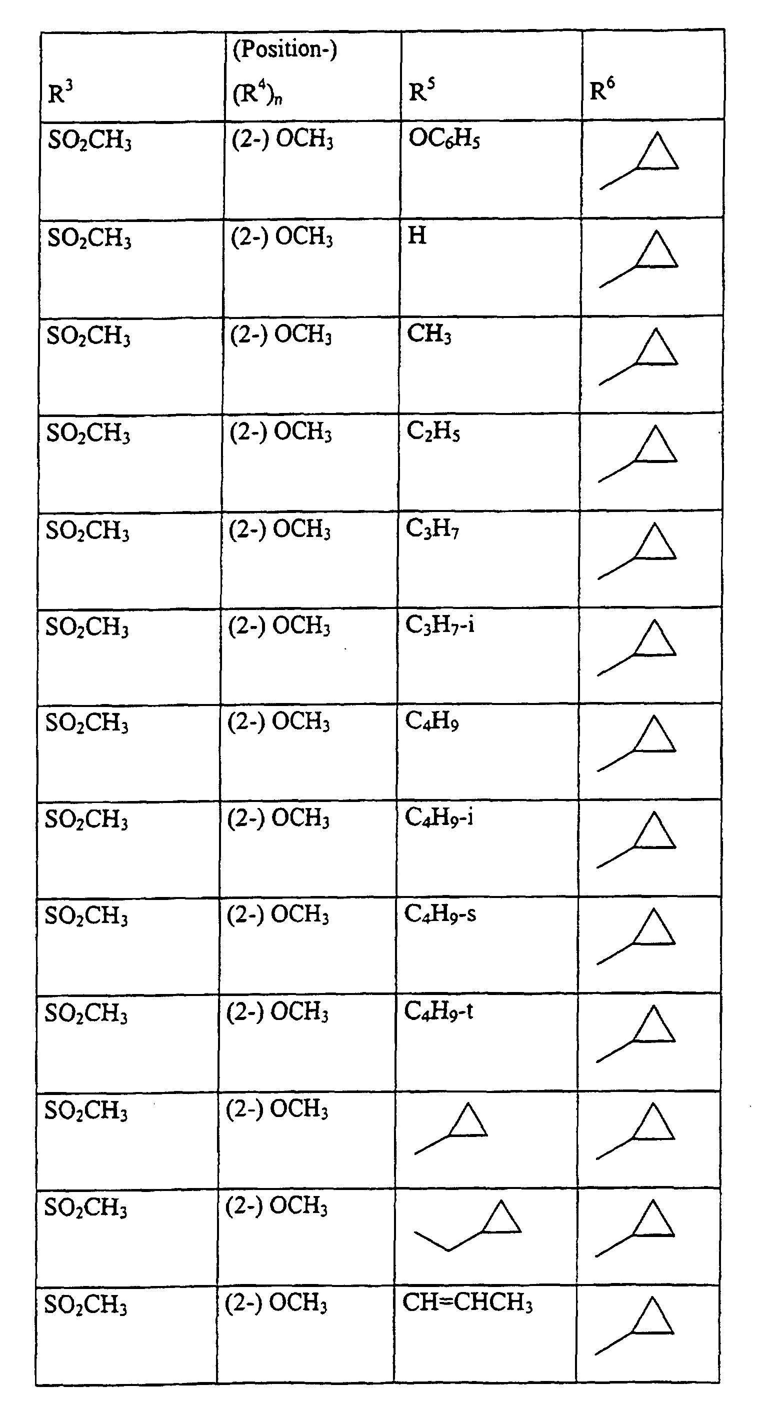

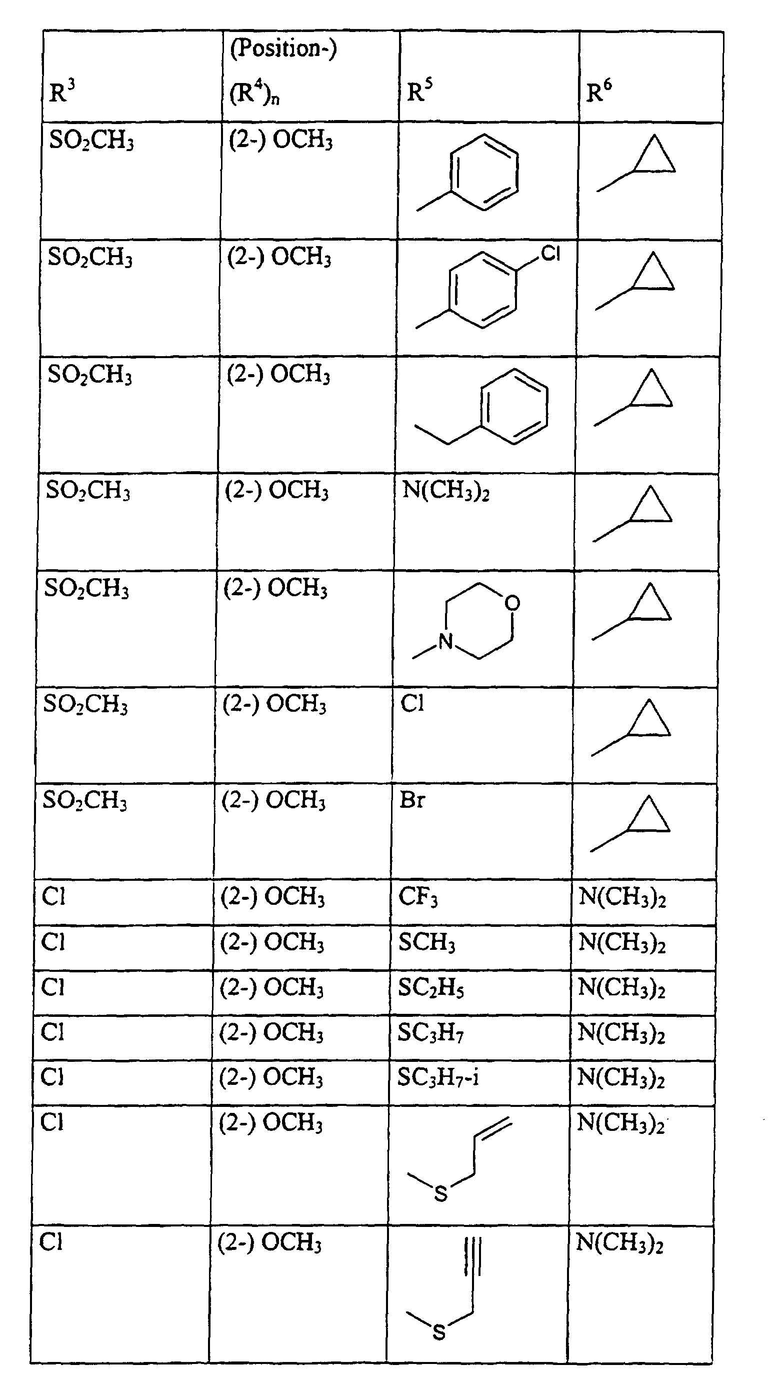

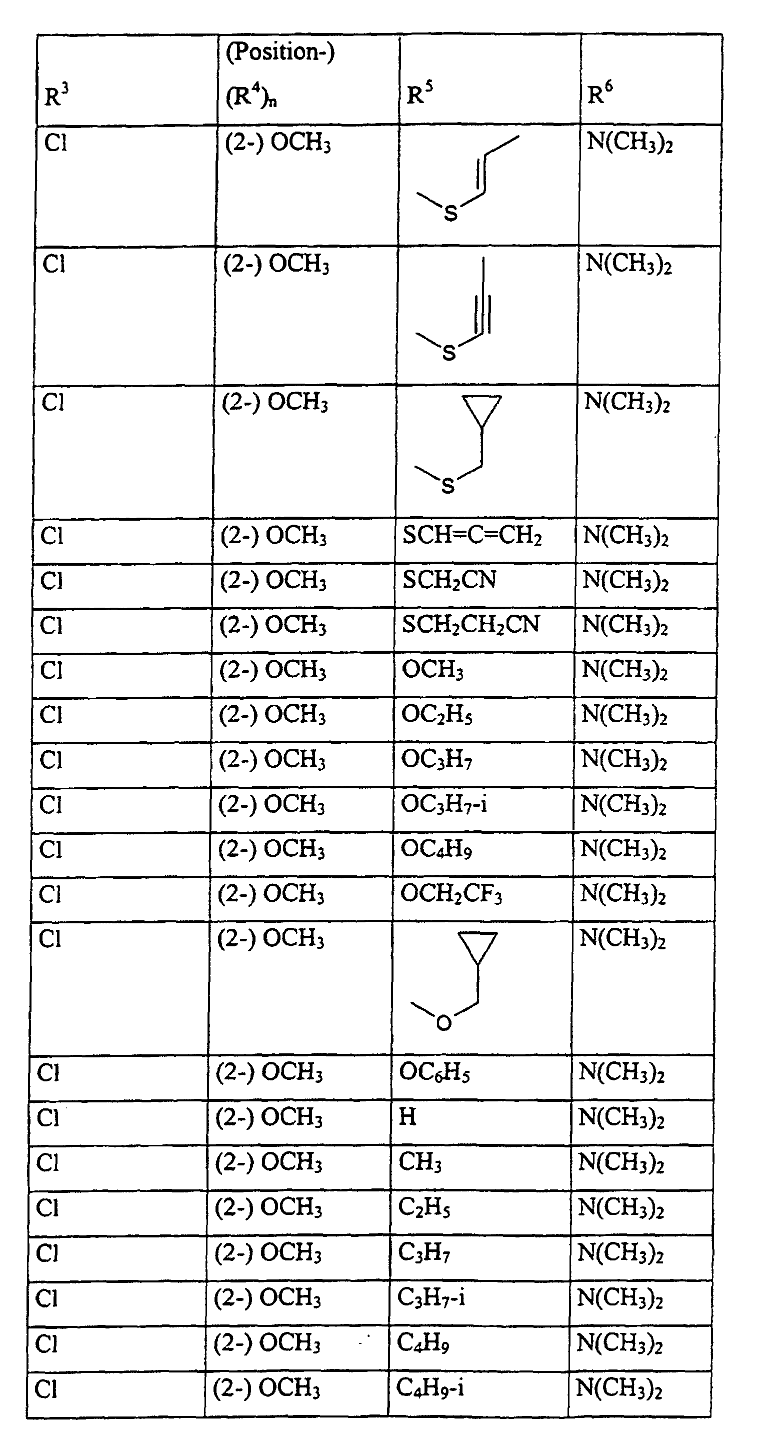

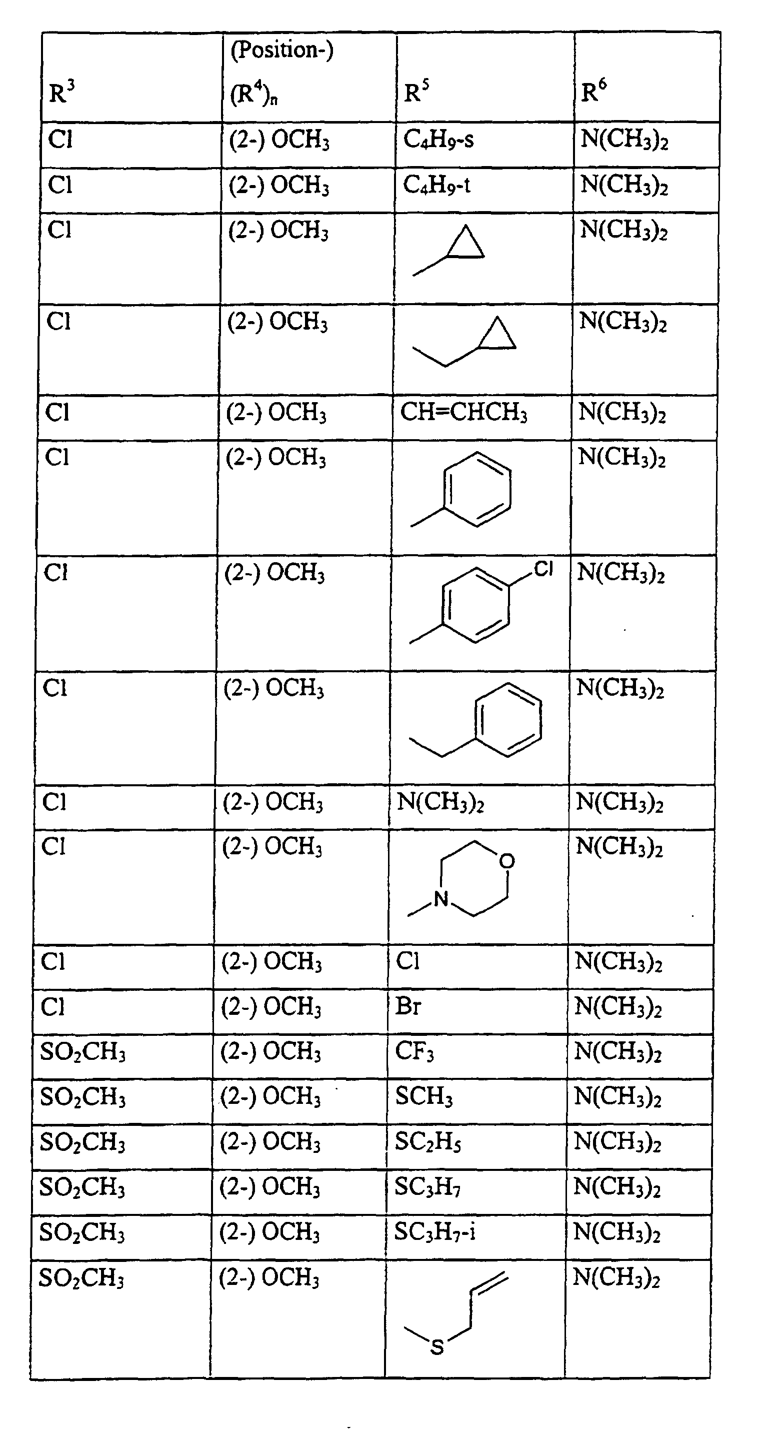

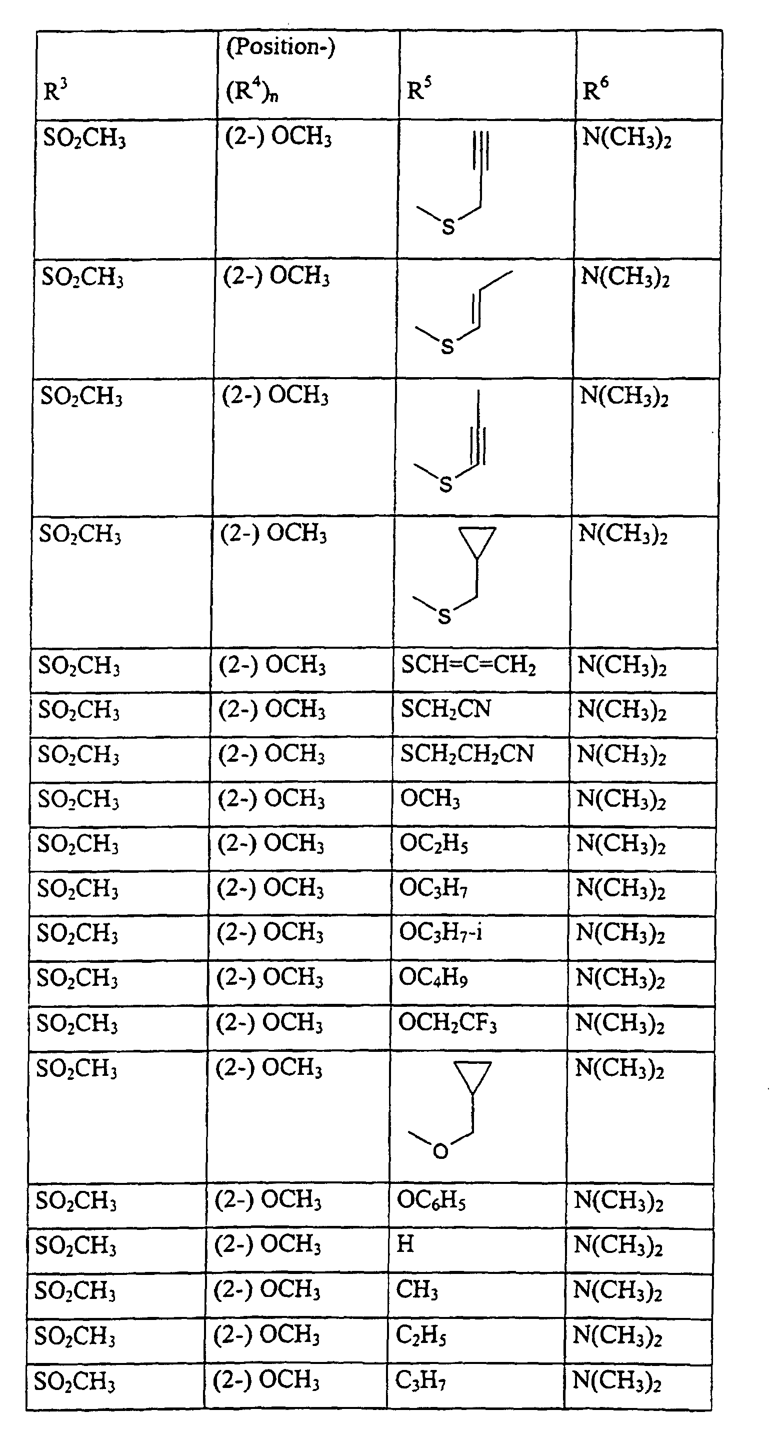

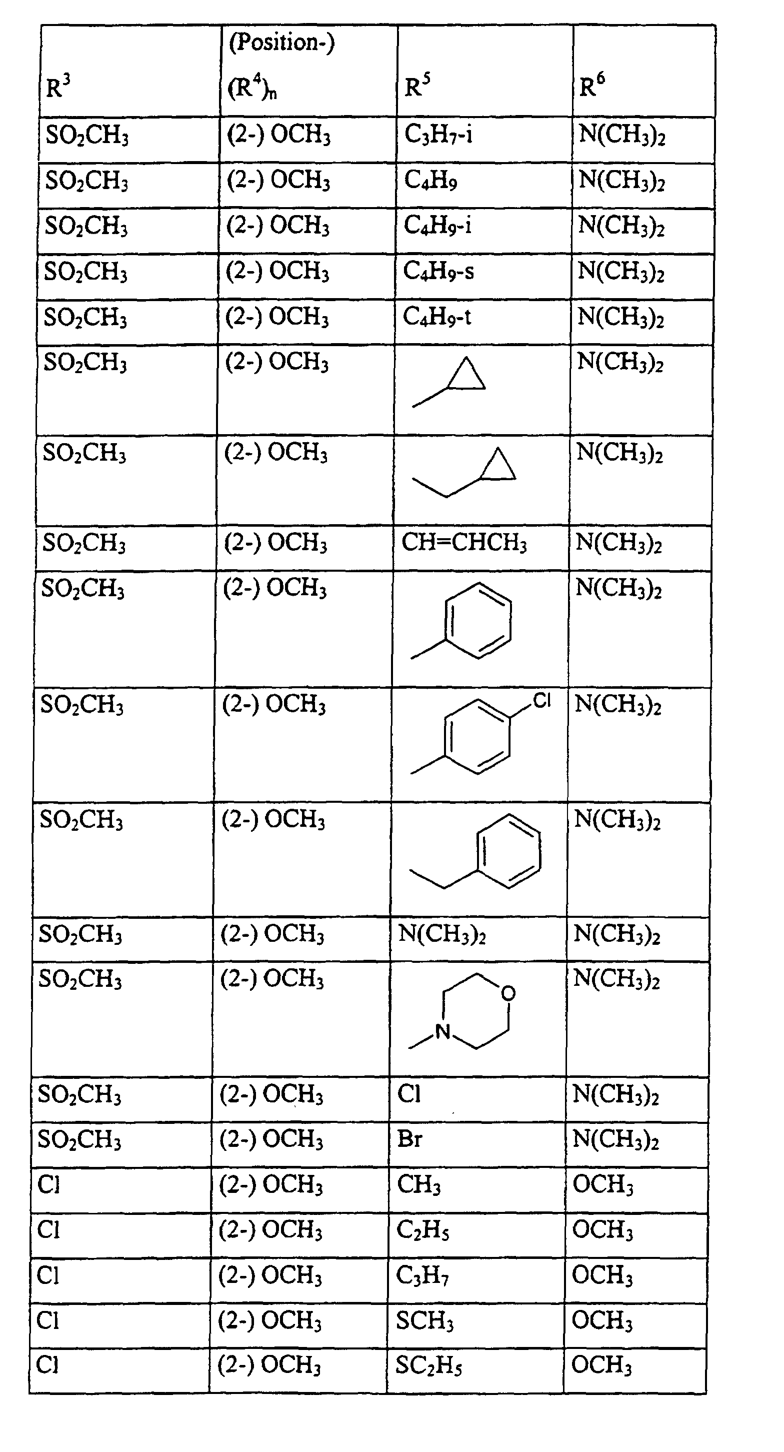

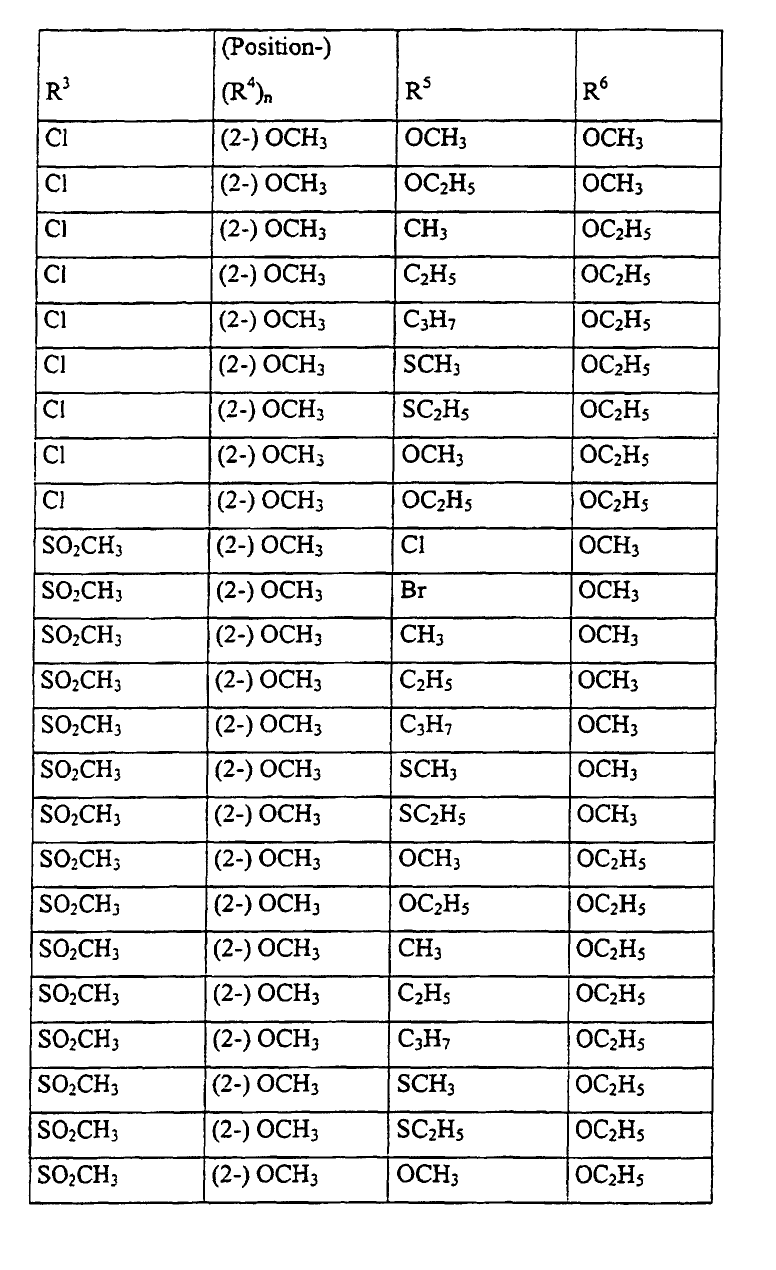

Die neuen substituierten Benzoylketone der allgemeinen Formel (I) zeichnen sich durch starke und selektive herbizide Wirksamkeit aus.

Man erhält die neuen substituierten Benzoylketone der allgemeinen Formel (I), wenn man
(a) Ketone der allgemeinen Formel (II) 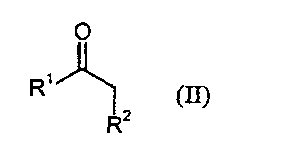 in welcher
   - R¹ und R²: die vorausgehend angegebenen Bedeutungen haben,
   mit substituierten Benzoesäuren der allgemeinen Formel (III) 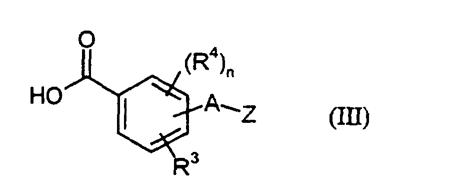 in welcher
   n, A, R³, R⁴ und Z die vorausgehend angegebenen Bedeutungen haben,
   gegebenenfalls in Gegenwart eines oder mehrerer Reaktionshilfsmittel und gegebenenfalls in Gegenwart eines oder mehrerer Verdünnungsmittels umsetzt,
   und gegebenenfalls im Anschluss daran, d.h. nach Durchführung der erfindungsgemäßen Verfahren (a) an den so erhaltenen Verbindungen der Formel (I) im Rahmen der Substituentendefinition auf übliche Weise elektrophile oder nucleophile bzw. Oxidations- oder Reduktionsreaktionen durchführt oder die Verbindungen der Formel (I) auf übliche Weise in Salze überführt.

Die Verbindungen der Formel (I) können nach üblichen Methoden in andere Verbindungen der Formel (I) gemäß vorausgehender Definition umgewandelt werden, beispielsweise durch nucleophile Substitution (z.B. R⁵: Cl → OC₂H₅, SCH₃) oder durch Oxidation (z.B. R⁵: CH₂SCH₃ → CH₂S(O)CH₃).

Die Verbindungen der allgemeinen Formel (I) können prinzipiell auch wie im Folgenden schematisch dargestellt synthetisiert werden:

Umsetzung von Ketonen der allgemeinen Formel (II) - oben - mit reaktiven Derivaten der substituierten Benzoesäuren der allgemeinen Formel (III) - oben - insbesondere mit entsprechenden Carbonsäurechloriden, Carbonsäureanhydriden, Carbonsäure-cyaniden, Carbonsäure-methylestern oder -ethylestem - gegebenenfalls in Gegenwart von Reaktionshilfsmitteln, wie z.B. Triethylamin (und gegebenenfalls zusätzlich Zinkchlorid), und gegebenenfalls in Gegenwart eines Verdünnungsmittels, wie z.B. Methylenchlorid: 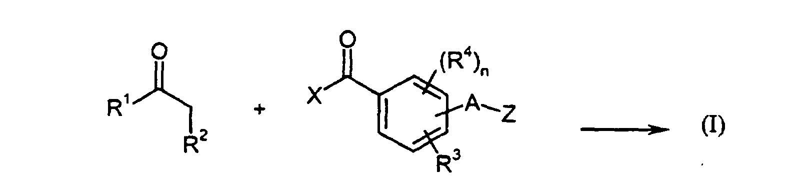 (X z.B. CN, Cl)
Verwendet man beispielsweise Ethyl-methylsulfonylmethyl-keton und 2-(3-Carboxy-5-fluor-benzyl)-5-ethyl-4-methoxy-2,4-dihydro-3H-1,2,4-triazol-3-on als Ausgangsstoffe, so kann der Reaktionsablauf beim erfindungsgemäßen Verfahren (a) durch das folgende Formelschema skizziert werden: 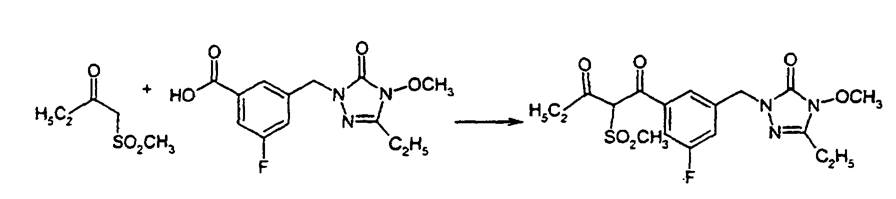 Verwendet man beispielsweise (5-Cyclopropyl-isoxazol-4-yl)-[2-(4-methyl-3-methylthio-5-oxo-4,5-dihydro-[1,2,4]-triazol-1-yl-methyl)-4-trifluormethyl-phenyl]-methanon als Ausgangsstoff, so kann der Reaktionsablauf beim erfindungsgemäßen Verfahren (b) durch das folgende Formelschema skizziert werden:

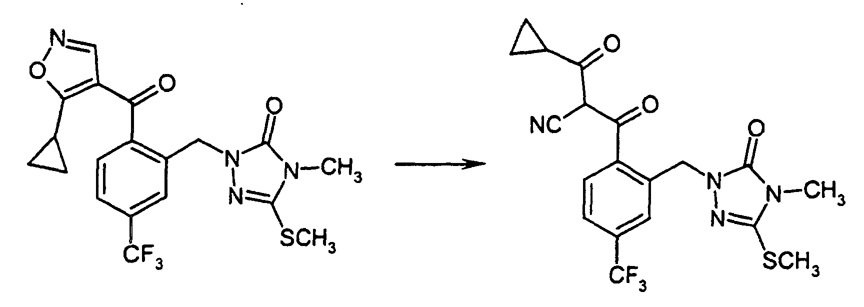

Die beim erfindungsgemäßen Verfahren (a) zur Herstellung von Verbindungen der allgemeinen Formel (I) als Ausgangsstoffe zu verwendenden Ketone sind durch die Formel (II) allgemein definiert. In der allgemeinen Formel (II) haben R¹ und R² vorzugsweise diejenigen Bedeutungen, die bereits oben im Zusammenhang mit der Beschreibung der erfindungsgemäßen Verbindungen der allgemeinen Formel (I) als bevorzugt, besonders bevorzugt, ganz besonders bevorzugt, oder am meisten bevorzugt für R¹ und R² angegeben worden sind.

Die Ausgangsstoffe der allgemeinen Formel (II) sind bekannt und/oder können nach an sich bekannten Verfahren hergestellt werden (vgl. die Herstellungsbeispiele).

Die beim erfindungsgemäßen Verfahren (a) zur Herstellung von Verbindungen der Formel (I) weiter als Ausgangsstoffe zu verwendenden substituierten Benzoesäuren sind durch die Formel (III) allgemein definiert. In der Formel (III) haben n, A, R³, R⁴ und Z vorzugsweise diejenigen Bedeutungen, die bereits vorstehend im Zusammenhang mit der Beschreibung der erfindungsgemäßen Verbindungen. der Formel (I) als bevorzugt, besonders bevorzugt, ganz besonders bevorzugt, oder am meisten bevorzugt für n, A, R³, R⁴ und Z angegeben wurden.

Die Ausgangsstoffe der allgemeinen Formel (III) sind mit Ausnahme von 2-(5-Carboxy-2,4-dichlor-phenyl)-4-difluormethyl-5-methyl-2,4-dihydro-3H-1,2,4-triazol-3-on - alias 2,4-Dichlor-5-(4-difluormethyl-4,5-dihydro-3-methyl-5-oxo-1H-1,2,4-triazol-1-yl)-benzoesäure (CAS-Reg.-Nr. 90208-77-8) und 2-(5-Carboxy-2,4-dichlor-phenyl)-4,5-dimethyl-2,4-dihydro-3H-1,2,4-triazol-3-on - alias 2,4-Dichlor-5-(4,5-dihydro-3,4-dimethyl-5-oxo-1H-1,2,4-triazol-1-yl)-benzoesäure (CAS-Reg.-Nr. 90208-76-7) - noch nicht aus der Literatur bekannt. Sie sind jedoch unter Ausnahme von 2-(5-Carboxy-2,4-dichlor-phenyl)-4-difluormethyl-5-methyl-2,4-dihydro-3H-1,2,4-triazol-3-on und 2-(5-Carboxy-2,4-dichlor-phenyl)-4,5-dimethyl-2,4-dihydro-3H-1,2,4-triazol-3-on (vgl. JP-A-58225070 - zitiert in Chem. Abstracts 100:209881, JP-A-02015069 - zitiert in Chem. Abstracts 113:23929) Gegenstand einer vorgängigen, jedoch nicht vorveröffentlichten Anmeldung (vgl. DE-A-19833360).

Man erhält die substituierten Benzoesäuren der allgemeinen Formel (III), wenn man Benzoesäurederivate der allgemeinen Formel (V), 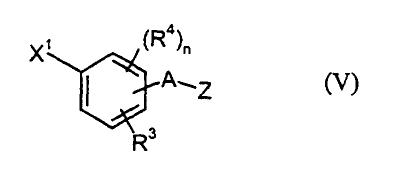 in welcher
- n, A, R³ und R⁴ und Z: die vorangehend angegebenen Bedeutungen haben, und
- X¹: für Cyano, Carbamoyl oder Alkoxycarbonyl steht,
mit Wasser, gegebenenfalls in Gegenwart eines Hydrolysehilfsmittels, wie z.B. Schwefelsäure, bei Temperaturen zwischen 50°C und 120°C umsetzt (vgl. die Herstellungsbeispiele).

Die als Vorprodukte benötigten Benzoesäurederivate der allgemeinen Formel (V) sind bekannt und/oder können nach an sich bekannten Verfahren hergestellt werden (vgl. DE-A-3839480, DE-A-4239296, EP-A-597360, EP-A-609734, DE-A-4303676, EP-A-617026, DE-A-4405614, US-A-5378681).

Man erhält die neuen substituierten Benzoesäuren der allgemeinen Formel (III) auch, wenn man Halogen(alkyl)benzoesäuren der allgemeinen Formel (VI), 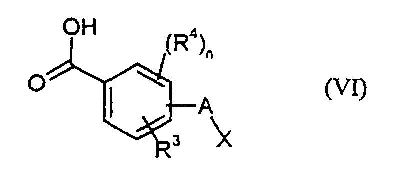 in welcher
- n, A, R³ und R⁴: die voranstehend angegebenen Bedeutungen haben und
- X: für Halogen (insbesondere Chlor oder Brom) steht,
mit Verbindungen der allgemeinen Formel (VII), 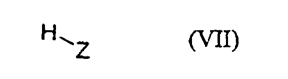 in welcher
- Z: die vorangehend angegebenen Bedeutungen hat,
gegebenenfalls in Gegenwart eines Reaktionshilfsmittel, wie z.B. Triethylamin oder Kaliumcarbonat, und gegebenenfalls in Gegenwart eines Verdünnungsmittels, wie z.B. Aceton, Acetonitril, N,N-Dimethyl-formamid oder N,N-Dimethyl-acetamid, bei Temperaturen zwischen 50°C und 200°C umsetzt (vgl. die Herstellungsbeispiele).

An Stelle der Halogen(alkyl)benzoesäuren der allgemeinen Formel (VI) können analog zur oben beschriebenen Methodik auch entsprechende Nitrile, Amide und Ester - insbesondere die Methylester oder die Ethylester - mit Verbindungen der allgemeinen Formel (VII) umgesetzt werden. Durch anschließende Hydrolyse nach üblichen Methoden, beispielsweise durch Umsetzung mit wässrig-ethanolischer Kalilauge, können dann die entsprechenden substituierten Benzoesäuren erhalten werden.

Die als Vorprodukte benötigten Halogen(alkyl)benzoesäuren der Formel (VI) - bzw. entsprechende Nitrile oder Ester - sind bekannt und/oder können nach an sich bekannten Verfahren hergestellt werden (vgl. EP-A-90369, EP-A-93488, EP-A-399732, EP-A-480641, EP-A-609798, EP-A-763524, DE-A-2126720, WO-A-93/03722, WO-A-97/38977, US-A-3978127, US-A-4837333).

Die weiter als Vorprodukte benötigten Verbindungen der allgemeinen Formel (VII) sind bekannt und/oder können nach an sich bekannten Verfahren hergestellt werden.

Das erfindungsgemäße Verfahren (a) zur Herstellung der neuen substituierten Benzoylketone der allgemeinen Formel (I) wird gegebenenfalls unter Verwendung eines oder mehrerer Reaktionshilfsmittel durchgeführt.

Als Beispiele hierfür seien Natriumcyanid, Kaliumcyanid, Acetoncyanhydrin, Imidazol, Triazol, Phosphorsäure-cyanid-diethylester, 2-Cyano-2-(trimethylsilyloxy)-propan und Trimethylsilylcyanid genannt.

Als besonders gut geeignetes Reaktionshilfsmittel seien Phosphorsäure-cyanid-diethylester und Trimethylsilylcyanid genannt.

Die erfindungsgemäßen Verfahren (a) und (b) zur Herstellung der neuen substituierten Benzoylketone der allgemeinen Formel (I) werden gegebenenfalls unter Verwendung eines (weiteren) Reaktionshilfsmittels durchgeführt. Als (weitere) Reaktionshilfsmittel für die erfindungsgemäßen Verfahren kommen im allgemeinen basische organische Stickstoffverbindungen, wie beispielsweise Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8-Diazabicyclo[5,4,0]-undec-7-en (DBU) in Betracht.

Als Verdünnungsmittel zur Durchführung der erfindungsgemäßen Verfahren (a) und (b) kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan oder 1,2-Dichlor-ethan; Ether, wie Diethylether, Diisopropylether, Dioxan, Tetrahydrofuran, Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid.

Die Reaktionstemperaturen können bei der Durchführung der erfindungsgemäßen Verfahren (a) und (b) in einem größeren Bereich variiert werden. Im allgemeinen arbeitet man bei Temperaturen zwischen 0°C und 150°C, vorzugsweise zwischen 10°C und 120°C.

Die erfindungsgemäßen Verfahren (a) und (b) werden im allgemeinen unter Normaldruck durchgeführt. Es ist jedoch auch möglich, die erfindungsgemäßen- Verfahren unter erhöhtem oder vermindertem Druck - im allgemeinen zwischen 0,1 bar und 10 bar - durchzuführen.

Zur Durchführung der erfindungsgemäßen Verfahren (a) und (b) werden die Ausgangsstoffe im allgemeinen in angenähert äquimolaren Mengen eingesetzt. Es ist jedoch auch möglich, eine der Komponenten in einem größeren Überschuss zu verwenden. Die Umsetzung wird im allgemeinen in einem geeigneten Verdünnungsmittel in Gegenwart eines Dehydratisierungsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera, Aegilops, Phalaris.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise

Acetochlor, Acifluorfen(-sodium), Aclonifen, Alachlor, Alloxydim(-sodium), Ametryne, Amicarbazone, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzobicyclon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caioxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron, Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Picolinafen, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin, Triflusulfuron, Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

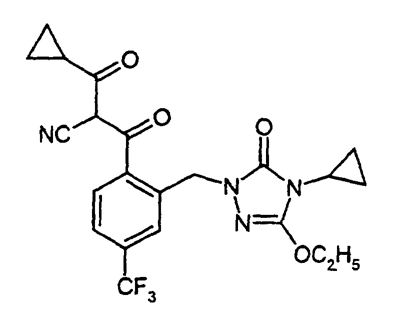

2,38 g (6,4 mMol) 4-Cyclopropyl-5-ethoxy-2-(2-carboxy-5-trifluormethyl-benzyl)-2,4-dihydro-3H-1,2,4-triazol-3-on werden in 20 ml N,N-Dimethyl-formamid vorgelegt und bei Raumtemperatur (ca. 20°C) unter Rühren nacheinander mit 0,7 g (6,4 mMol) Cyanomethyl-cyclopropyl-keton, 2,7 ml (19 mMol) Triethylamin und 1,04 g (6,4 mMol) Phosphorsäure-cyanid-diethylester versetzt. Die Reaktionsmischung wird zwei Tage bei Raumtemperatur gerührt, auf etwa die doppelte Menge Wasser gegossen, mit 2N-Salzsäure angesäuert und mit Methylenchlorid geschüttelt. Die organische Phase wird abgetrennt, mit 2N-Salzsäure gewaschen, mit Natriumsulfat getrocknet und filtriert. Das Filtrat wird eingeengt und der Rückstand säulenchromatografisch (Kieselgel, Hexan/Essigsäureethylester, Vol.: 4/1) gereinigt.

Man erhält 1,18 g (40% der Theorie) 3-Cyclopropyl-2-{2-[4-(cyclopropyl-3-ethoxy-5-oxo-4,5-dihydro-1H-1,2,4-triazol-1-yl)-methyl]-4-trifluormethyl-benzoyl}-3-oxopropannitril als amorphes Produkt.

logP (bei pH=2 bestimmt): 3,35.

Analog zu Beispiel 1 sowie entsprechend der allgemeinen Beschreibung des erfindungsgemäßen Herstellungsverfahrens können beispielsweise auch die in den nachstehenden Tabellen 1 und la aufgeführten Verbindungen der allgemeinen Formel (I) - bzw. der Formeln (IA-3), (IB-3), (IC-3) hergestellt werden.

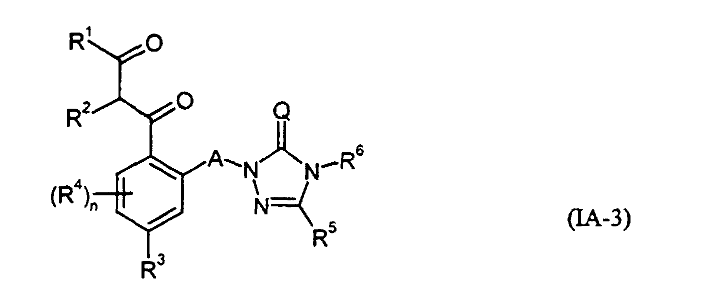

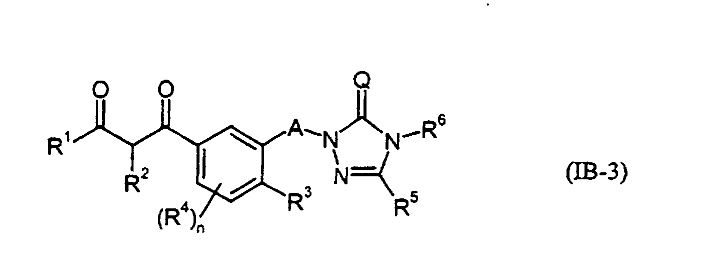

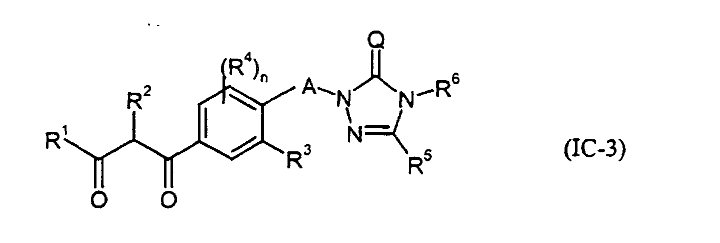

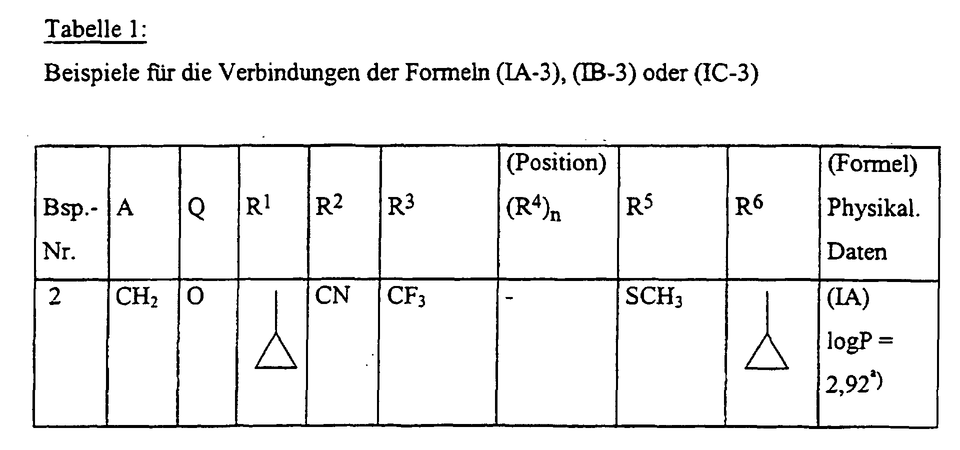

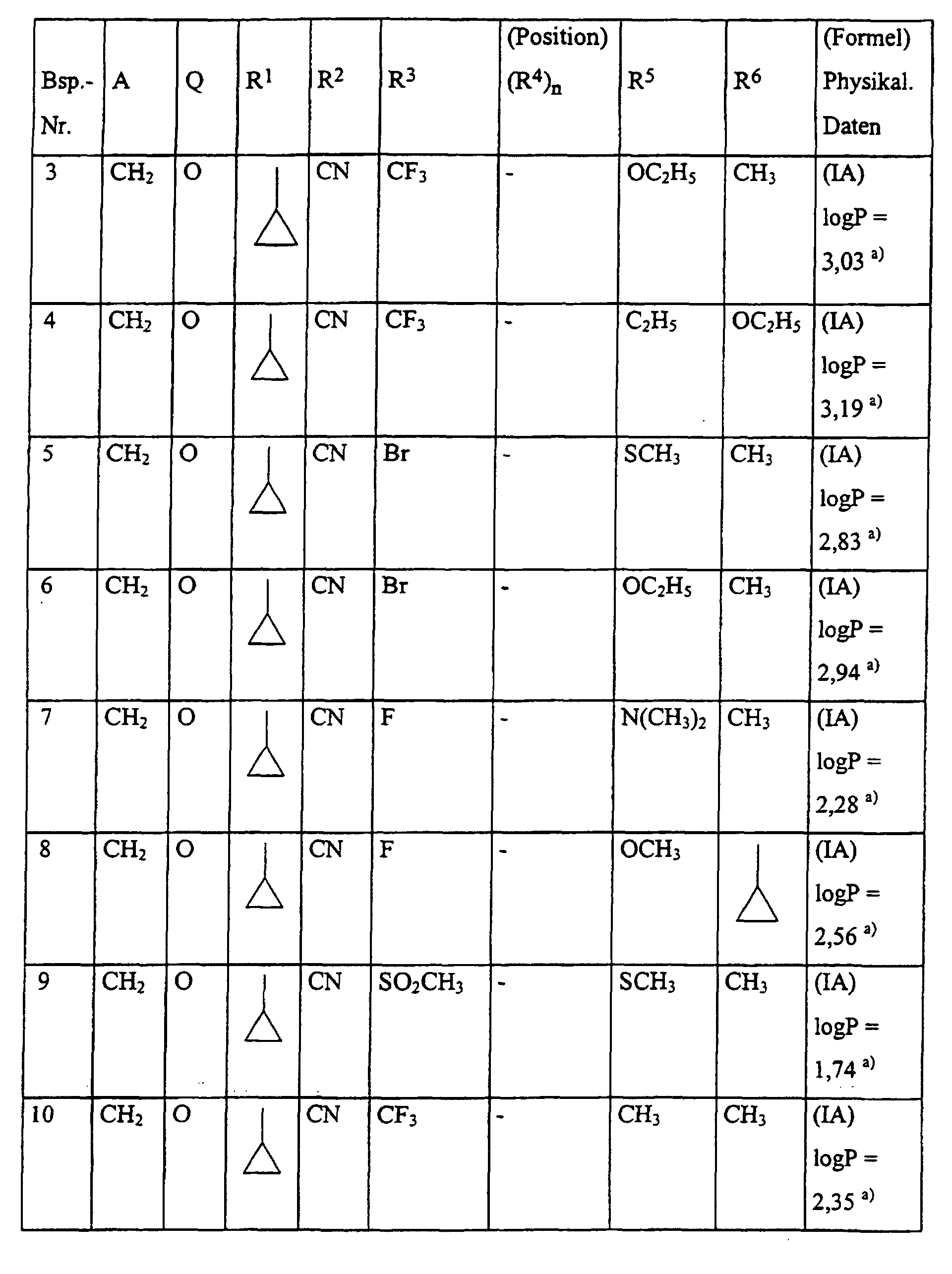

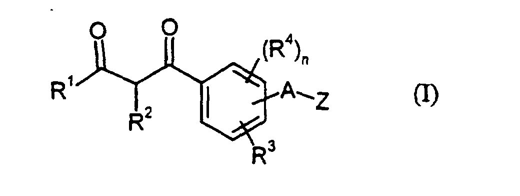

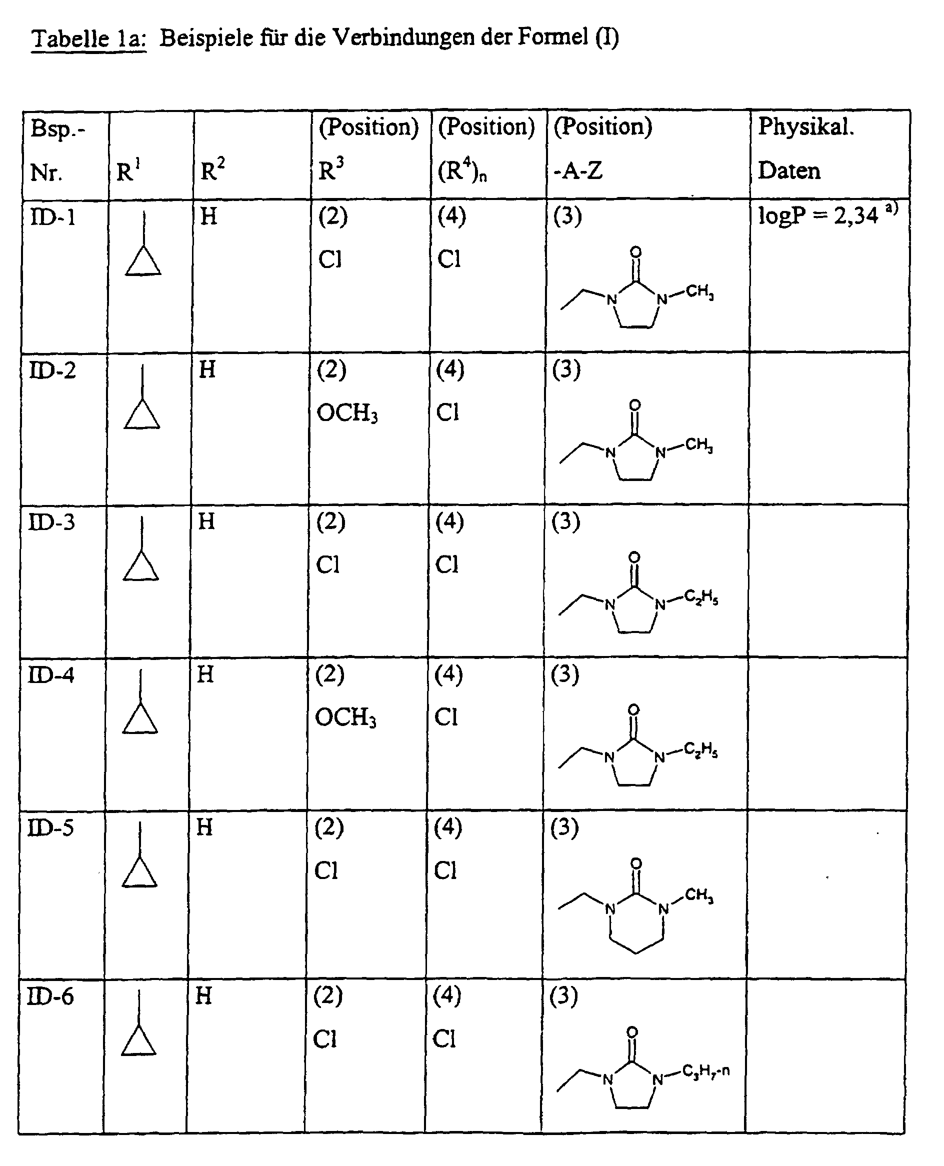

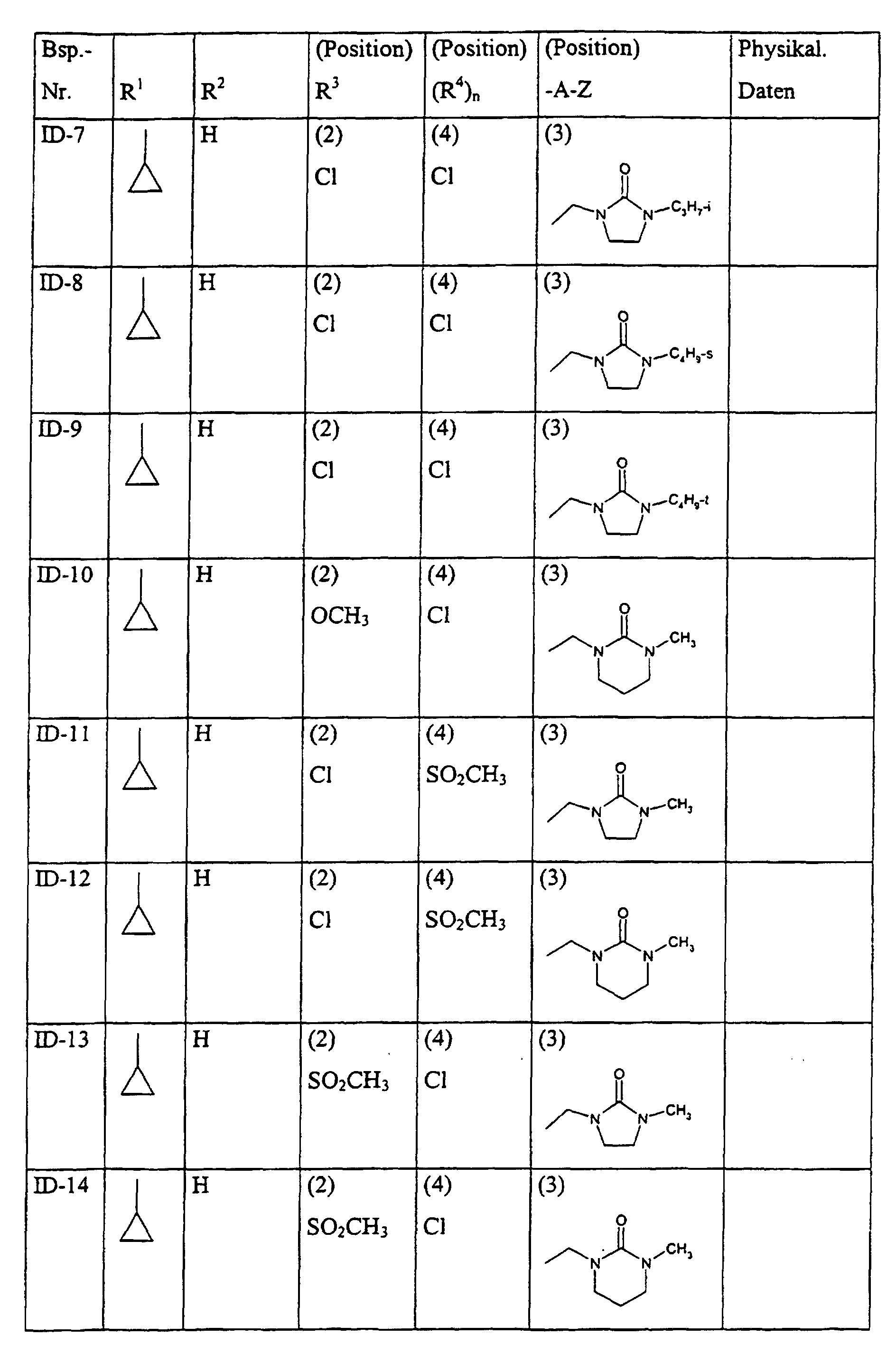

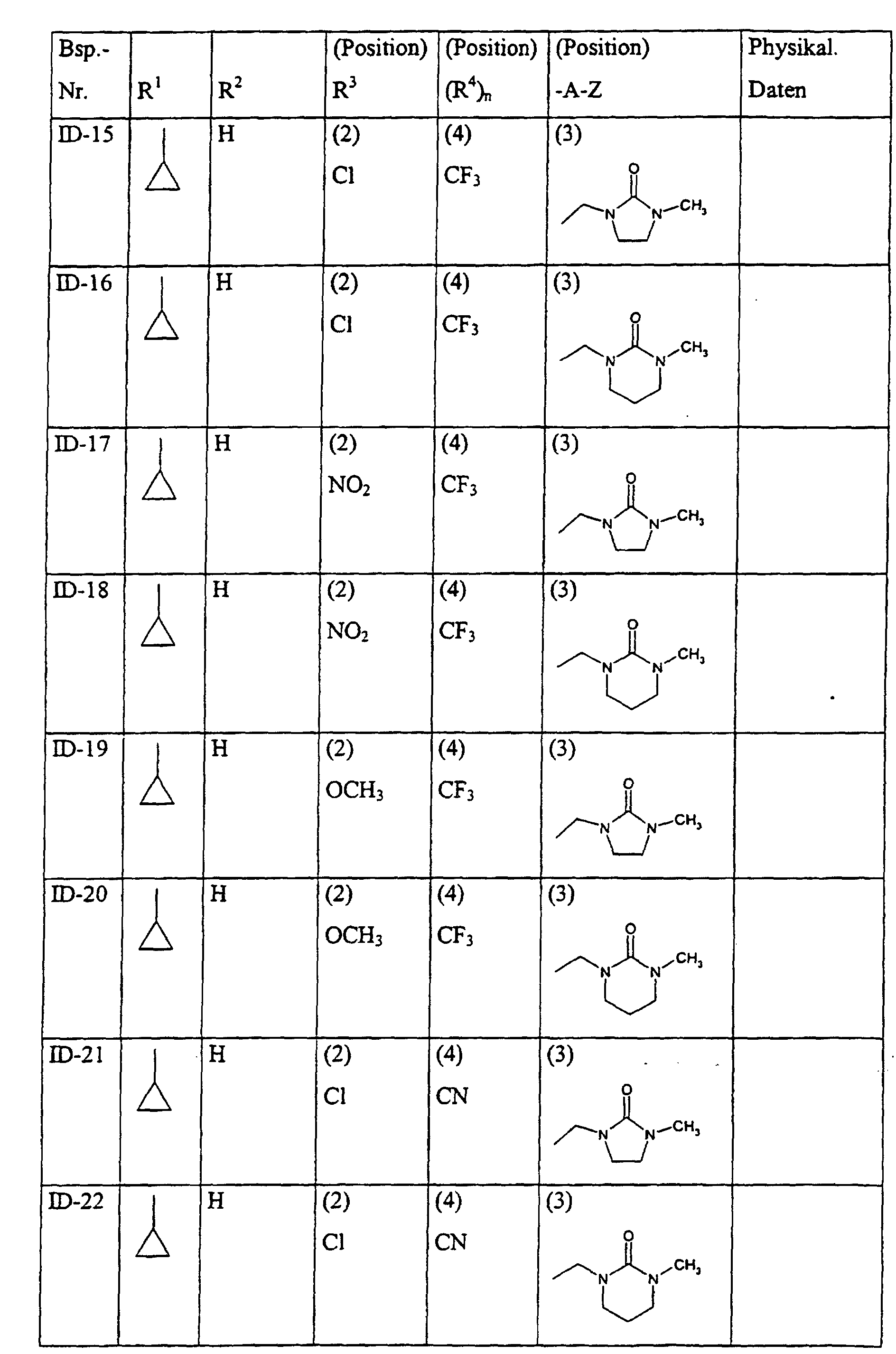

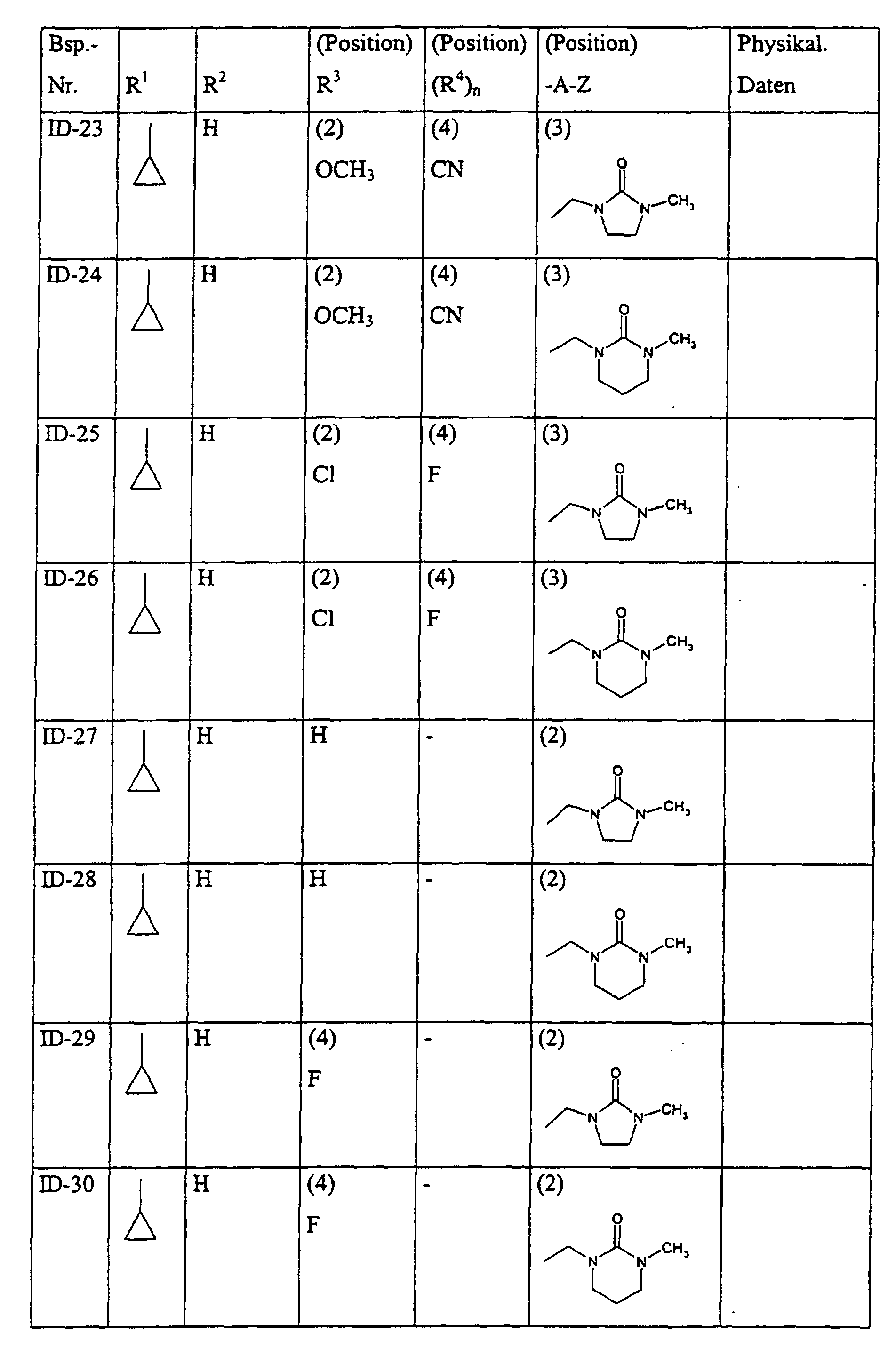

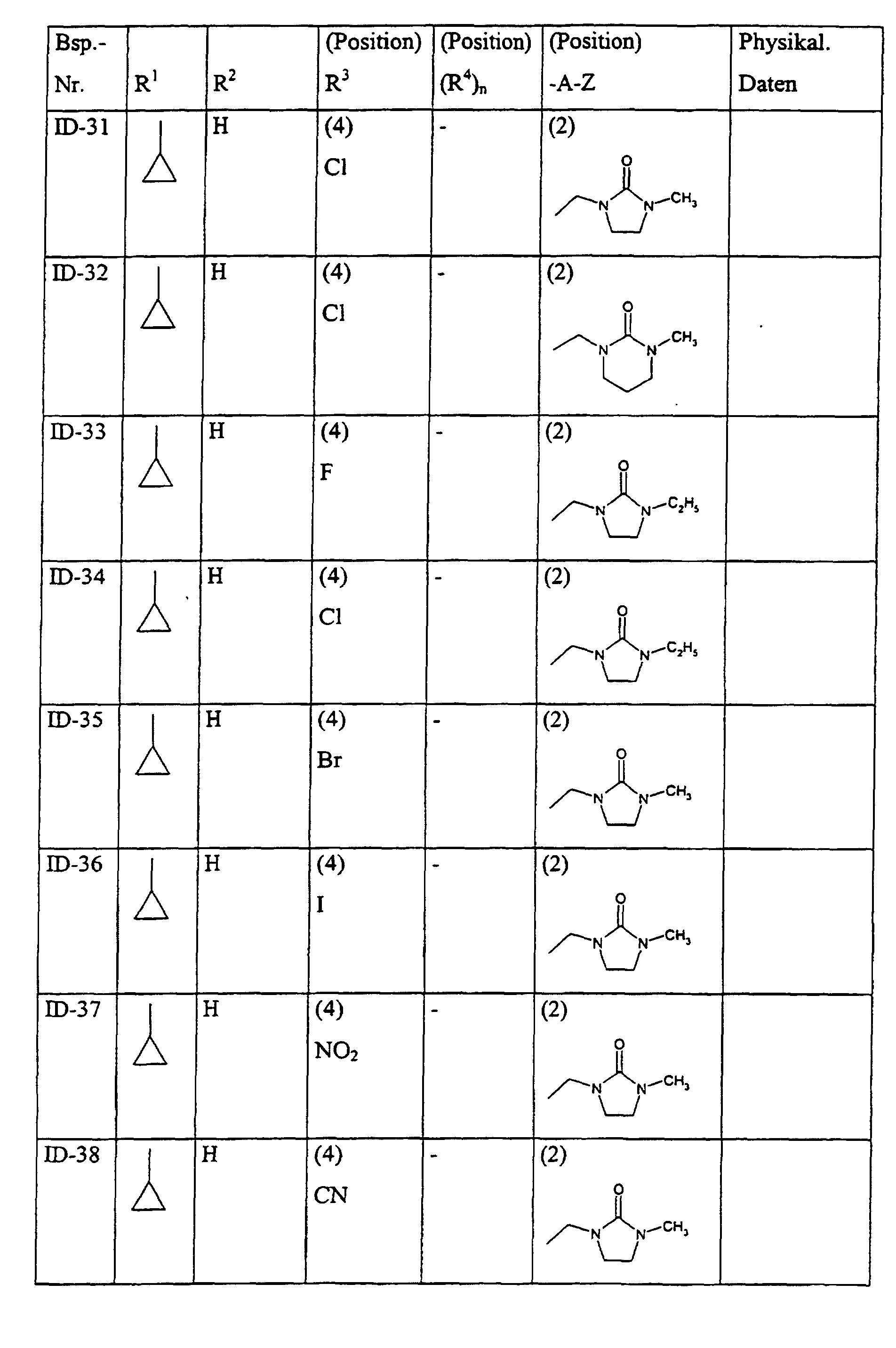

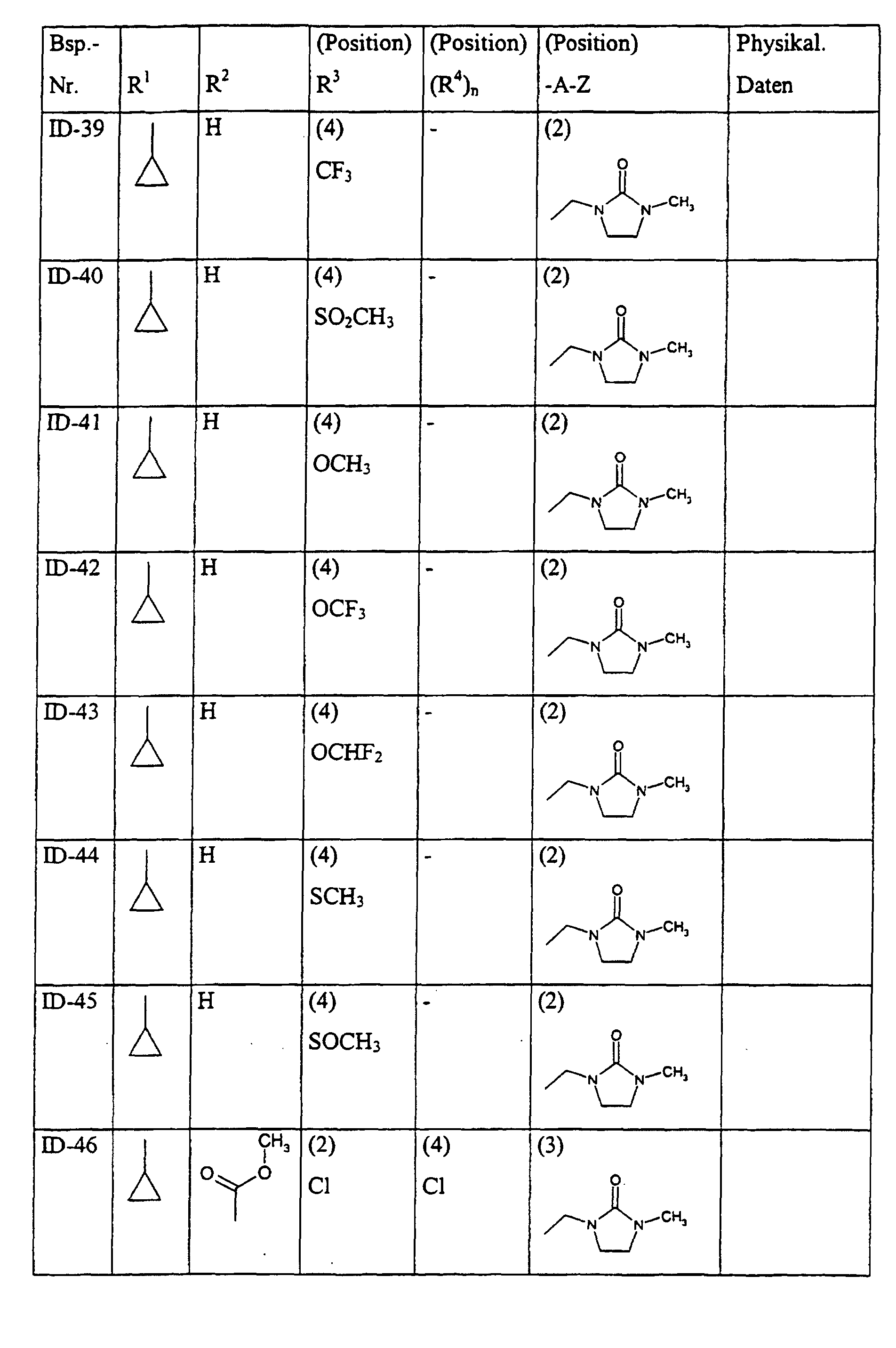

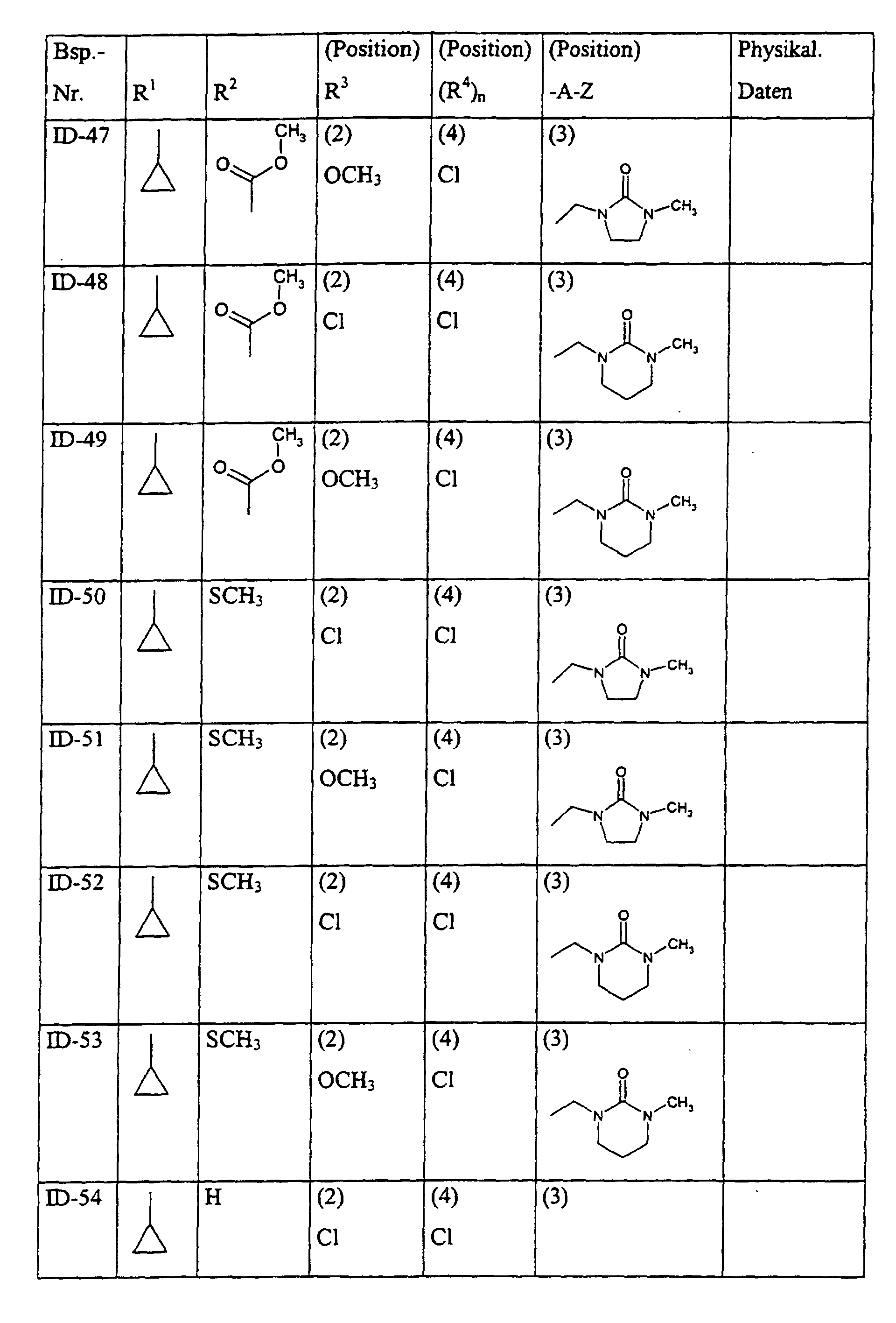

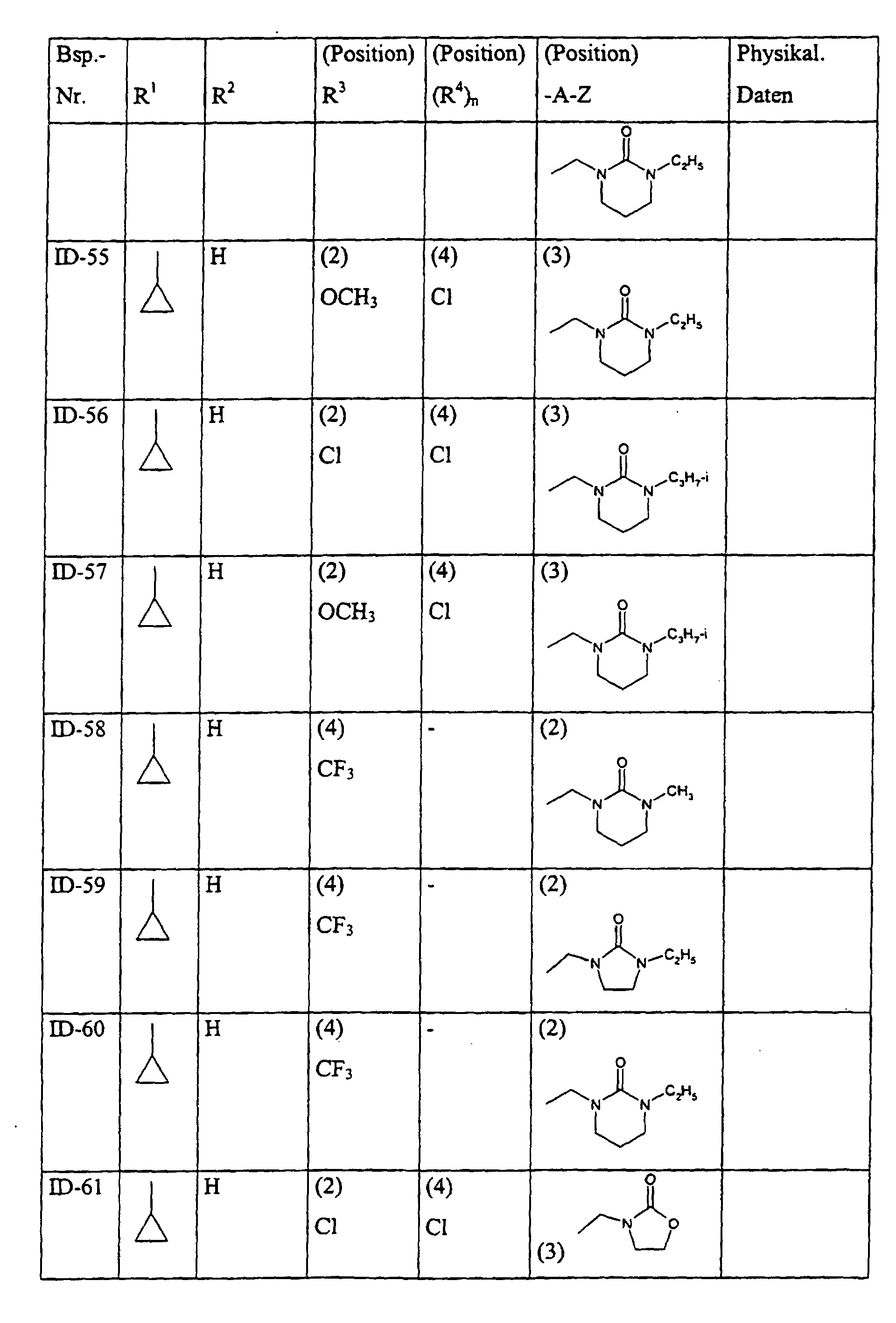

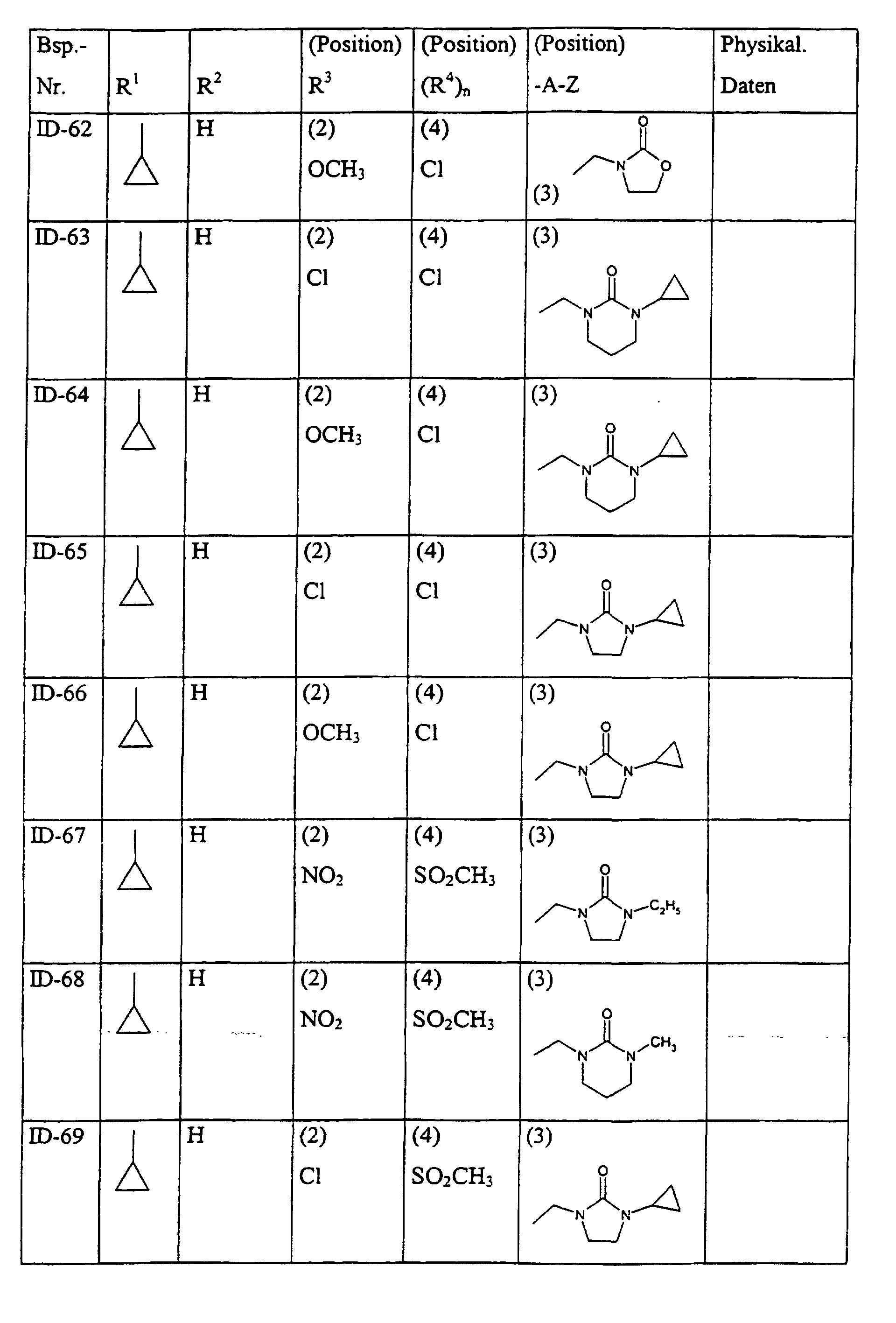

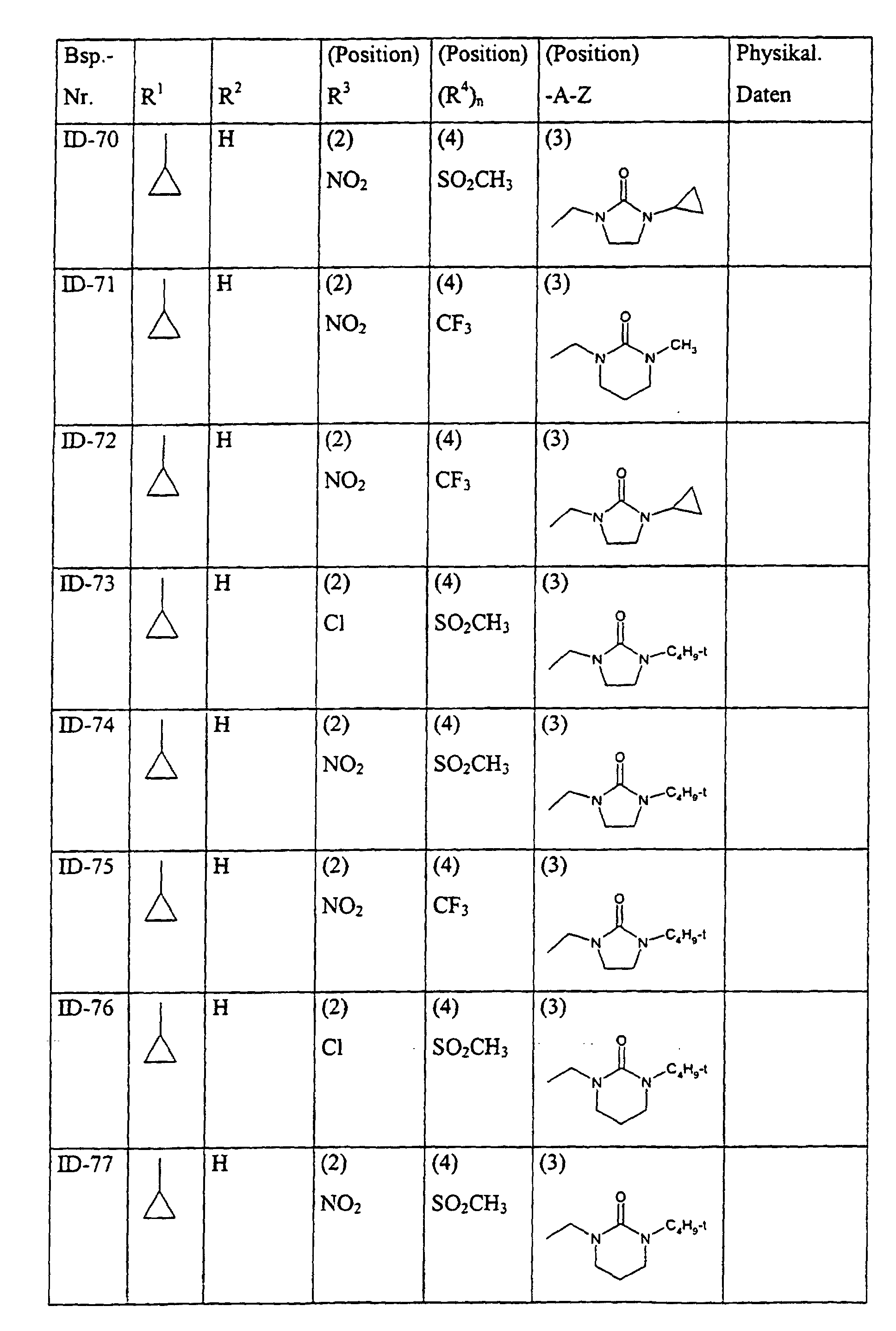

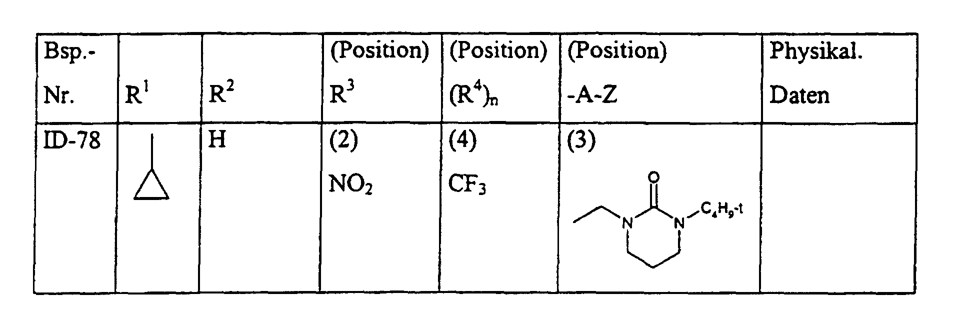

Die Bestimmung der in Tabelle 1 bzw. la angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1% wässrige Phosphorsäure, Acetonitril; linearer Gradient von 10% Acetonitril bis 90% Acetonitril - entspre-. chende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Ausgangsstoffe der Formel (II):

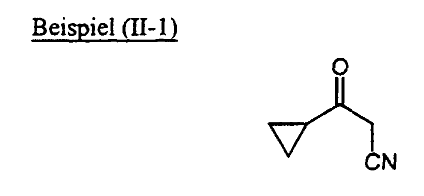

100 ml einer 1,6-molaren Lösung von Butyllithium in Hexan werden bei -60°C zu 30 ml Tetrahydrofuran gegeben. Bei -60°C werden dann nacheinander 6,6 g (0,16 Mol) Acetonitril und 14,6 g (0,15 Mol) Cyclopropancarbonsäure-methylester dazu gegeben. Die hierbei gebildete weiße Suspension wird nach Entfernen des Kühlbades und nach Erreichen der Raumtemperatur zu etwa der gleichen Menge 2N-Salzsäure gegossen und dann dreimal mit Methylenchlorid extrahiert. Die organischen Extraktionslösungen werden vereinigt, mit Natriumsulfat getrocknet und filtriert. Vom Filtrat wird das Lösungsmittel im Wasserstrahlvakuum sorgfältig abdestilliert und der Rückstand wird bei stärker vermindertem Druck destilliert.

Man erhält 7,5 g (46% der Theorie) Cyanomethyl-cyclopropyl-keton vom Siedepunkt 56°C (bei 0,8 mbar).

### Ausgangsstoffe der Formel (III):

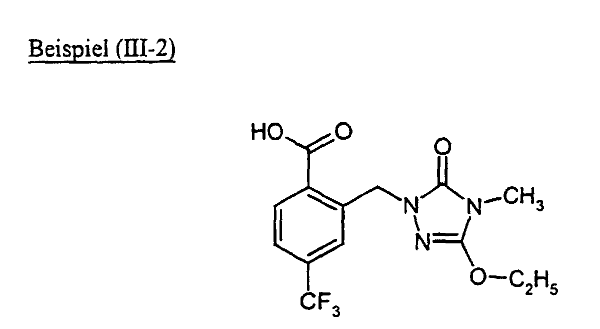

2 g (4,9 mMol) 5-Brom-4-methyl-2-(2-ethoxycarbonyl-5-trifluormethyl-benzyl)-2,4-dihydro-3H-1,2,4-triazol-3-on (vgl. Beispiel IV-1) werden in 30 ml 10%iger ethanolischer Kalilauge gelöst und 2 Stunden unter Rückfluss erhitzt. Das Reaktionsgemisch wird im Wasserstrahlvakuum eingeengt, in 20 ml Wasser aufgenommen und mit verdünnter Salzsäure angesäuert. Der ausfallende Feststoff wird filtriert und getrocknet.

Man erhält 1,2 g (71% der Theorie) 5-Ethoxy-4-methyl-2-(2-carboxy-5-trifluormethyl-benzyl)-2,4-dihydro-3H-1,2,4-triazol-3-on als festes Produkt.
logP: 2,18^{a)}

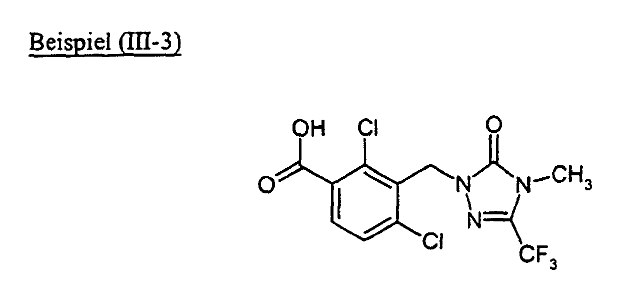

13,4 g (35 mMol) 4-Methyl-5-trifluormethyl-2-(2,6-dichlor-3-methoxycarbonylbenzyl)-2,4-dihydro-3H-1,2,4-triazol-3-on werden in 60 ml 1,4-Dioxan vorgelegt und eine Lösung von 1,54 g (38,5 mMol) Natriumhydroxid in 20 ml Wasser wird bei Raumtemperatur langsam eindosiert. Die Reaktionsmischung wird 150 Minuten bei 60°C gerührt und anschließend im Wasserstrahlvakuum eingeengt. Der Rückstand wird in 100 ml Wasser gelöst und durch Zugabe von konz. Salzsäure wird der pH-Wert der Lösung auf 1 eingestellt. Das hierbei kristallin angefallene Produkt wird durch Absaugen isoliert.

Man erhält 11,7 g (90% der Theorie) 4-Methyl-5-trifluormethyl-2-(2,6-dichlor-3-carboxy-benzyl)-2,4-dihydro-3H-1,2,4-triazol-3-on vom Schmelzpunkt 207°C.

Analog zu den Beispielen (III-1) bis (III-3) können beispielsweise auch die in der nachstehenden Tabelle 2 aufgeführten Verbindungen der allgemeinen Formel (III) hergestellt werden.

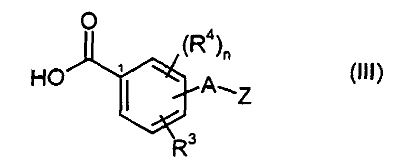

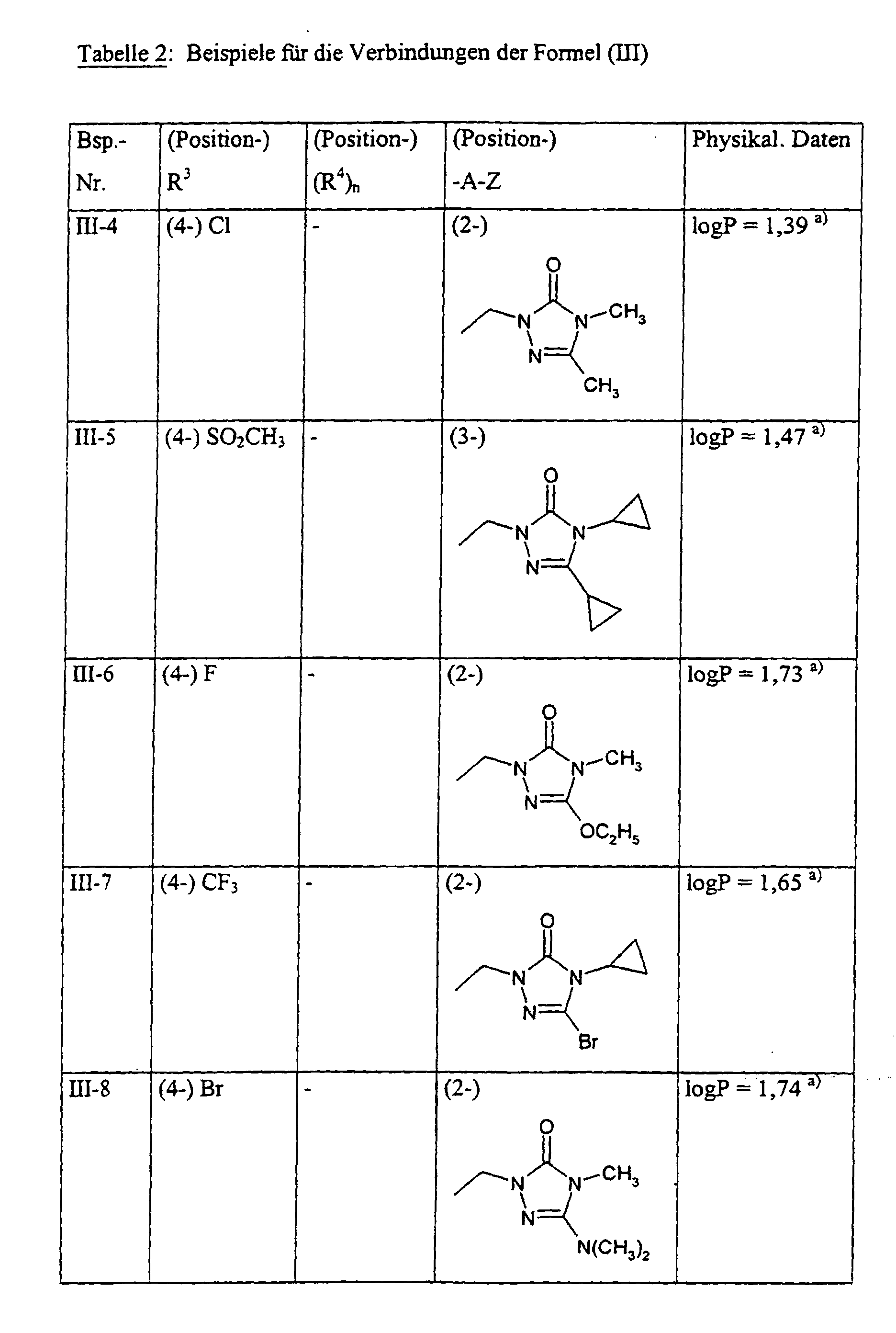

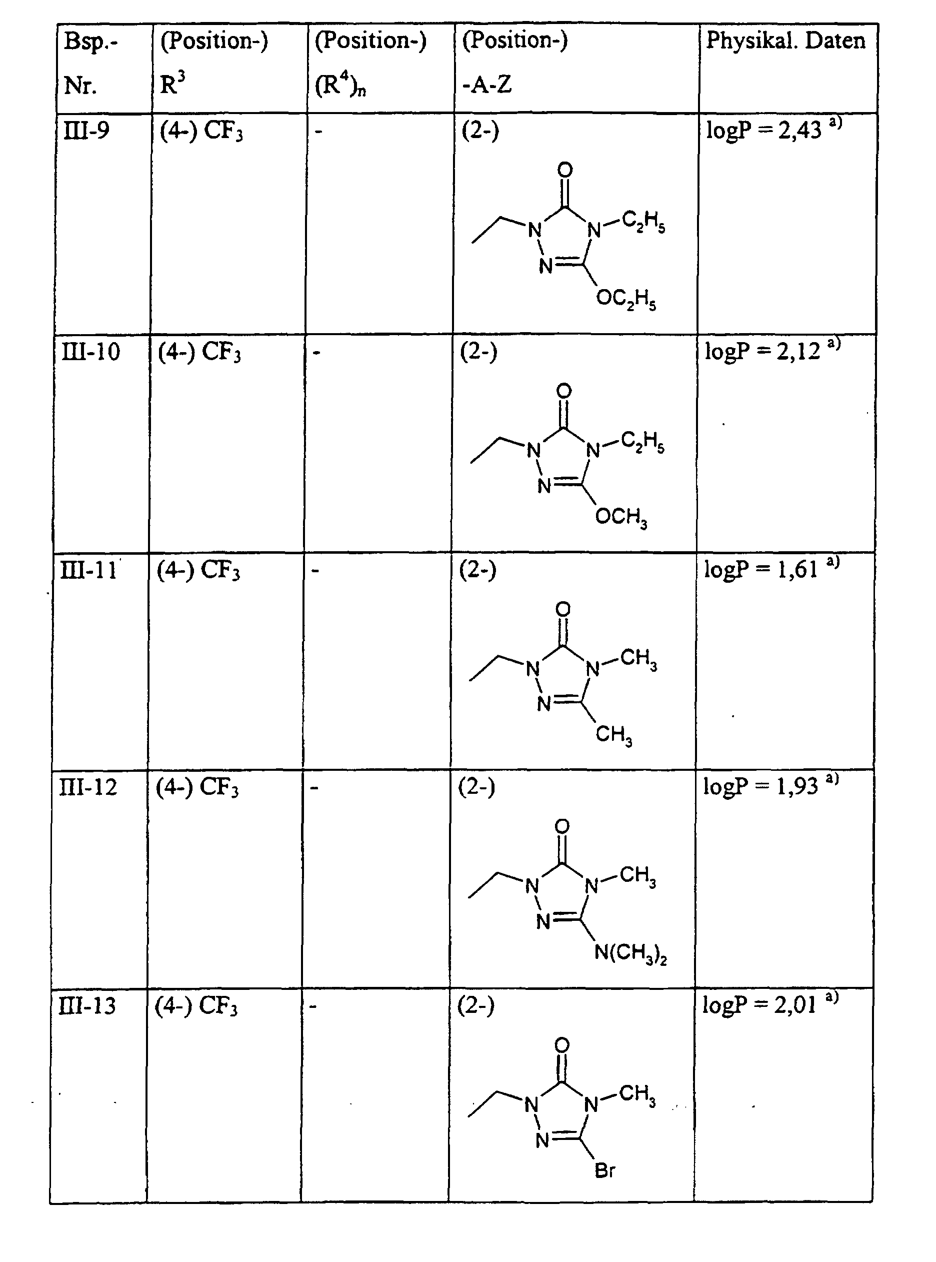

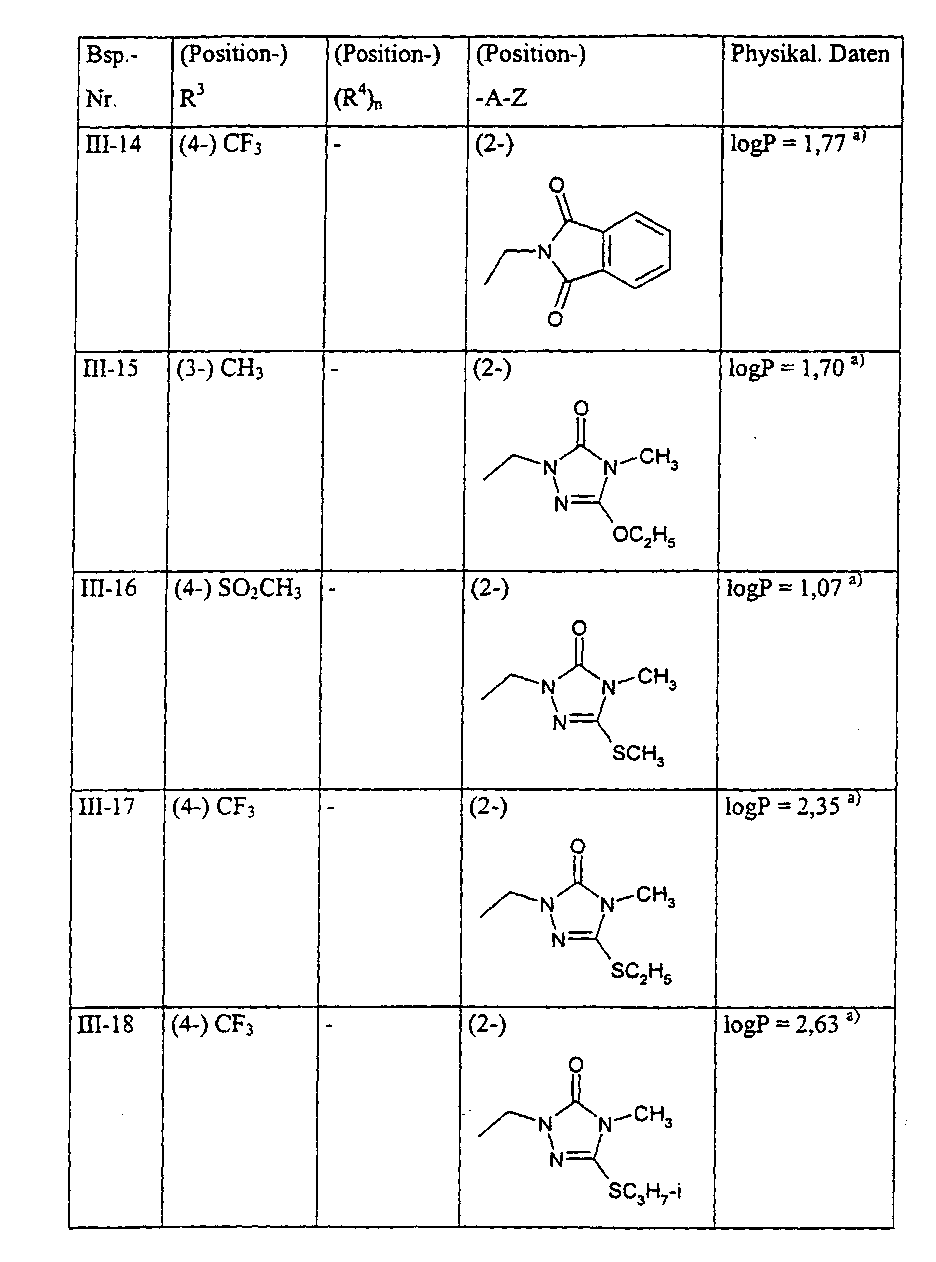

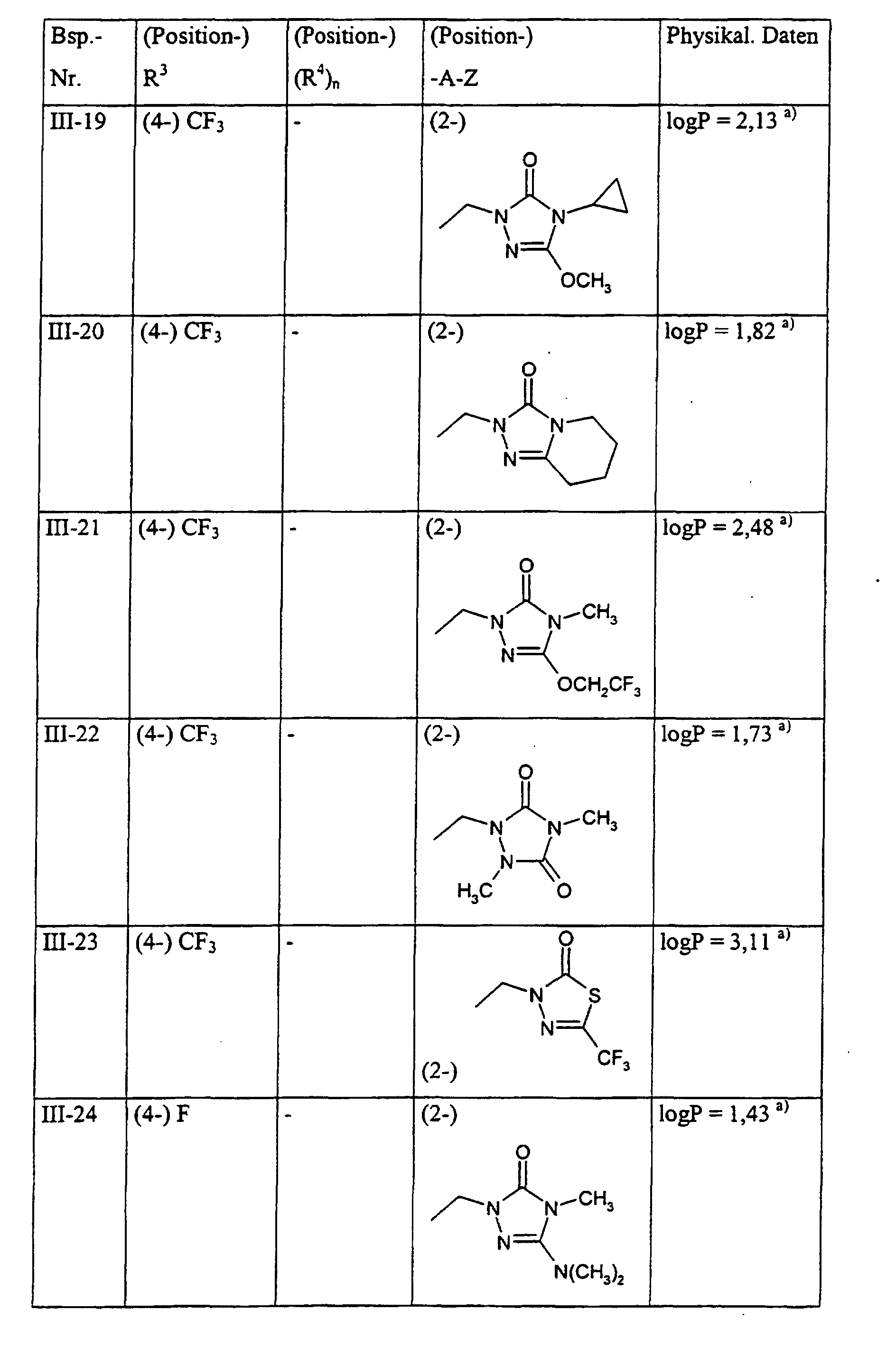

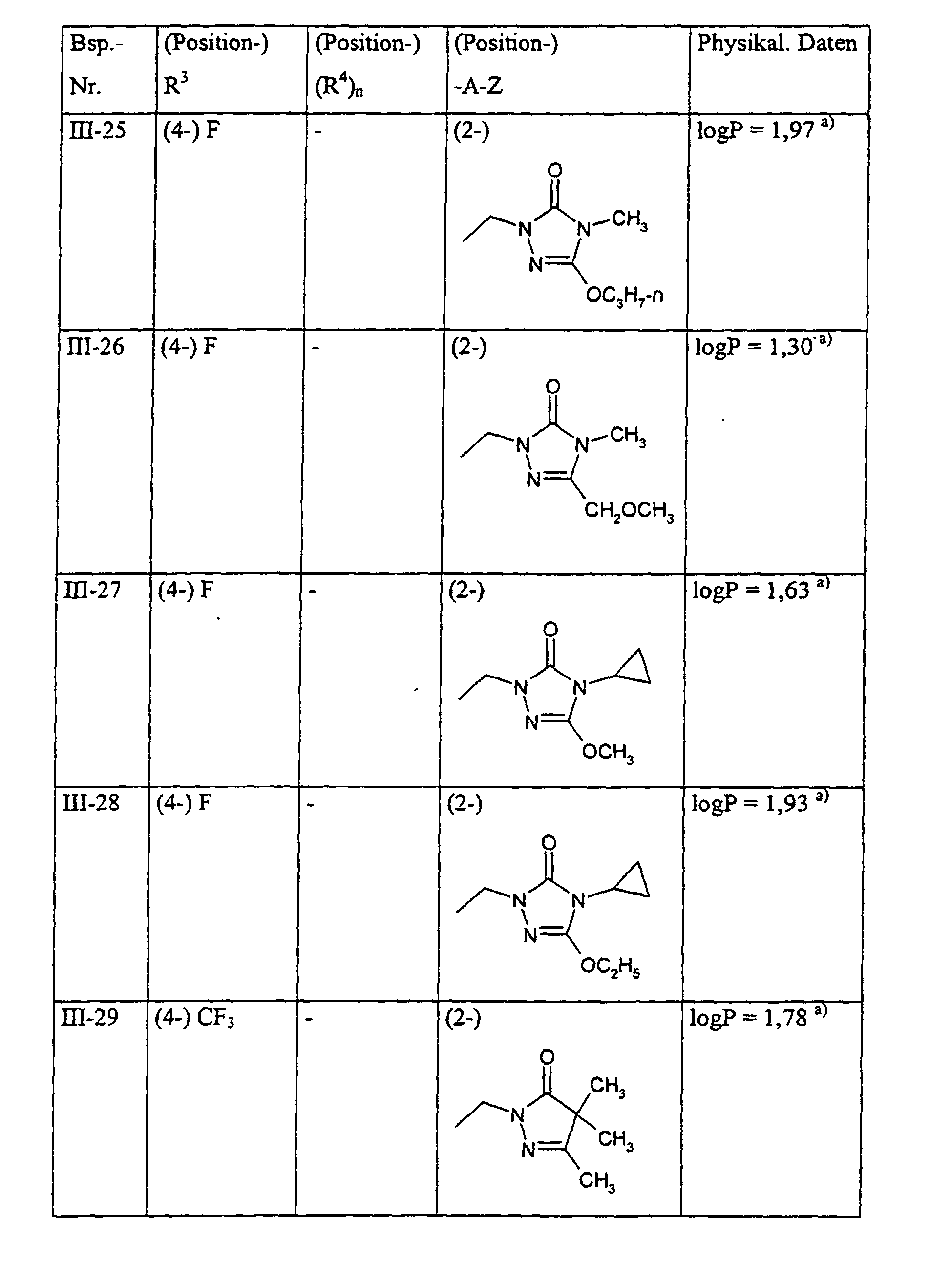

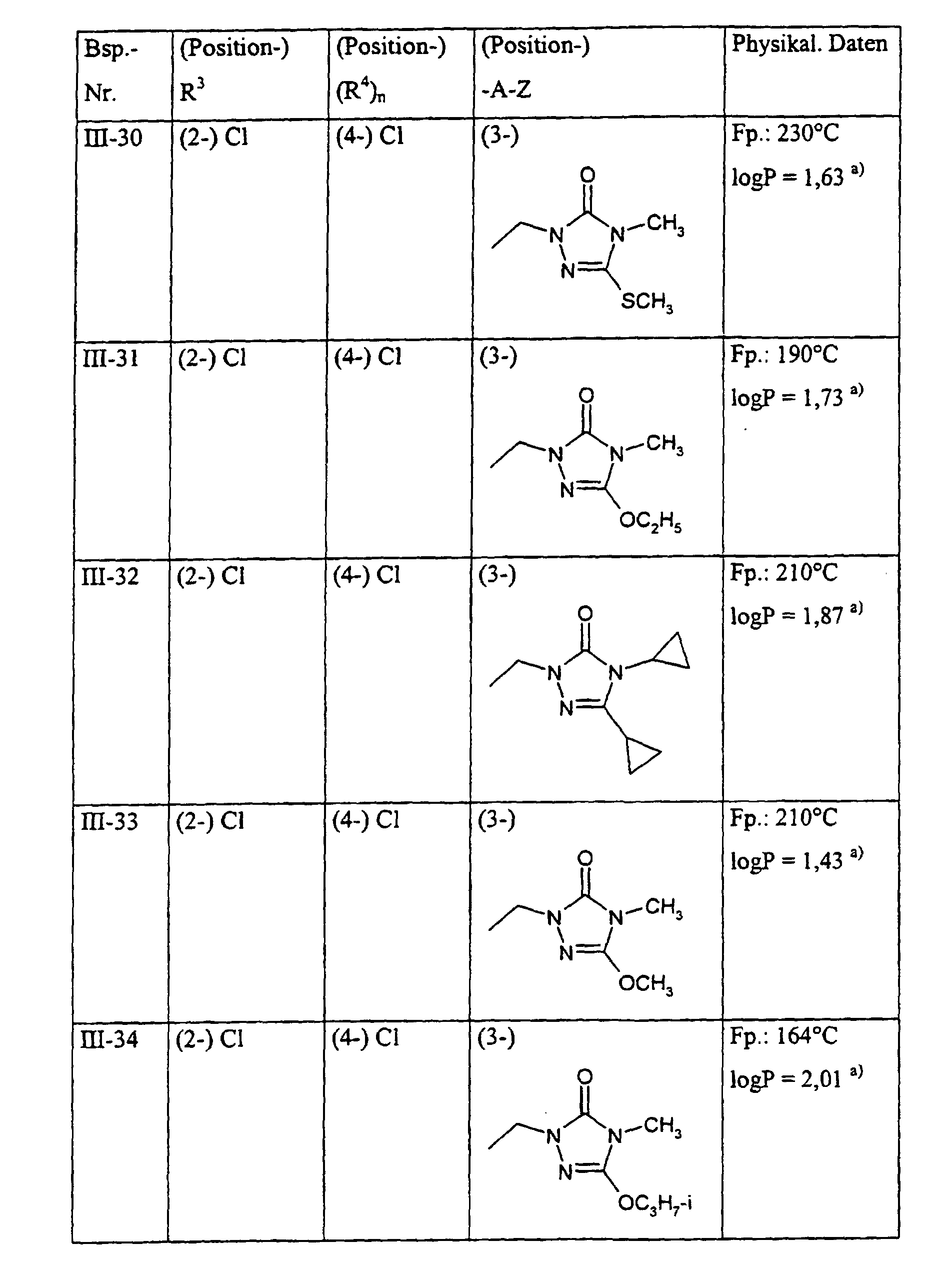

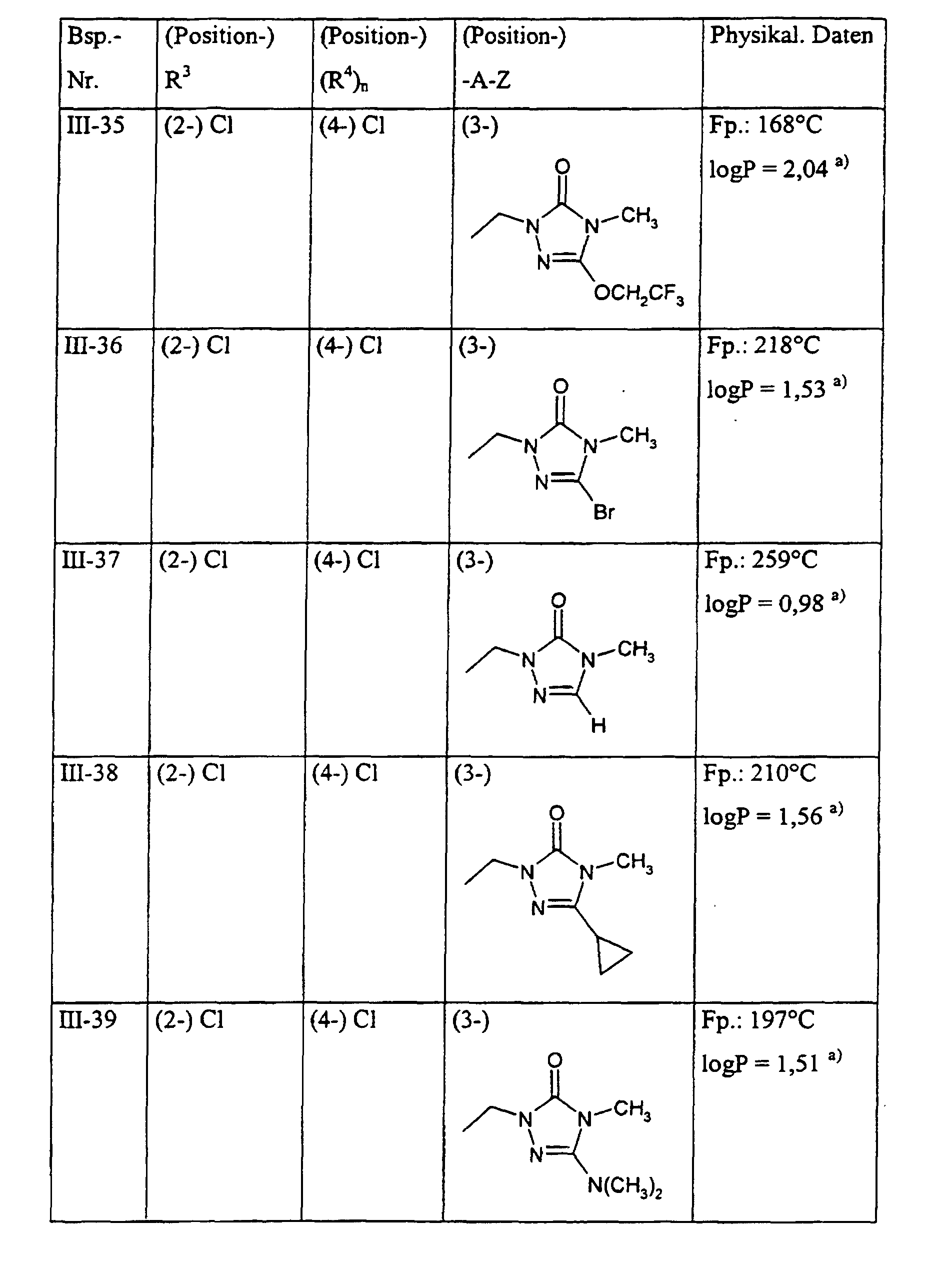

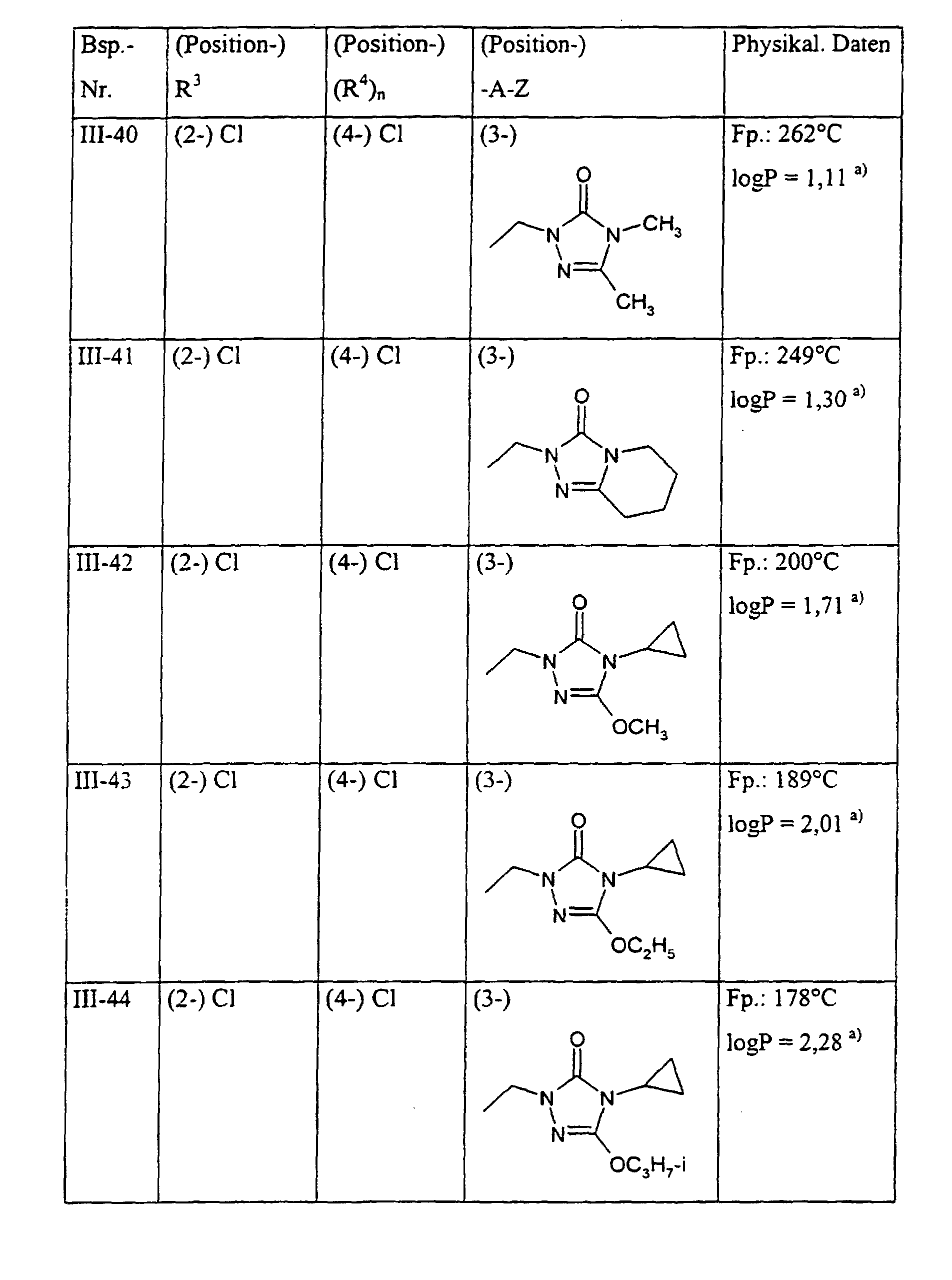

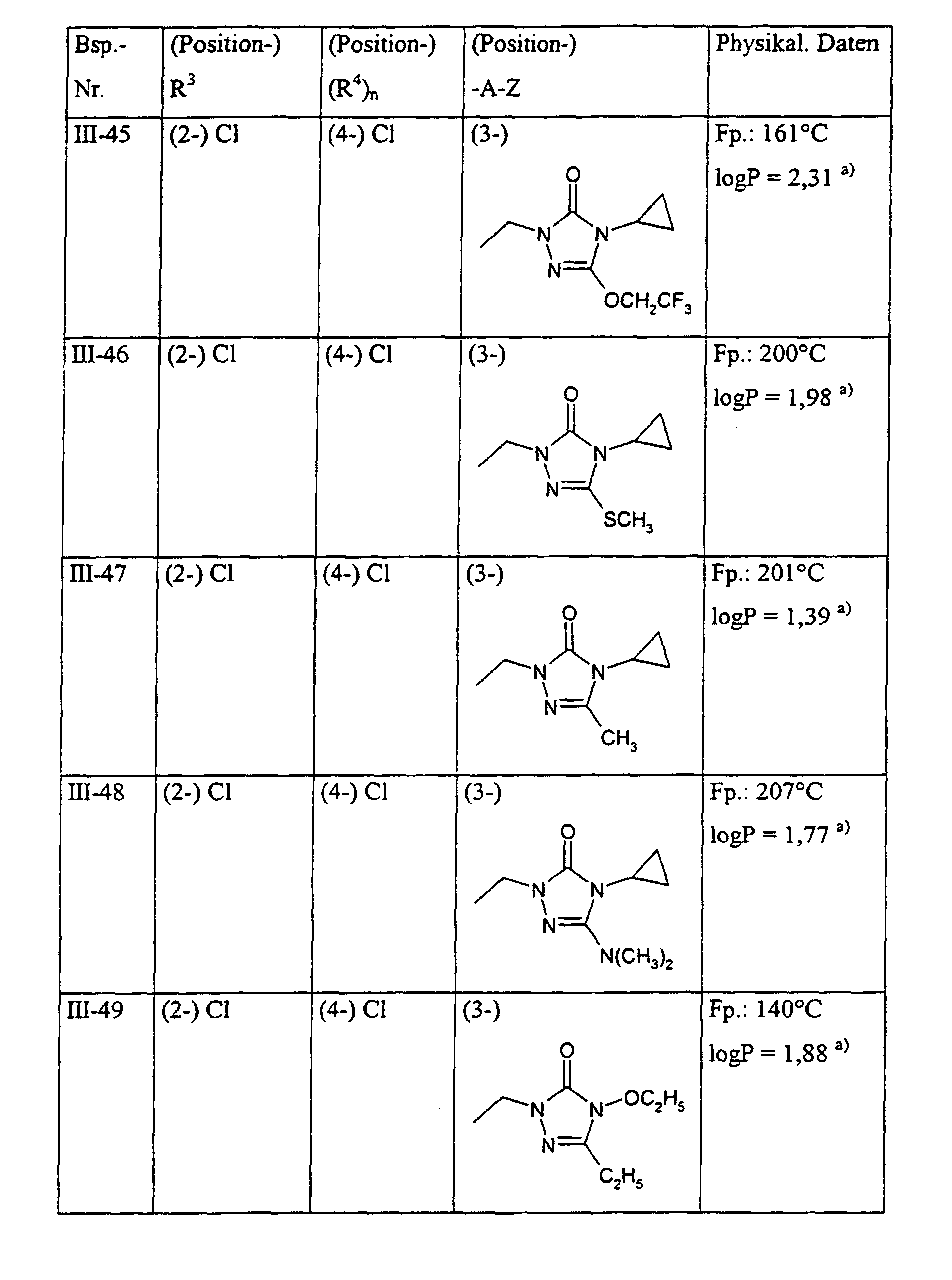

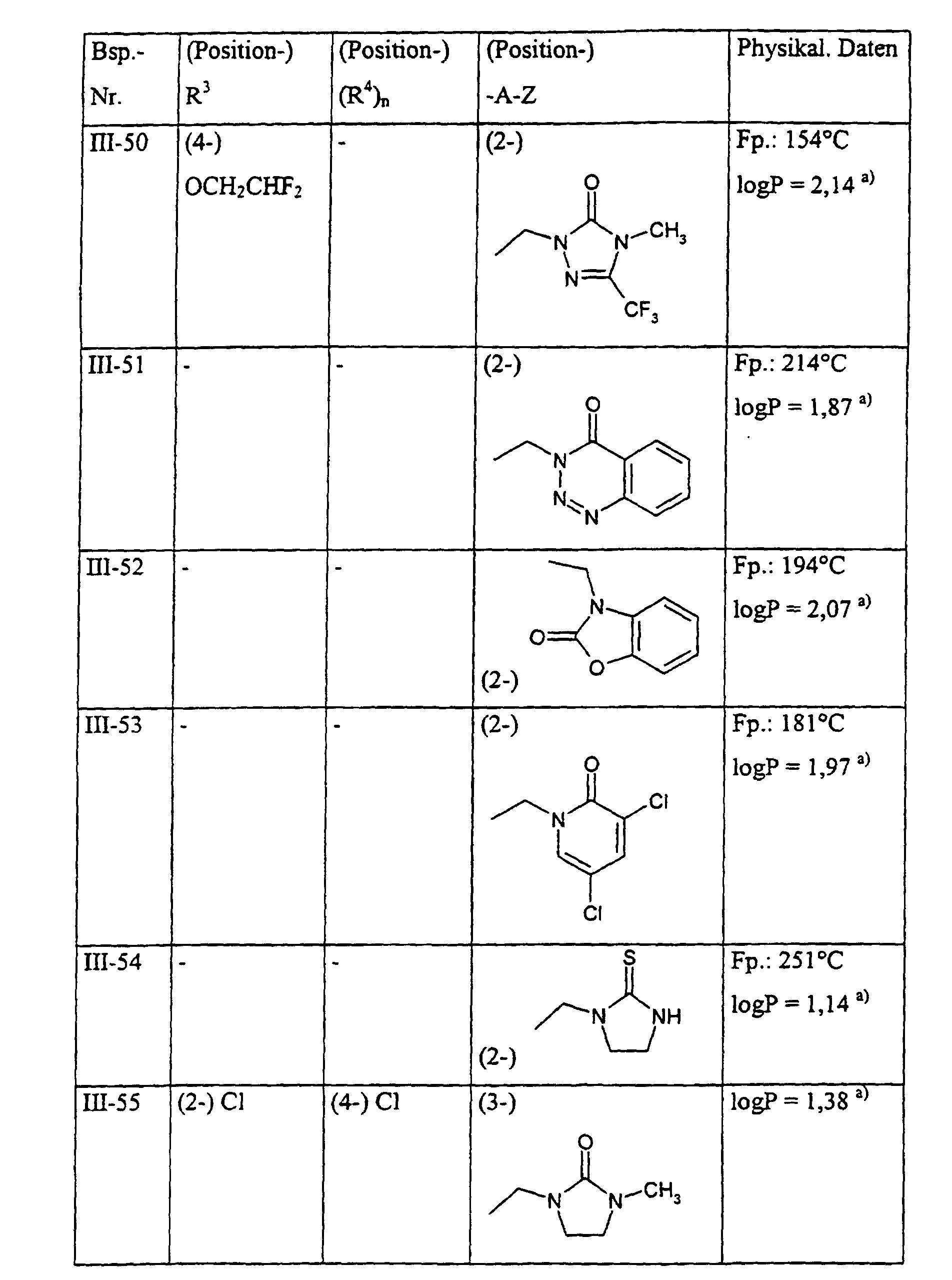

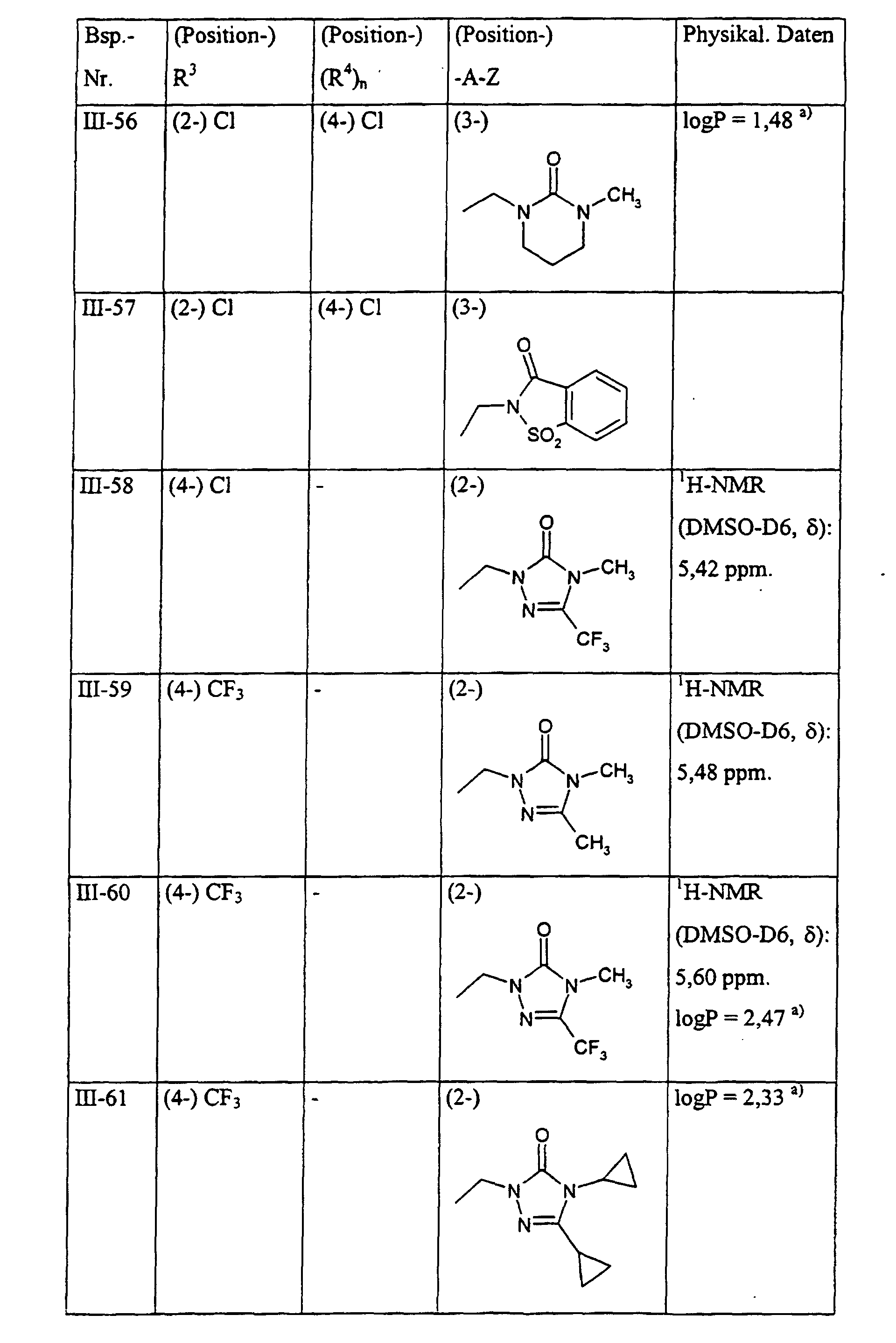

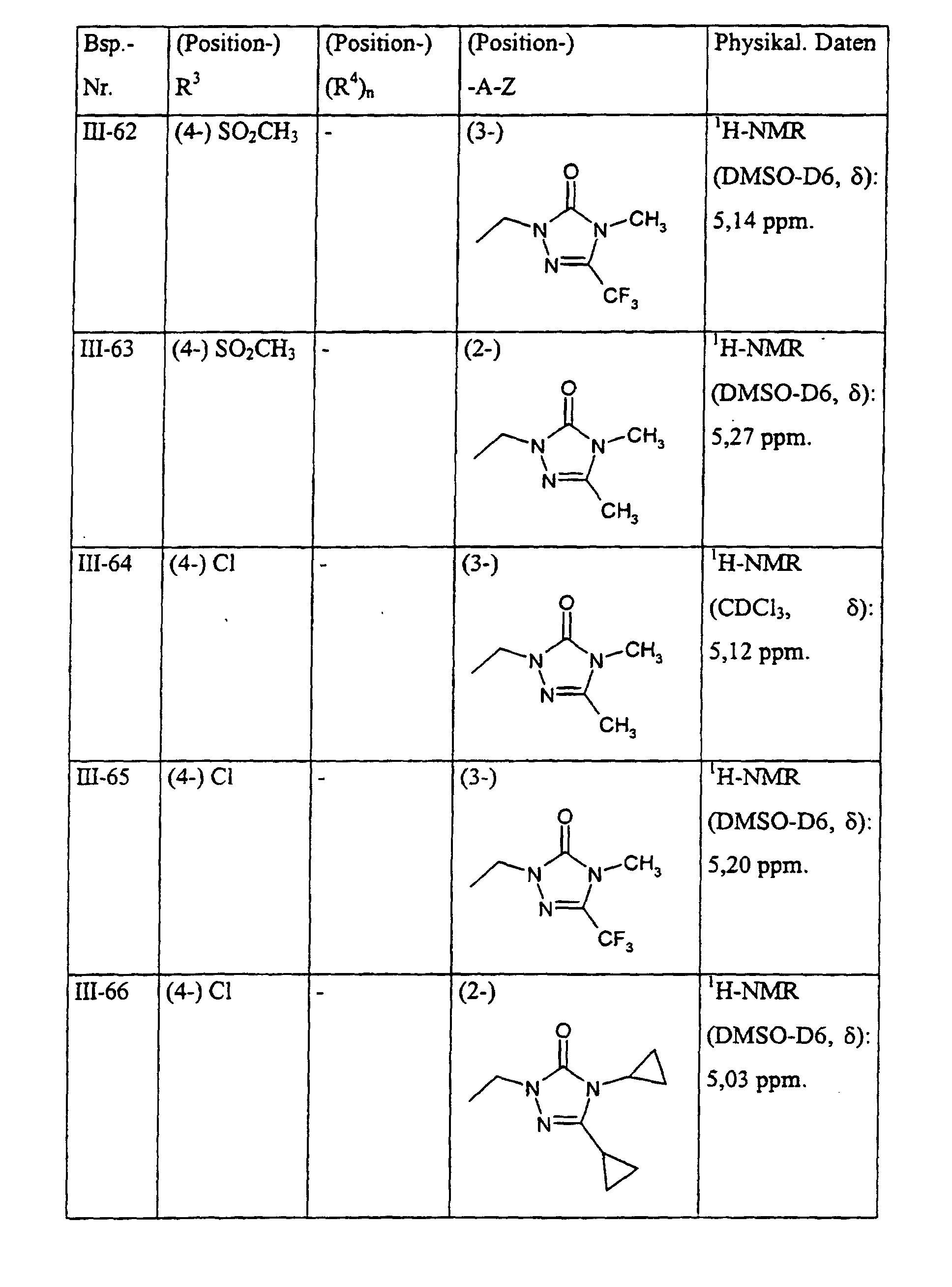

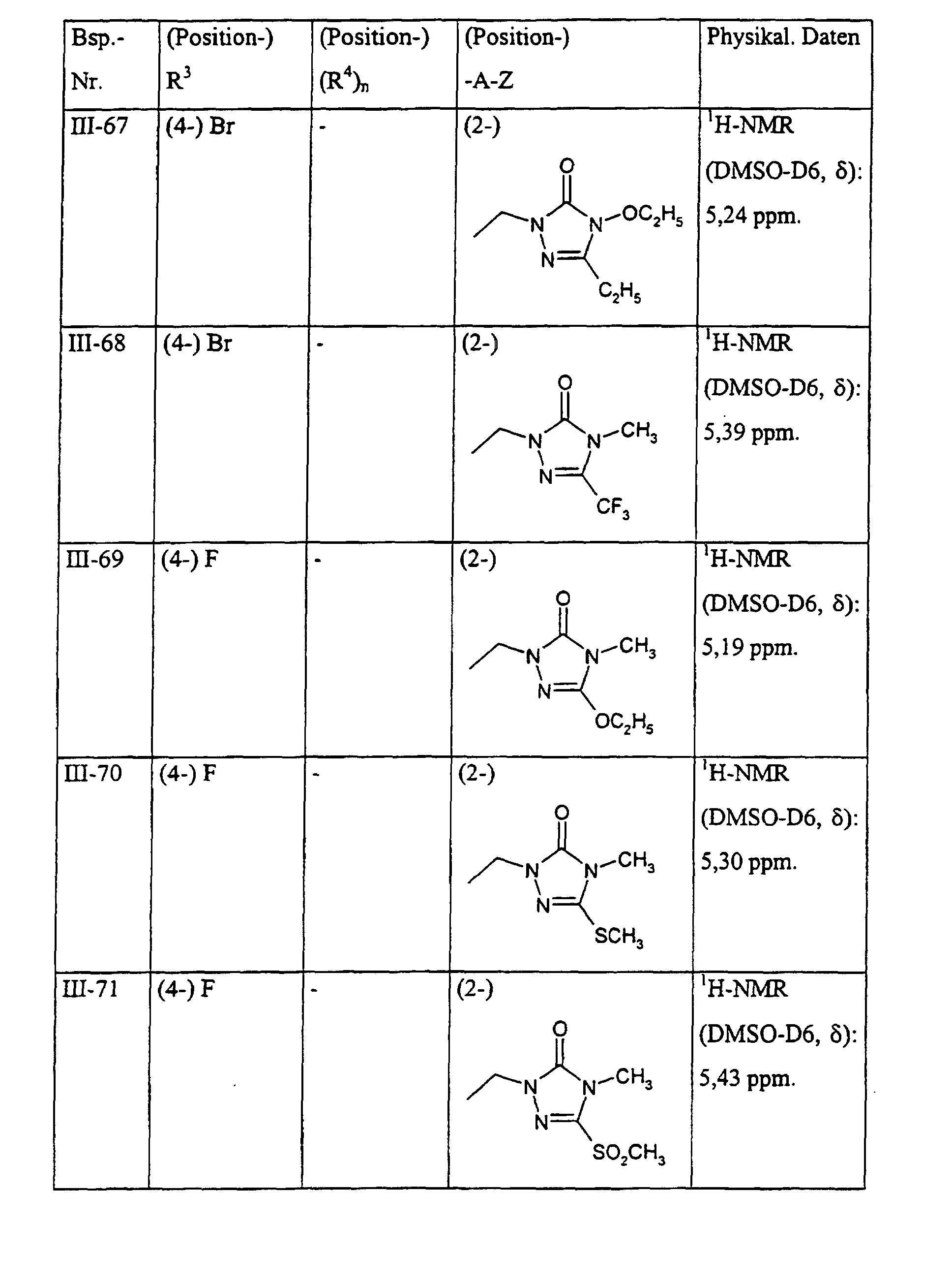

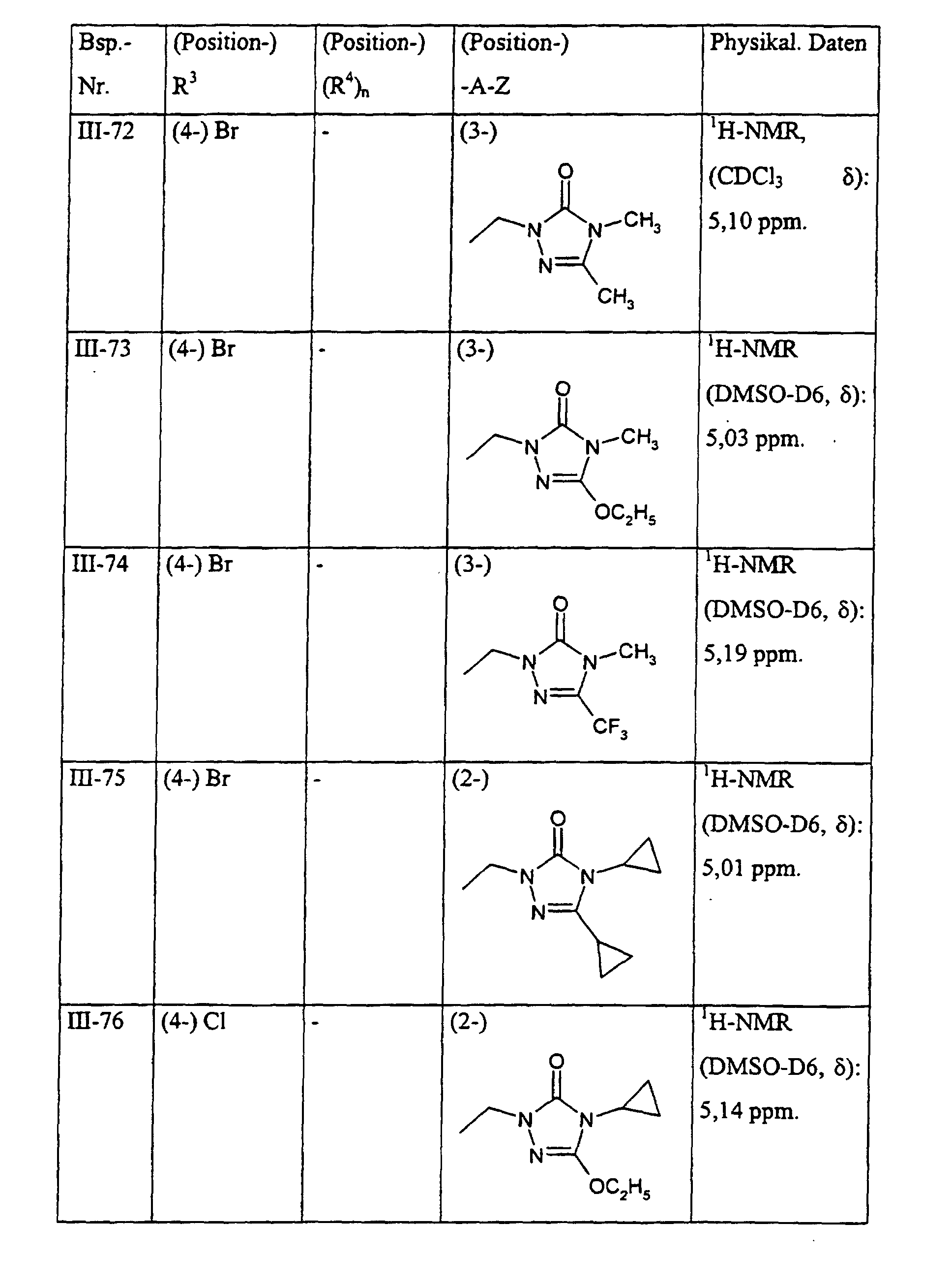

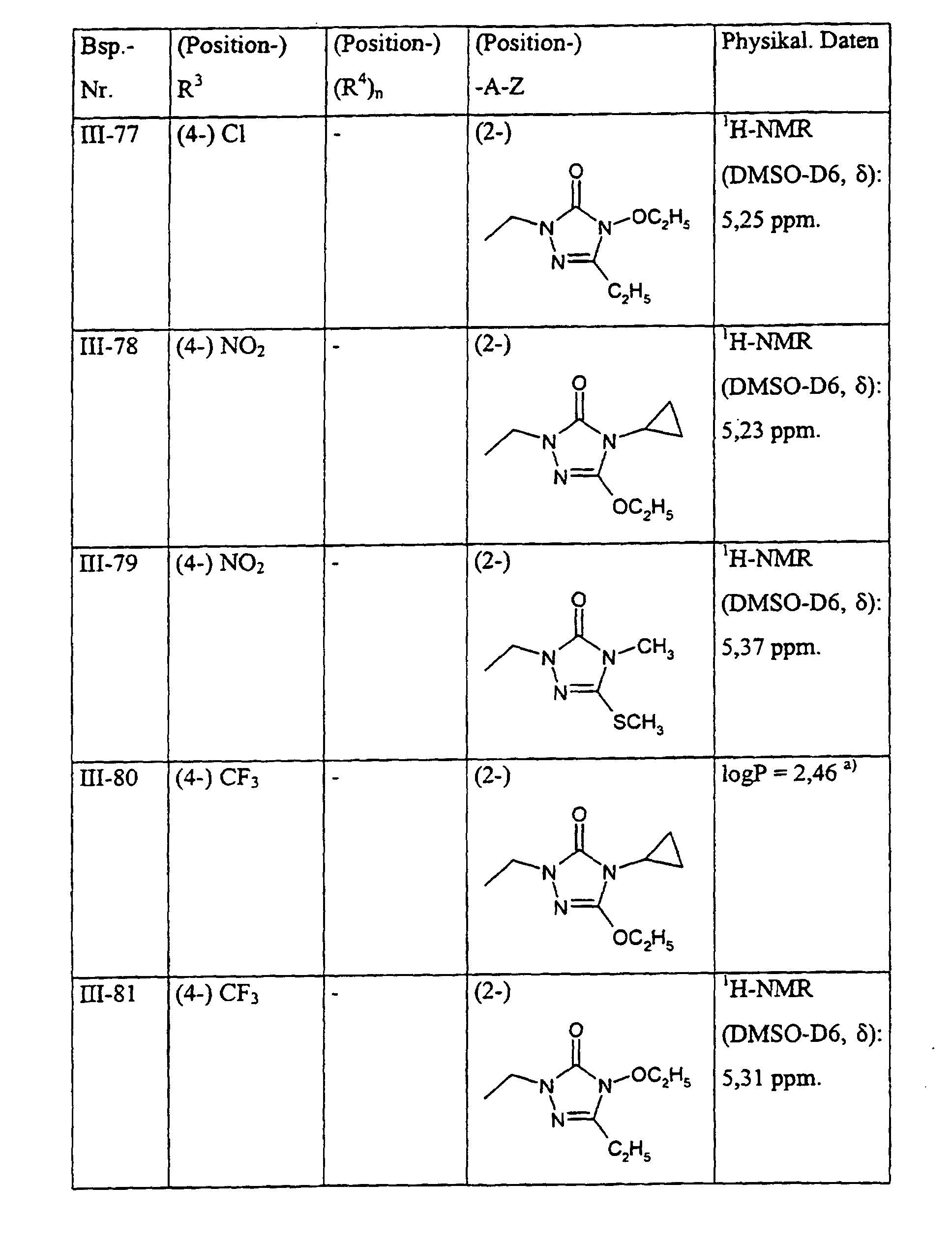

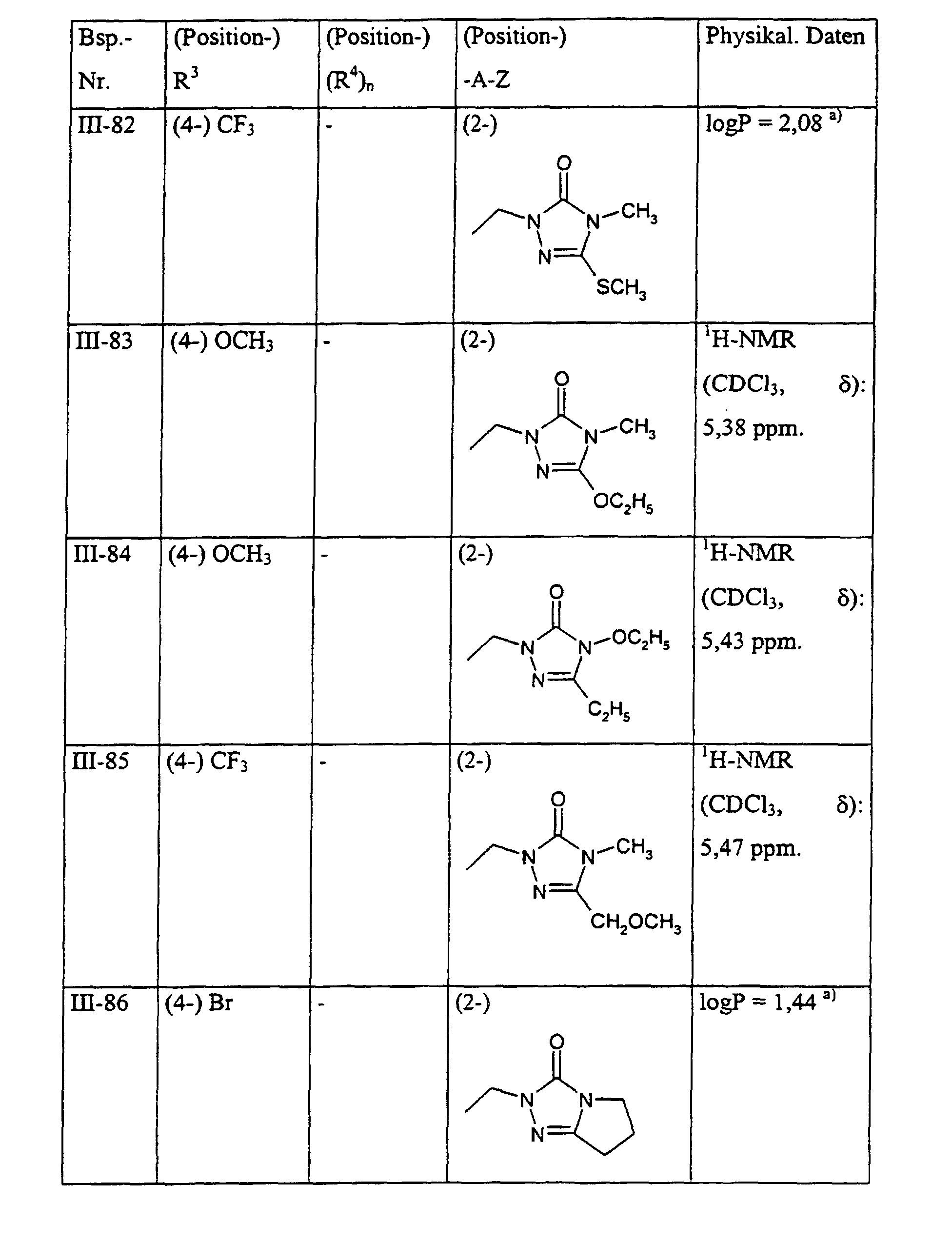

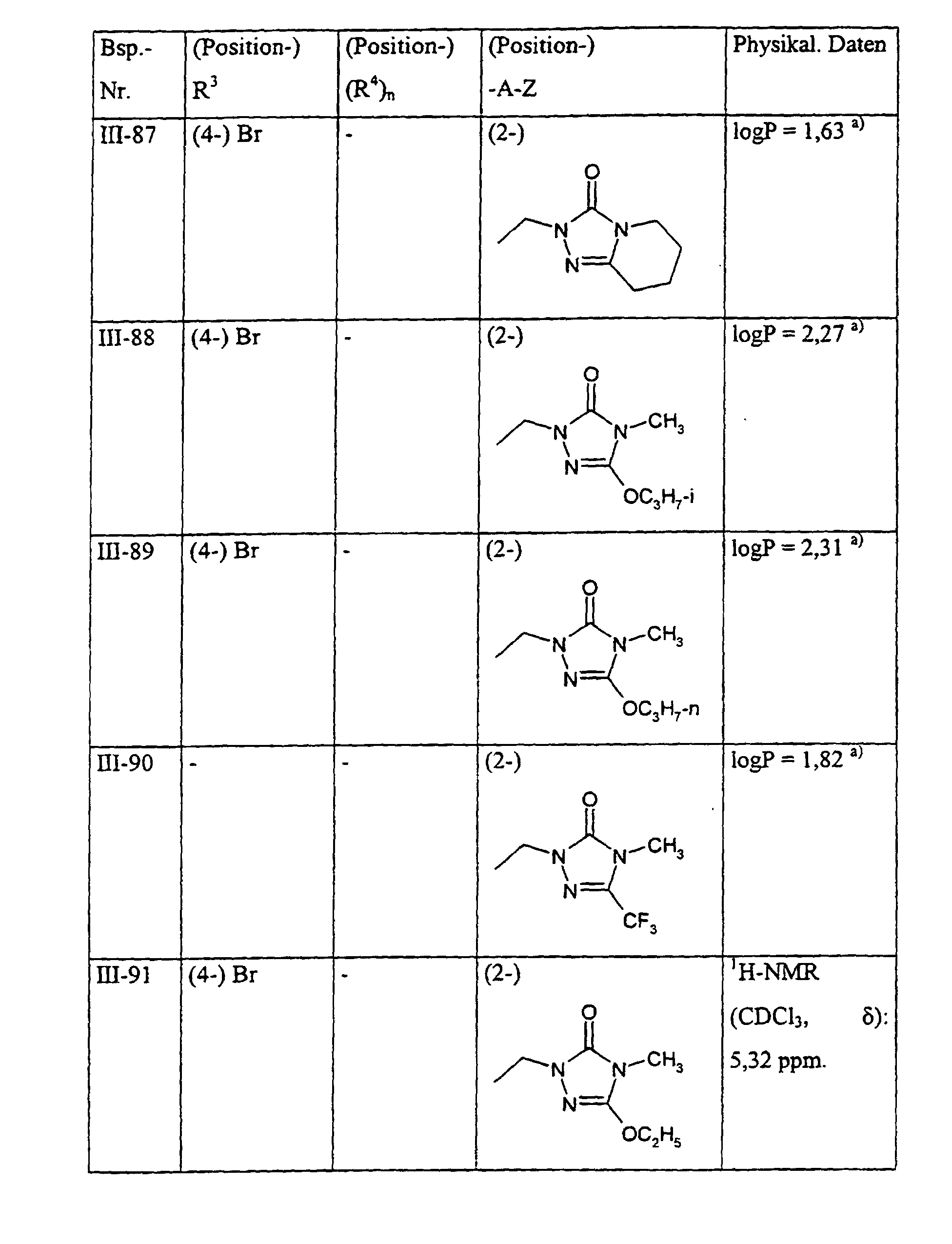

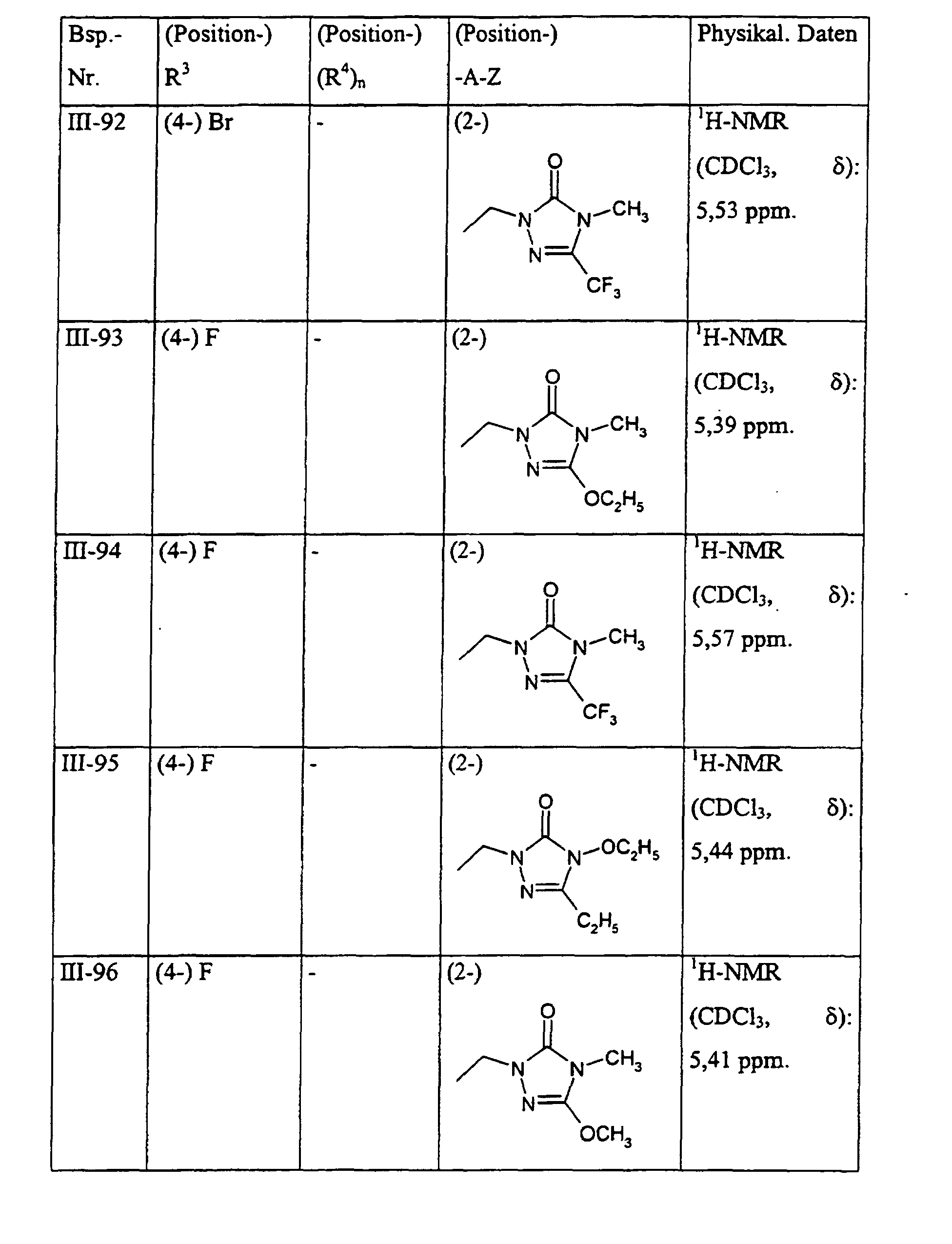

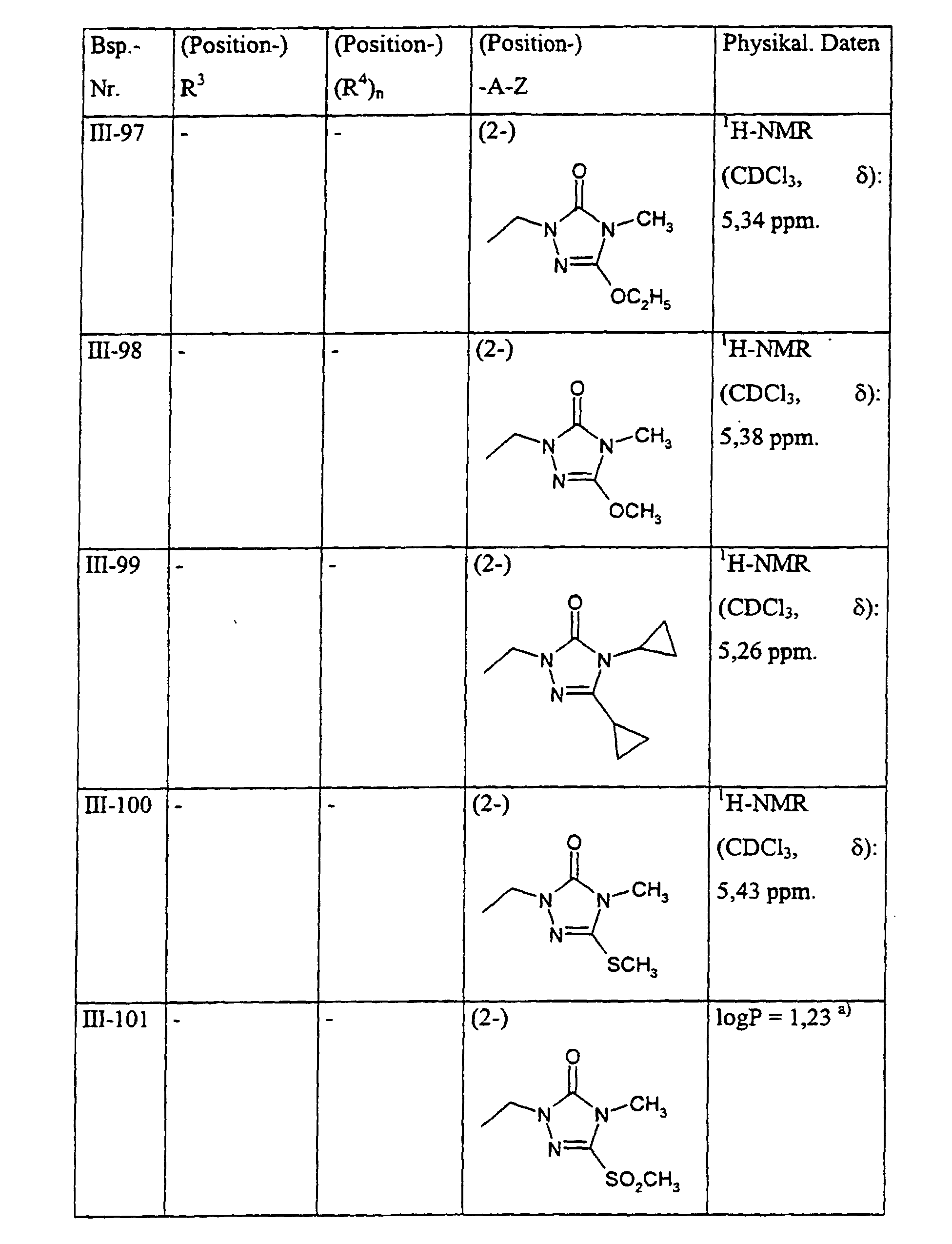

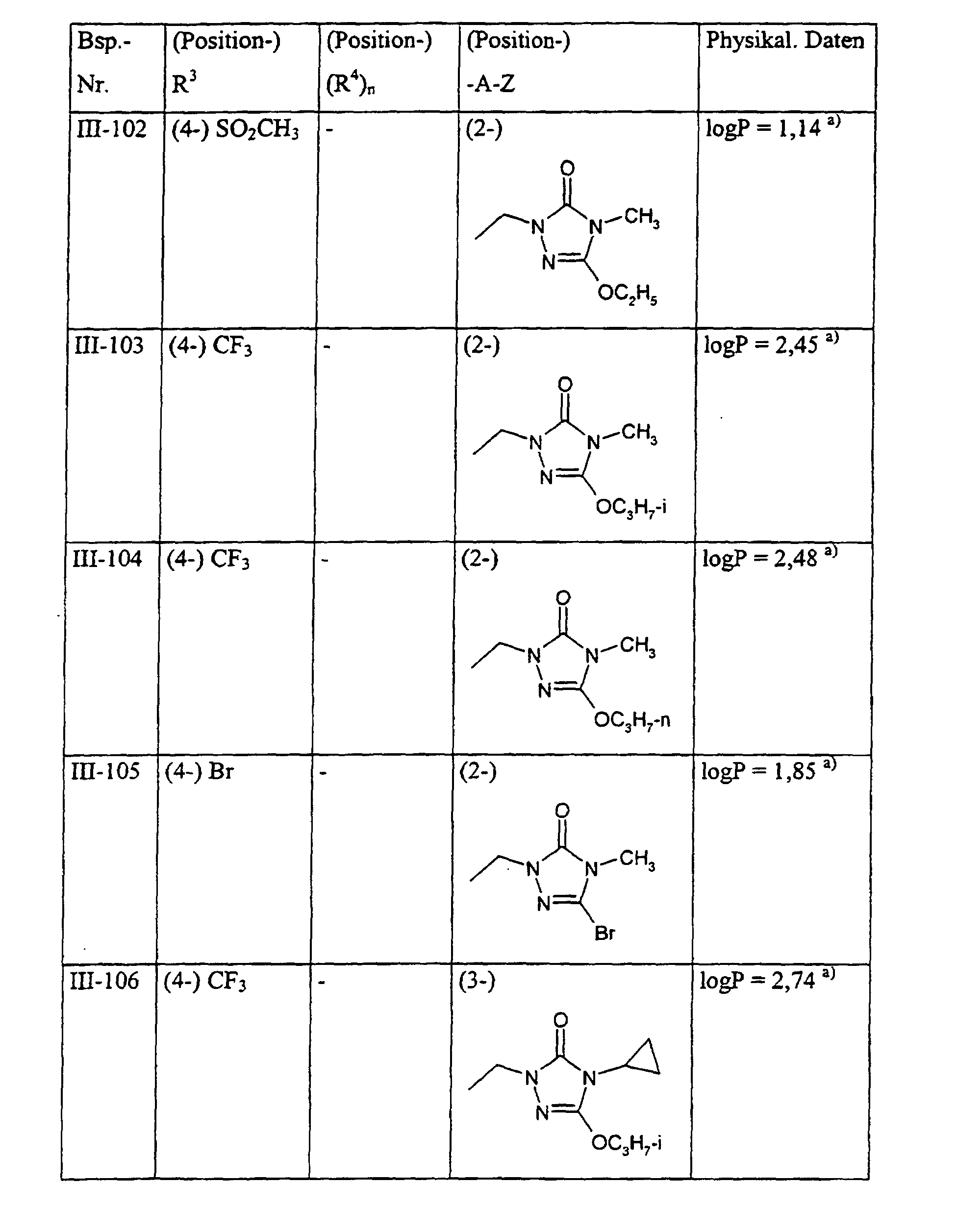

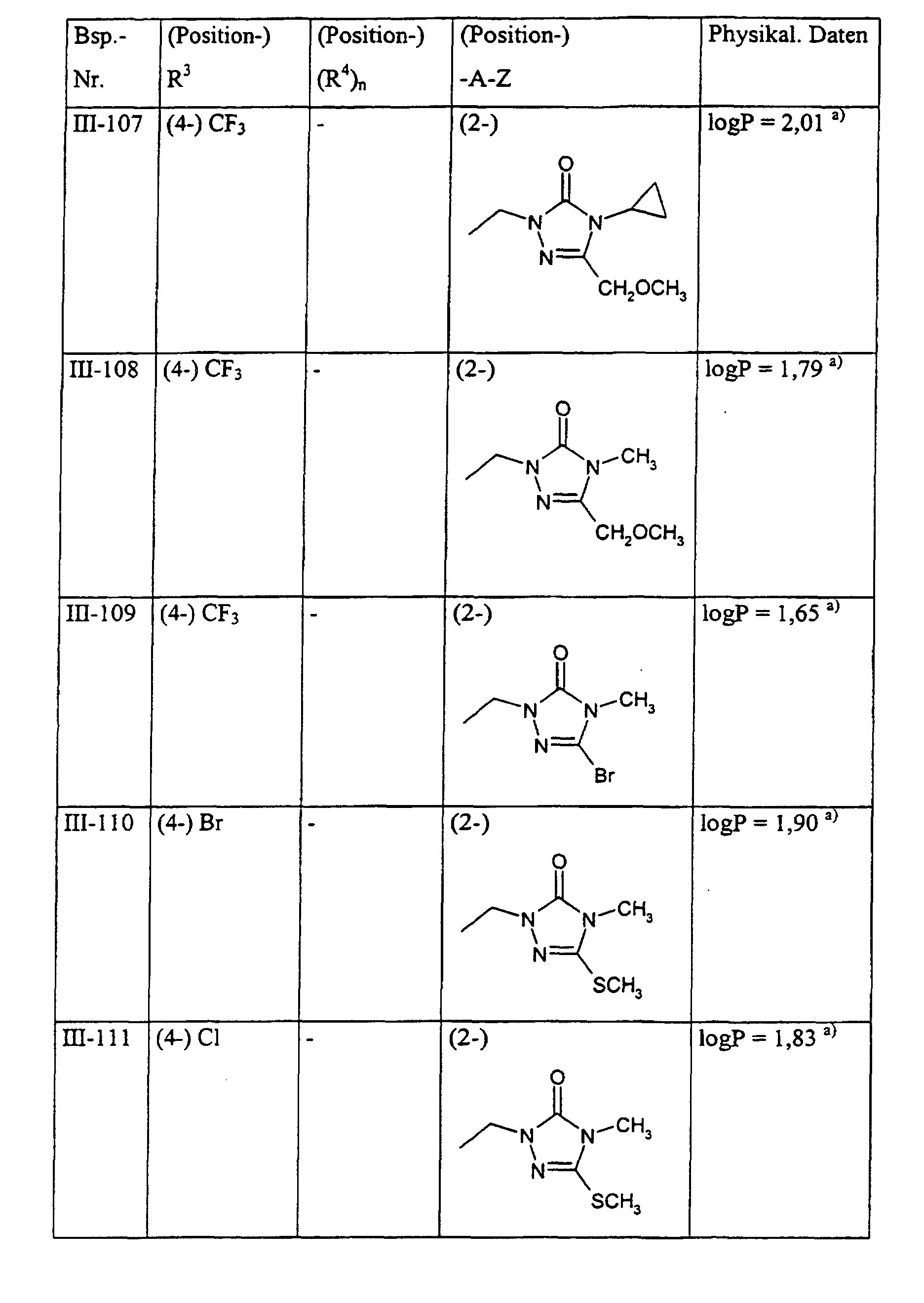

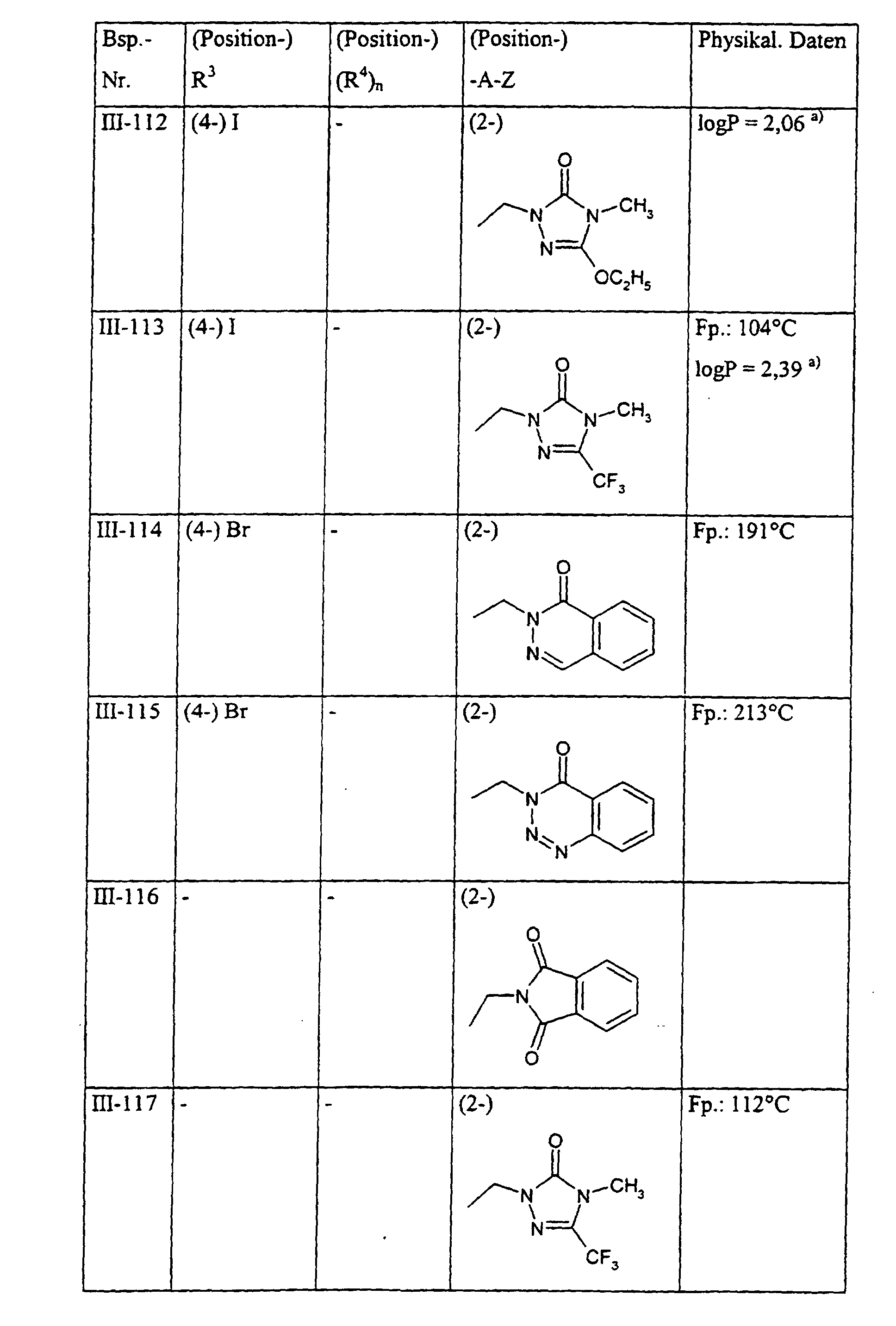

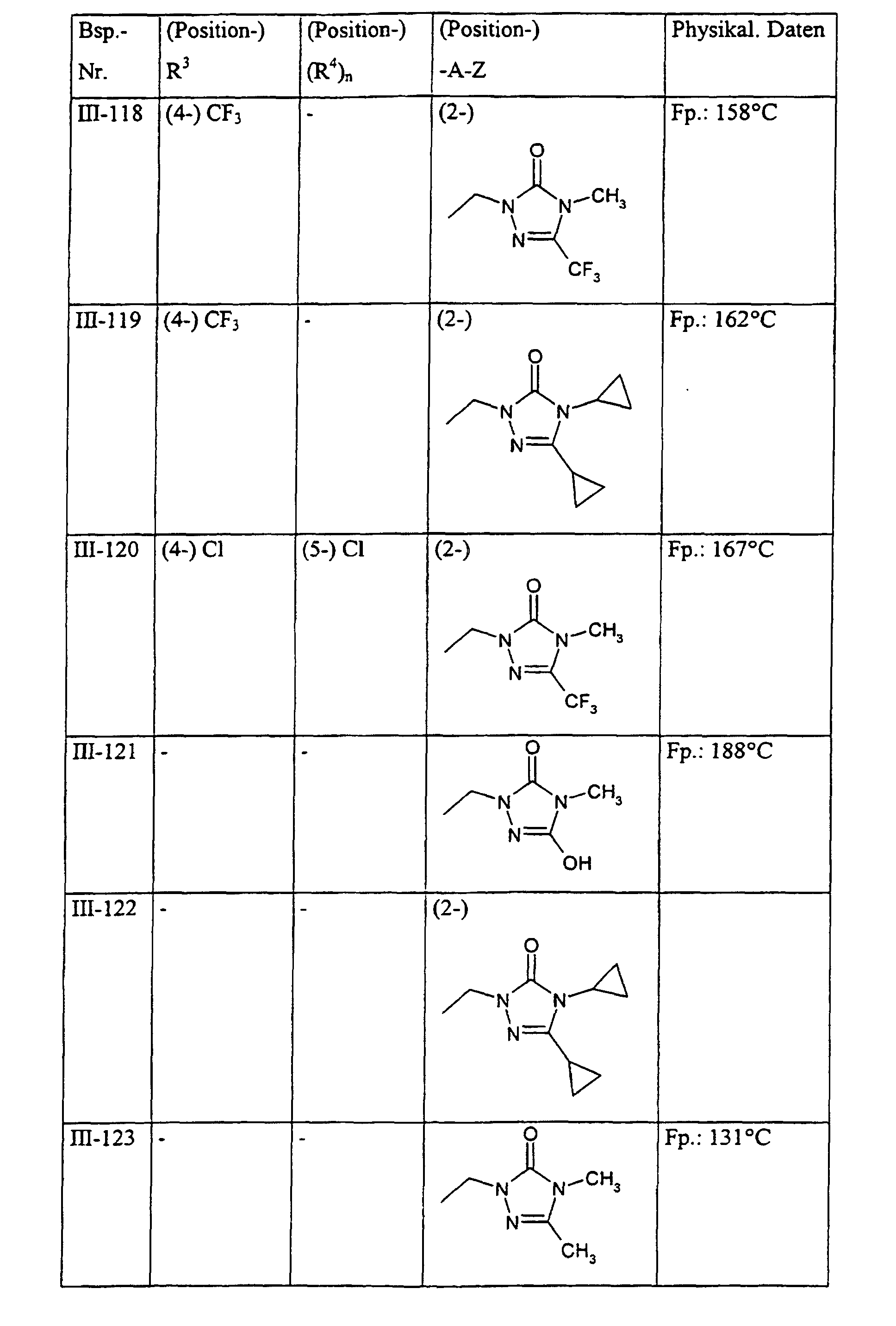

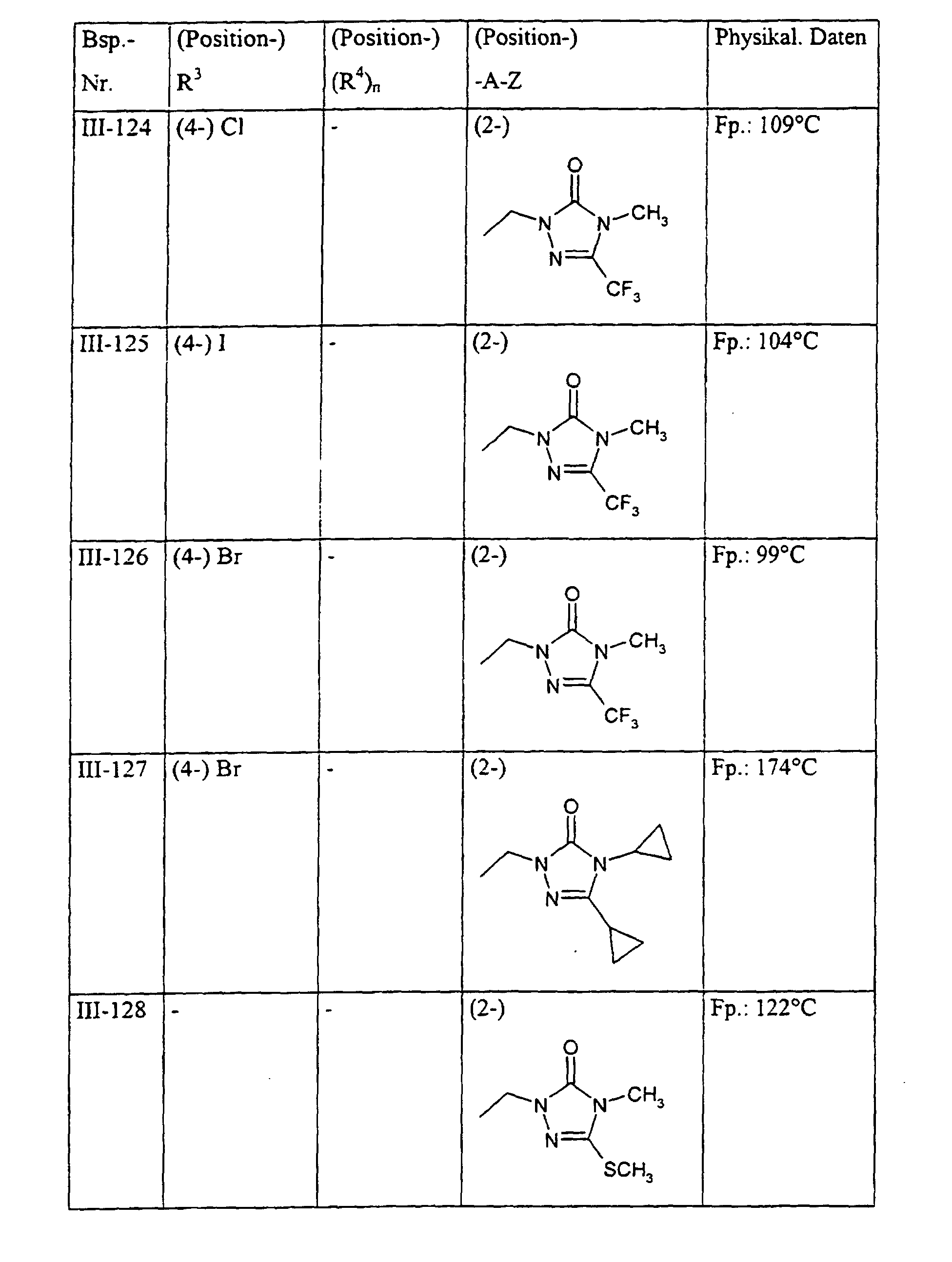

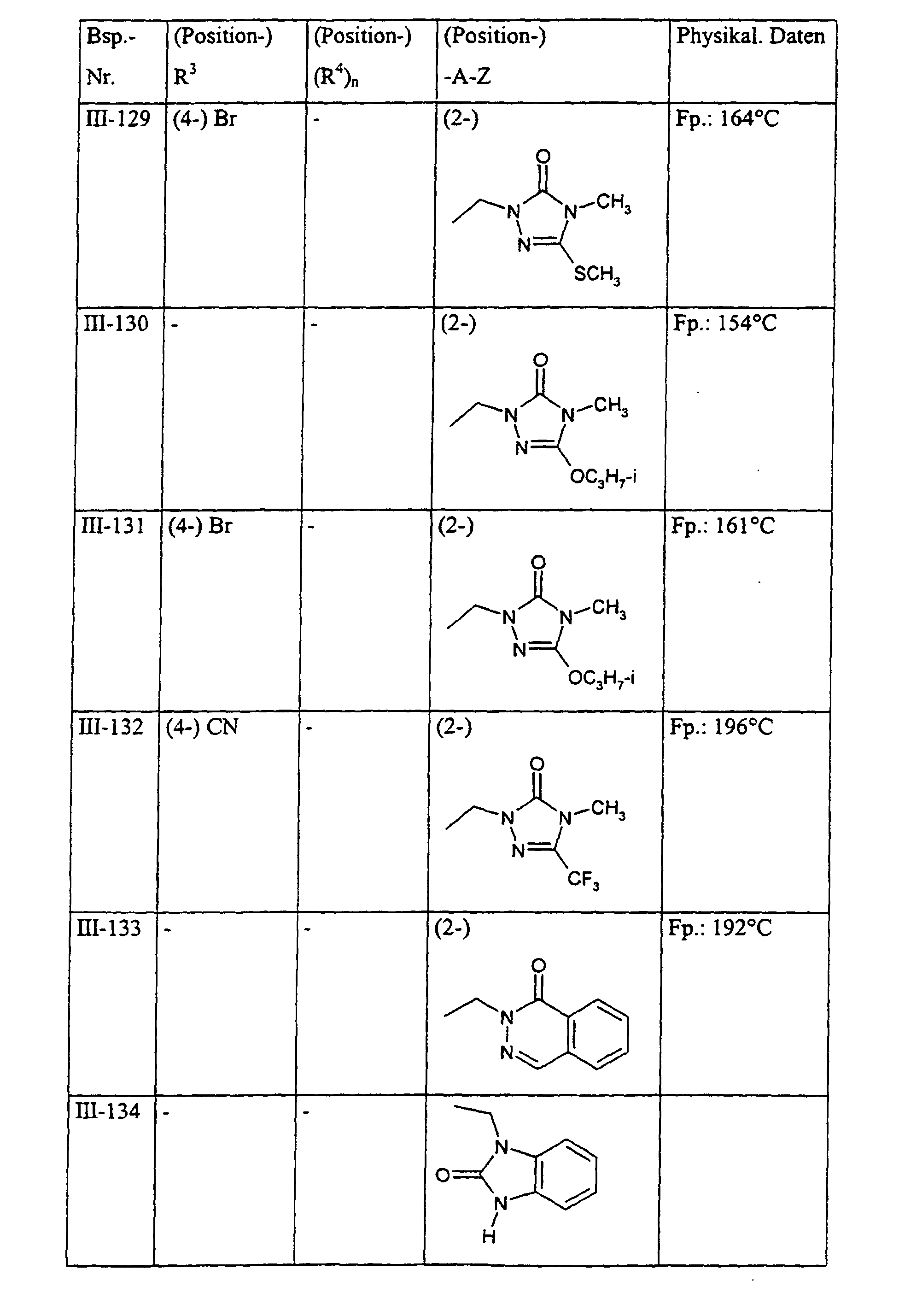

Die Bestimmung der in Tabelle 2 angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1 % wässrige Phosphorsäure, Acetonitril; linearer Gradient von 10% Acetonitril bis 90% Acetonitril - entsprechende Messergebnisse sind in Tabelle 2 mit ^{a)} markiert.
(b) Eluenten für die Bestimmung im neutralen Bereich: 0,01-molare wässrige Phosphatpuffer-Lösung, Acetonitril; linearer Gradient von 10% Acetonitril bis 90% Acetonitril- entsprechende Messergebnisse sind in Tabelle 2 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, dass die jeweils gewünschte Wirkstoffmenge pro Flächeneinheit ausgebracht wird. Die Konzentration der Spritzbrühe wird so gewählt, dass in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0% = | keine Wirkung (wie unbehandelte Kontrolle) |
| 100% = | totale Vernichtung |

In diesem Test zeigen beispielsweise die Verbindungen gemäß Herstellungsbeispiel 1 , 2, 3, 4, 5 und 6 bei guter Verträglichkeit gegenüber Kulturpflanzen, wie z.B. Mais, starke Wirkung gegen Unkräuter.

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.
Mit der Wirkstoffzubereitung spritzt man Testpflanzen, welche eine Höhe von 5 bis 15 cm haben so, dass die jeweils gewünschten Wirkstoffmengen pro Flächeneinheit ausgebracht werden. Die Konzentration der Spritzbrühe wird so gewählt, dass in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0% = | keine Wirkung (wie unbehandelte Kontrolle) |
| 100% = | totale Vernichtung |

In diesem Test zeigen beispielsweise die Verbindungen gemäß Herstellungsbeispiel 1, 2, 3, 4, 5 und 6 bei teilweise guter Verträglichkeit gegenüber Kulturpflanzen, wie z.B. Mais, starke Wirkung gegen Unkräuter.

## Patentansprüche

1. Substituierte Benzoylketone der Formel (1), 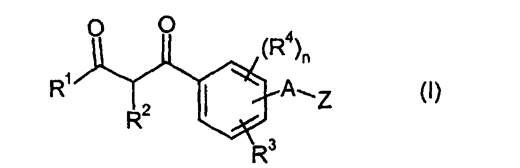 in welcher
n für 0, 1 oder 2 steht,
A für Alkandiyl (Alkylen) mit 1 bis 4 Kohlenstoffatomen steht,
R¹ für Wasserstoff, für gegebenenfalls durch Cyano, Carboxy, Carbamoyl, Halogen, C₁-C₄-Alkoxy, C₁-C₄-Alkylthio, C₁-C₄-Alkylsulfinyl oder C₁-C₄-Alkylsulfonyl substituiertes Alkyl mit 1 bis 6 Kohlenstoffatomen, oder für gegebenenfalls durch Cyano, Carboxy, Carbamoyl, Halogen, C₁-C₄-Alkyl oder C₁-C₄-Alkoxy-carbonyl substituiertes Cycloalkyl mit 3 bis 6 Kohlenstoffatomen steht,
R² für Wasserstoff, Cyano, Carbamoyl, Halogen, für jeweils gegebenenfalls durch Cyano, Carbamoyl, Halogen oder C₁-C₄-Alkoxy substituiertes Alkyl, Alkoxy oder Alkoxycarbonyl mit jeweils bis zu 6 Kohlenstoffatomen, oder für jeweils gegebenenfalls durch Halogen substituiertes Alkylthio, Alkylsulfinyl oder Alkylsulfonyl mit jeweils 1 bis 6 Kohlenstoffatomen steht,
R³ für Wasserstoff, Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen steht,
R⁴ für Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen steht, und
Z für eine der nachstehenden heterocyclischen Gruppierungen steht 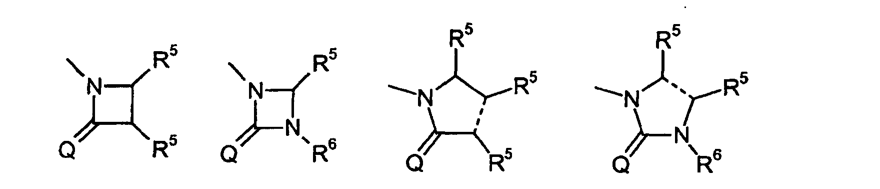 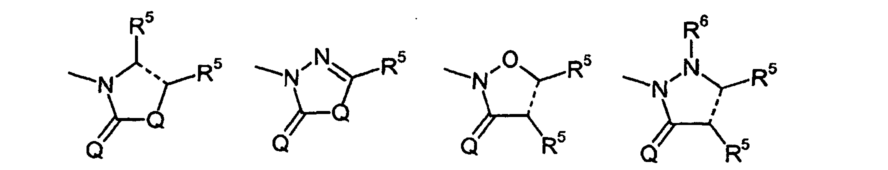 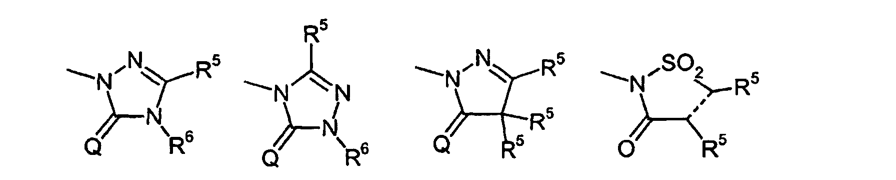 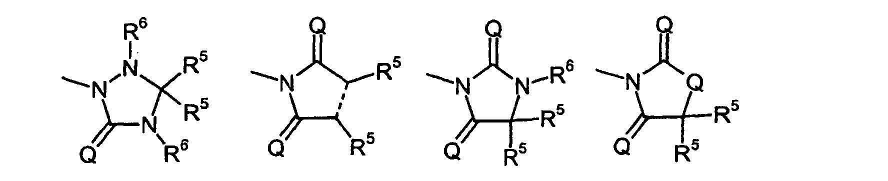 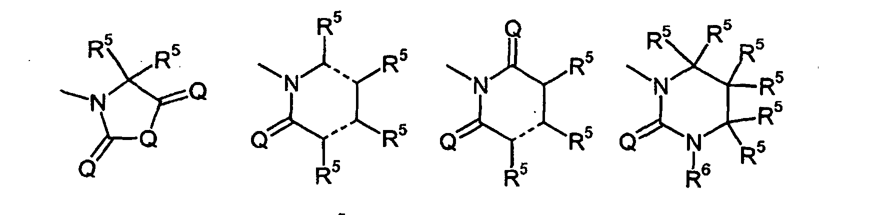 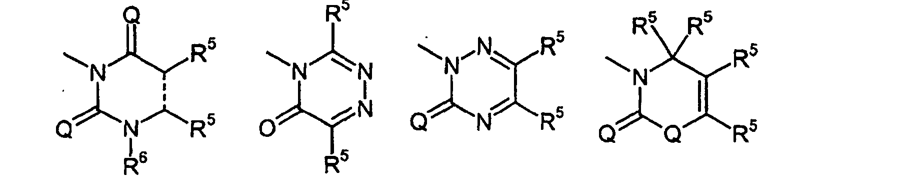 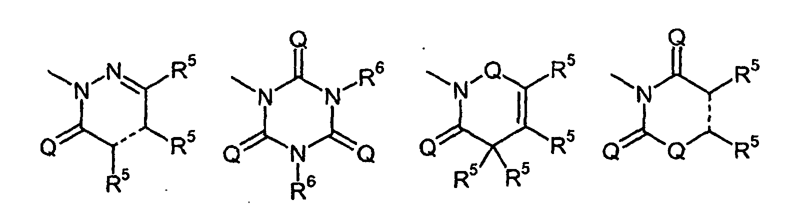 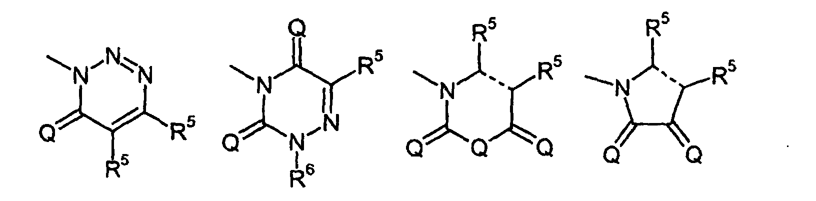 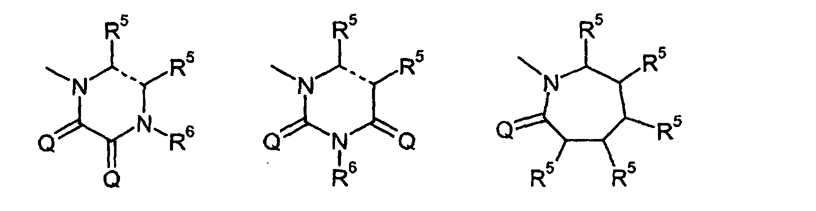 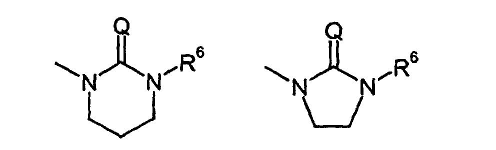 worin jeweils die gestrichelt gezeichnete Bindung eine Einfachbindung oder eine Doppelbindung ist, und jede heterocyclische Gruppierung vorzugsweise nur zwei Substituenten der Definition R⁵ und/oder R⁶ trägt,
Q für Sauerstoff oder Schwefel steht,
R⁵ für Wasserstoff, Hydroxy, Mercapto, Cyano, Halogen, für jeweils gegebenenfalls durch Cyano, Halogen, C₁-C₄-Alkoxy, C₁-C₄-Alkylthio, C₁-C₄-Alkylsulfinyl oder C₁-C₄-Alkylsulfonyl substituiertes Alkyl, Alkylcarbonyl, Alkoxy, Alkoxycarbonyl, Atkylthio, Alkylsulfinyl oder Alkylsulfonyl mit jeweils bis zu 6 Kohlenstoffatomen in den Alkylgruppen, für jeweils gegebenenfalls durch Halogen substituiertes Alkylamino oder Dialkylamino mit jeweils bis zu 6 Kohlenstoffatomen in den Alkylgruppen, für jeweils gegebenenfalls durch Halogen substituiertes Alkenyl, Alkinyl, Alkenyloxy, Alkenylthio oder Alkenylamino mit jeweils bis zu 6 Kohlenstoffatomen in den Alkenyl- bzw. Alkinylgruppen, für jeweils gegebenenfalls durch Halogen substituiertes Cycloalkyl, Cycloalkyloxy, Cycloalkylthio, Cycloalkylamino, Cycloalkylalkyl, Cydoalkylalkoxy, Cycloalkylalkylthio oder Cycloalkylalkylamino mit jeweils 3 bis 6 Kohlenstoffatomen in den Cycloalkylgruppen und gegebenenfalls bis zu 4 Kohlenstoffatomen im Alkylteil, oder für jeweils gegebenenfalls durch Halogen, C₁-C₄-Alkyl oder C₁-C₄-Alkoxy substituiertes Phenyl, Phenyloxy, Phenylthio, Phenylamino, Benzyl, Benzyloxy, Benzylthio oder Benzylamino steht, oder - für den Fall, dass zwei benachbarte Reste R⁵ und R⁵ sich an einer Doppelbindung befinden - zusammen mit dem benachbarten Rest R⁵ auch für eine Benzogruppierung steht, und
R⁶ für Wasserstoff, Hydroxy, Amino, Alkylidenamino mit bis zu 4 Kohlenstoffatomen, für jeweils gegebenenfalls durch Halogen oder C₁-C₄-Alkoxy substituiertes Alkyl, Alkoxy, Alkylamino, Dialkylamino oder Alkanoylamino mit jeweils bis zu 6 Kohlenstoffatomen in den Alkylgruppen, für jeweils gegebenenfalls durch Halogen substituiertes Alkenyl, Alkinyl oder Alkenyloxy mit jeweils bis zu 6 Kohlenstoffatomen in den Alkenyl- bzw. Alkinylgruppen, für jeweils gegebenenfalls durch Halogen substituiertes Cycloalkyl, Cycloalkylalkyl oder Cycloalkylamino mit jeweils 3 bis 6 Kohlenstoffatomen in den Cycloalkylgruppen und gegebenenfalls bis zu 3 Kohlenstoffatomen im Alkylteil, oder für jeweils gegebenenfalls durch Halogen, C₁-C₄-Alkyl oder C₁-C₄-Alkoxy substituiertes Phenyl oder Benzyl steht, oder zusammen mit einem benachbarten Rest R⁵ oder R⁶ für gegebenenfalls durch Halogen oder C₁-C₄-Alkyl substituiertes Alkandiyl mit 3 bis 5 Kohlenstoffatomen steht,
wobei die einzelnen Reste R⁵ und R⁶ - soweit mehrere davon an gleiche heterocyclische Gruppierungen gebunden sind, gleiche oder verschiedene Bedeutungen im Rahmen der obigen Definition haben können,
- einschließlich aller möglichen tautomeren Formen und der Natrium-, Kalium-, Magnesium-, Calcium-, Ammonium-, C₁-C₄-Alkyl-ammonium-, Di-(C₁-C₄alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammoniumund Di-(C₁-C₂-alkyl)-benzyl-ammonium-Salze.

2. Verbindungen gemäß Anspruch 1, **dadurch gekennzeichnet, dass**
A für Methylen, Ethyliden (Ethan-1,1-diyl) oder Dimethylen (Ethan-1,2-diyl) steht,
R¹ für Wasserstoff, für jeweils gegebenenfalls durch Cyano, Carboxy, Carbamoyl, Fluor, Chlor, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n-oder i-Propyl, n-, i-, s- oder t-Butyl, oder für jeweils gegebenenfalls durch Cyano, Carboxy, Carbamoyl, Fluor, Chlor, Methyl, Ethyl, Methoxycarbonyl oder Ethoxycarbonyl substituiertes Cyclopropyl, Cyclobutyl, Cyclopentyl oder Cyclohexyl steht,
R² für Wasserstoff, Cyano, Carbamoyl, Fluor, Chlor, Brom, für jeweils gegebenenfalls durch Cyano, Carbamoyl, Fluor, Chlor, Methoxy oder Ethoxy substituiertes Methyl, Ethyl, n- oder i-Propyl, Methoxy, Ethoxy, n- oder i-Propoxy, Methoxycarbonyl, Ethoxycarbonyl, n- oder i-Propoxycarbonyl, oder für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl steht,
R³ für Wasserstoff, Nitro, Cyano, Carboxy, Carbamoyl, Thiocarbamoyl, Fluor, Chlor, Brom, lod, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht,
R⁴ für Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n-oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht,
R⁵ für Wasserstoff, Hydroxy, Mercapto, Cyano, Fluor, Chlor, Brom, lod, für jeweils gegebenenfalls durch Fluor, Chlor, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, n-, i-, s- oder t-Butylamino, Dimethylamino, Diethylamino, Di-n-propylamino oder Di-i-propylamino, für jeweils gegebenenfalls durch Fluor und/oder Chlor substituiertes Ethenyl, Propenyl, Butenyl, Ethinyl, Propinyl, Butinyl, Propenyloxy, Butenyloxy, Propenylthio, Butenylthio, Propenylamino oder Butenylamino, für jeweils gegebenenfalls durch Fluor und/oder Chlor substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl, Phenyloxy, Phenylthio, Phenylamino, Benzyl, Benzyloxy, Benzylthio oder Benzylamino steht, oder - für den Fall, dass zwei benachbarte Reste R⁵ und R⁵ sich an einer Doppelbindung befinden - zusammen mit dem benachbarten Rest R⁵ auch für eine Benzogruppierung steht,
R⁶ für Wasserstoff, Hydroxy, Amino, für jeweils gegebenenfalls durch Fluor und/oder Chlor, Methoxy oder Ethoxy substituiertes Methyl, Ethyl, n- oder i-Propyl, n-, i- oder s-Butyl, Methoxy, Ethoxy, n- oder i-Propoxy, Methylamino, Ethylamino oder Dimethylamino, für jeweils gegebenenfalls durch Fluor und/oder Chlor substituiertes Ethenyl, Propenyl, Ethinyl, Propinyl oder Propenyloxy, für jeweils gegebenenfalls durch Fluor und/oder Chlor substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl oder Benzyl steht, oder zusammen mit einem benachbarten Rest R⁵ oder R⁶ für jeweils gegebenenfalls durch Methyl und/oder Ethyl substituiertes Propan-1,3-diyl (Trimethylen), Butan-1,4-diyl (Tetramethylen) oder Pentan-1,5-diyl (Pentamethylen) steht, und
Z für eine der nachstehenden heterocyclischen Gruppierungen steht

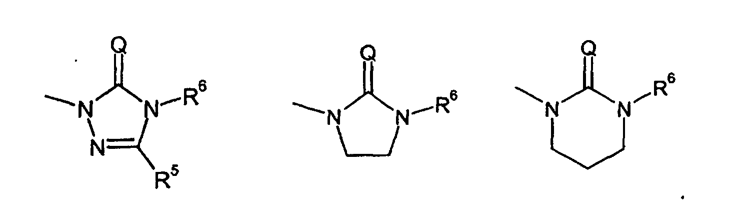

3. Verbindungen gemäß einem der Ansprüche 1 oder 3, **dadurch gekennzeichnet, dass**
n für 0 oder 1 steht,
A für Methylen steht,
R¹ für jeweils gegebenenfalls Cyano, Fluor, Chlor, Methoxy, Ethoxy, n- oder i-Propoxy, Methylthio, Ethylthio, n- oder o-Propylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, oder für jeweils gegebenenfalls durch Cyano, Fluor, Chlor, Methyl oder Ethyl substituiertes Cyclopropyl, Cyclobutyl, Cyclopentyl oder Cyclohexyl steht,
R² Wasserstoff, Cyano, Carbamoyl, Fluor, Chlor, Brom, für jeweils gegebenenfalls durch Cyano, Carbamoyl, Fluor, Chlor, Methoxy oder Ethoxy substituiertes Methyl, Ethyl, n- oder i-Propyl, oder für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl steht,
R³ für Wasserstoff, Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methytthio, Ethylthio, n- oder i-Propylthio, Methylsulfinyl, Ethyfsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht,
R⁴ für Methylsulfonyl, Chlor, Methoxy, Nitro, Trifluormethyl oder Methyl steht,
R⁵ für Wasserstoff, Hydroxy, Chlor, Brom, Methyl, Ethyl, n- oder i-Propyl, n-, i-, s-oder t-Butyl, Difluormethyl, Dichlormethyl, Trifluormethyl, Trichlormethyl, Chlordifluormethyl, Fluordichlormethyl, Fluorethyl, Chlorethyl, Difluorethyl, Dichlorethyl, Fluor-n-propyl, Fluor-i-propyl, Chlor-n-propyl, Chlor-i-propyl, Methoxymethyl, Ethoxymethyl, Methoxyethyl, Ethoxyethyl, Methoxy, Ethoxy, n- oder i-Propoxy, n-, i-, s- oder t-Butoxy, Fluorethoxy, Chlorethoxy, Difluorethoxy, Dichlorethoxy, Trifluorethoxy, Trichlorethoxy, Chlorfluorethoxy, Chlordifluorethoxy, Fluordichlorethoxy, Methylthio, Ethylthio, n- oder i-Propylthio, Fluorethylthio, Chlorethylthio, Difluorethylthio, Dichlorethylthio, Chlorfluorethylthio, Chlordifluorethylthio, Fluordichlorethylthio, Methylsulfinyl, Ethylsulfinyl, n-oder i-Propylsulfinyl, Methylsulfonyl, Ethylsulfonyl, n- oder i-Propylsulfonyl, Methylamino, Dimethylamino, Propenylthio, Butenylthio, Propinylthio, Butinylthio, Cyclopropyl, Cyclopropylmethyl, Cyclopropylmethoxy, Phenyl oder Phenoxy steht, und
R⁶ für Amino, Methyl, Ethyl, n- oder i-Propyl, n-, i-, s- oder t-Butyl, Methoxy, Ethoxy, Methylamino, Dimethylamino, Cyclopropyl oder Cyclopropylmethyl steht, oder zusammen mit R⁵ für Propan-9,3-diyl (Trimethylen), Butan-1,4-diyl (Tetramethylen) oder Pentan-1,5-diyl (Pentamethylen) steht.

4. Verbindungen gemäß einem der Ansprüche 1, 3 bis 4, **dadurch gekennzeichnet, dass**
R³ für Wasserstoff, Nitro, Cyano, Fluor, Chlor, Brom, Methyl, Trifluormethyl oder Methoxy steht,
R⁵ für Wasserstoff, Brom, Chlor, Methyl, Ethyl, Trifluormethyl, Cyclopropyl, Difluorethyl, Methylthio, Ethylthio, Methoxy, Ethoxy, n- oder i-Propoxy, Trifluorethoxy, Methylamino oder Dimethylamino steht, und
R⁶ für Wasserstoff, Amino, Methyl, Ethyl, Methoxy, Ethoxy, Cyclopropyl oder Dimethylamino steht.

5. Verbindungen gemäß einem der Ansprüche 1, 3 bis 5, **dadurch gekennzeichnet, dass**
Q für Sauerstoff (O) steht.

6. Verbindungen gemäß einem der Ansprüche 1, 3 bis 6, **dadurch gekennzeichnet, dass**
Z für die heterocyclische Gruppierung 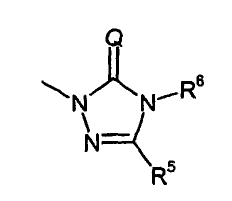 steht

7. Verbindungen gemäß einem der Ansprüche 1 , 3 bis 7, **dadurch gekennzeichnet, dass**
R¹ für Cyclopropyl steht.

8. Verbindungen gemäß einem der Ansprüche 1, 3 bis 8, **dadurch gekennzeichnet, dass**
R² für Wasserstoff oder Cyano steht.

9. Verbindungen gemäß einem der Ansprüche 1, 3 bis 9, **dadurch gekennzeichnet, dass**
R⁵ für Brom, Methyl, Ethyl, Methoxy, Methylthio, Ethoxy, Methylsulfonyl oder Dimethylamino steht, und
R⁶ für Amino, Methyl, Ethyl, Cyclopropyl, Dimethylamino, Methoxy oder Ethoxy steht.

10. Verbindungen der Formel (IA), 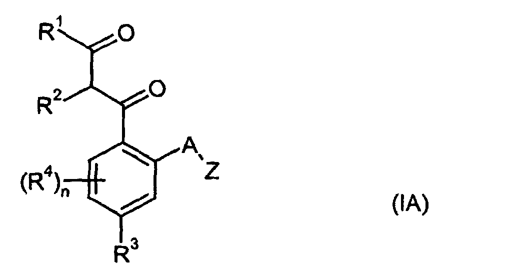 in welcher
n, A, R¹, R², R3, R⁴ und Z die in einem der Ansprüche 1 bis 5 und 7 bis 9 angegebenen Bedeutungen haben.

11. Verbindungen der Formel (IB), 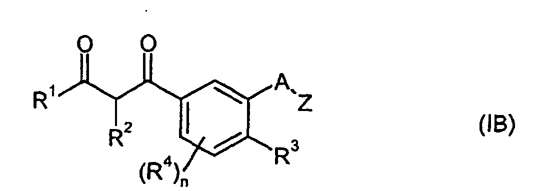 in welcher
n, A, R¹, R², R³, R⁴ und Z die in einem der Ansprüche 1 bis 5 und 7 bis 9 angegebenen Bedeutungen haben.

12. Verbindungen der Formel (IC), 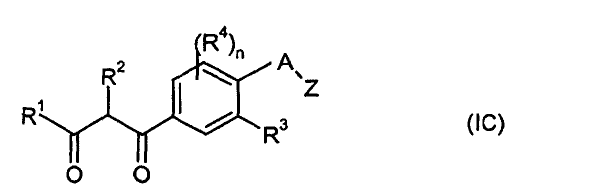 in welcher
n, A, R¹, R², R³, R⁴ und Z die in einem der Ansprüche 1 bis 5 und 7 bis 9 angegebenen Bedeutungen haben.

13. Verfahren zur Herstellung von Verbindungen gemäß einem der Ansprüche 1 bis 13,
**dadurch gekennzeichnet, dass** man
(a) Ketone der allgemeinen Formel (II) 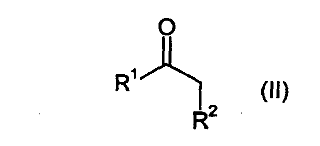
in welcher
R¹ und R² die in einem der Ansprüche 1 bis 4 sowie 8 und 9 angegebene Bedeutung haben,
mit substituierten Benzoesäuren der allgemeinen Formel (III) 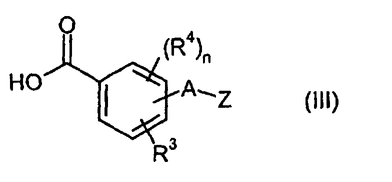 in welcher
n, A, R³, R⁴ und Z die in einem der Ansprüche 1 bis 5 sowie 7 angegebene Bedeutung haben,
gegebenenfalls in Gegenwart eines oder mehrerer Reaktionshilfsmittel und gegebenenfalls in Gegenwart eines oder mehrerer Verdünnungsmittel umsetzt,
und gegebenenfalls im Anschluss daran, d.h. nach Durchführung der erfindungsgemäßen Verfahren (a), an den so erhaltenen Verbindungen der Formel (I) im Rahmen der Substituentendefinition auf übliche Weise elektrophile oder nucleophile bzw. Oxidations- oder Reduktionsreaktionen durchführt oder die Verbindungen der Formel (I) auf übliche Weise in Salze überführt.

14. Verbindungen der Formel (III), 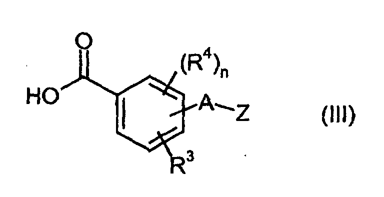 in welcher
n, A, R³, R⁴ und Z die in einem der Ansprüche 1 bis 5 sowie 7 angegebene Bedeutung haben,
mit Ausnahme von 2-(5-Carboxy-2,4-dichlor-phenyl)-4-difluormethyl-5-methyl-2,4-dihydro-3H-1,2,4-triazol-3-on und 2-(5-Carboxy-2,4-dichlorphenyl)-4,5-dimethyl-2,4-dihydro-3H-1,2,4-triazol-3-on.

15. Herbizide Mittel, **gekennzeichnet durch** den Gehalt mindestens einer Verbindung gemäß einem der Ansprüche 1 bis 13 und üblichen Streckmitteln.

16. Verwendung von mindestens einer Verbindung gemäß einem der Ansprüche 1 bis 13 zur Bekämpfung von unerwünschten Pflanzen.

## Claims

1. Substituted benzoyl ketones of the formula (I) 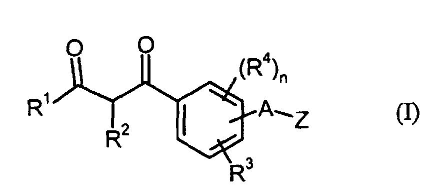 in which
A represents alkanediyl (alkylene) having 1 to 4 carbon atoms,
R¹ represents hydrogen, represents optionally cyano, carboxyl, carbamoyl, halogen, C₁-C₄-alkoxy-, C₁-C₄-alkylthio-, C₁-C₄-alkylsulphinyl- or C₁-C₄-alkylsulphonyl-substituted alkyl having 1 to 6 carbon atoms, or represents optionally cyano-, carboxyl-, carbamoyl-, halogen-, C₁-C₄-alkyl- or C₁-C₄-alkoxy-carbonyl-substituted cycloalkyl having 3 to 6 carbon atoms,
R² represents hydrogen, cyano, carbamoyl, halogen, represents in each case optionally cyano-, carbamoyl-, halogen- or C₁-C₄-alkoxy-substituted alkyl, alkoxy or alkoxycarbonyl having in each case up to 6 carbon atoms, or represents in each case optionally halogen-substituted alkylthio, alkylsulphinyl or alkylsulphonyl having in each case 1 to 6 carbon atoms,
R³ represents hydrogen, nitro, cyano, carboxyl, carbamoyl, thiocarbamoyl, halogen, represents in each case optionally halogen-, C₁-C₄-alkoxy-, C₁-C₄-alkylthio-, C₁-C₄-alkylsulphinyl- or C₁-C₄-alkylsulphonyl-substituted alkyl, alkoxy, alkylthio, alkylsulphinyl or alkylsulphonyl having in each case up to 4 carbon atoms in the alkyl groups, or represents alkylamino, dialkylamino or dialkylaminosulphonyl having in each case up to 4 carbon atoms in the alkyl groups,
R⁴ represents nitro, cyano, carboxyl, carbamoyl, thiocarbamoyl, halogen, represents in each case optionally halogen-, C₁-C₄-alkoxy-, C₁-C₄-alkylthio-, C₁-C₄-alkylsulphinyl- or C₁-C₄-alkylsulphonyl-substituted alkyl, alkoxy, alkylthio, alkylsulphinyl or alkylsulphonyl having in each case up to 4 carbon atoms in the alkyl groups, or represents alkylamino, dialkylamino or dialkylaminosulphonyl having in each case up to 4 carbon atoms in the alkyl groups, and
Z represents one of the heterocyclic groupings below 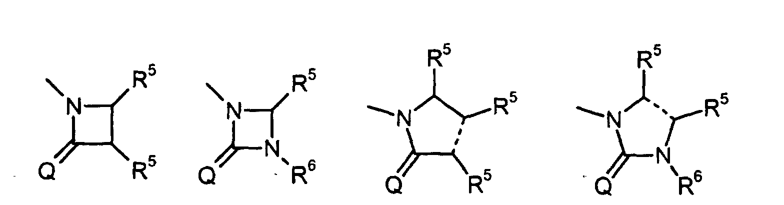 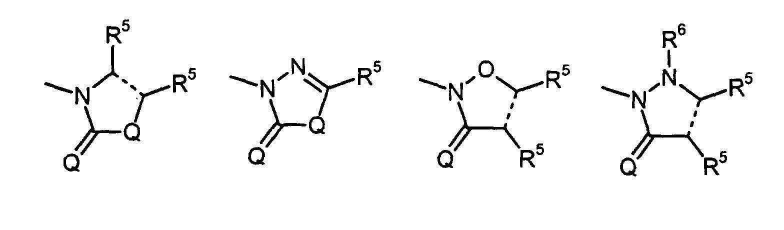 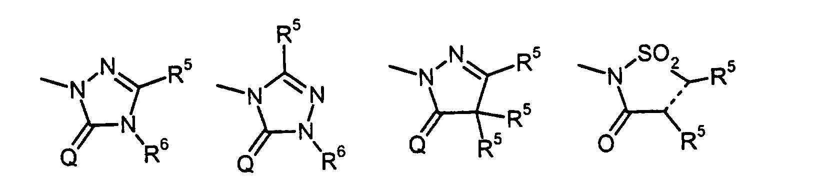 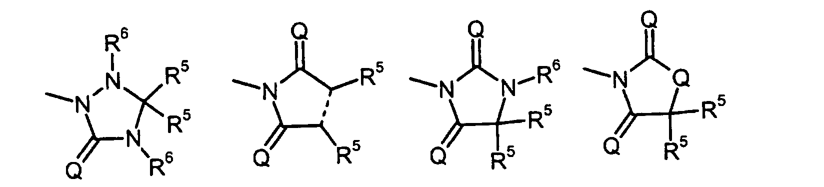 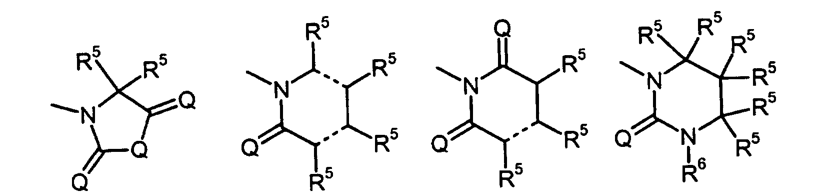 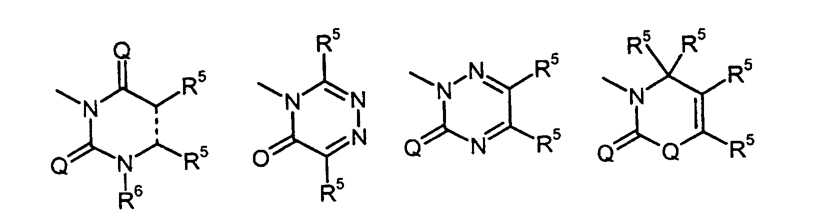 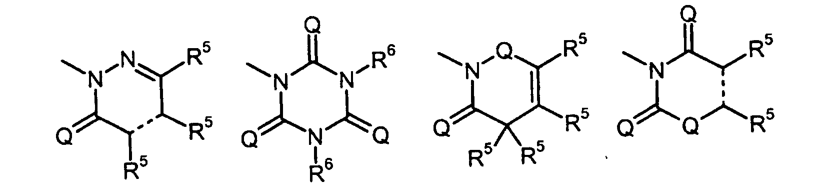 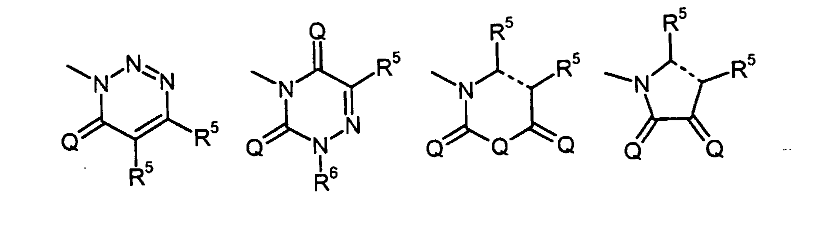 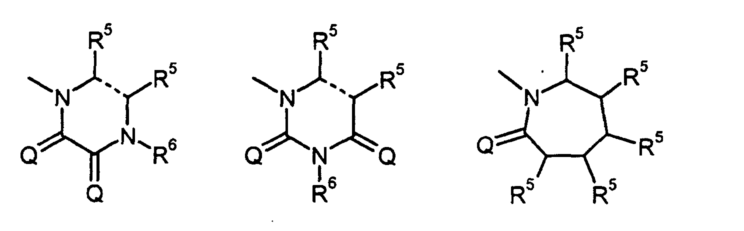 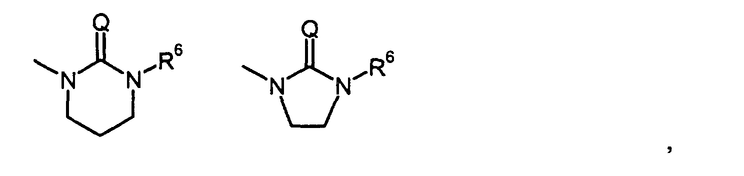 where the dotted line is in each case a single bond or a double bond, and each heterocyclic grouping preferably only carries two substituents of the definition R⁵ and/or R6,
Q represents oxygen or sulphur,
R⁵ represents hydrogen, hydroxyl, mercapto, cyano, halogen, represents in each case optionally cyano-, halogen-, C₁-C₄-alkoxy-, C₁-C₄-alkylthio-, C₁-C₄-alkylsulphinyl- or C₁-C₄-alkylsulphonyl-substituted alkyl, alkylcarbonyl, alkoxy, alkoxycarbonyl, alkylthio, alkylsulphinyl or alkylsulphonyl having in each case up to 6 carbon atoms in the alkyl groups, represents in each case optionally halogen-substituted alkylamino or dialkylamino having in each case up to 6 carbon atoms in the alkyl groups, represents in each case optionally halogen-substituted alkenyl, alkinyl, alkenyloxy, alkenylthio or alkenylamino having in each case up to 6 carbon atoms in the alkenyl- or alkinyl groups, represents in each case optionally halogen-substituted cycloalkyl, cycloalkyloxy, cycloalkylthio, cycloalkylamino, cycloalkylalkyl, cycloalkylalkoxy, cycloalkylalkylthio or cycloalkylalkylamino having in each case 3 to 6 carbon atoms in the cycloalkyl groups and optionally up to 4 carbon atoms in the alkyl moiety, or represents in each case optionally halogen-, C₁-C₄-alkyl- or C₁-C₄-alkoxy-substituted phenyl, phenyloxy, phenylthio, phenylamino, benzyl, benzyloxy, benzylthio or benzylamino, or - if two adjacent radicals R⁵ and R⁵ are located at a double bond - together with the adjacent radical R⁵ also represents a benzo grouping, and
R⁶ represents hydrogen, hydroxyl, amino, alkylideneamino having up to 4 carbon atoms, represents in each case optionally halogen- or C₁-C₄-alkoxy-substituted alkyl, alkoxy, alkylamino, dialkylamino or alkanoylamino having in each case up to 6 carbon atoms in the alkyl groups, represents in each case optionally halogen-substituted alkenyl, alkinyl or alkenyloxy having in each case up to 6 carbon atoms in the alkenyl- or alkinyl groups, represents in each case optionally halogen-substituted cycloalkyl, cycloalkylalkyl or cycloalkylamino having in each case 3 to 6 carbon atoms in the cycloalkyl groups and optionally up to 3 carbon atoms in the alkyl moiety, or represents in each case optionally halogen-, C₁-C₄-alkyl- or C₁-C₄-alkoxy-substituted phenyl or benzyl, or together with an adjacent radical R⁵ or R⁶ represents optionally halogen- or C₁-C₄-alkyl-substituted alkanediyl having 3 to 5 carbon atoms,
where the individual radicals R⁵ and R⁶ - if a plurality of them are attached to the same heterocyclic grouping - can have identical or different meanings within the scope of the above definition,
- including all possible tautomeric forms and the sodium, potassium, magnesium, calcium, ammonium, C₁-C₄-alkyl-ammonium-, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulphonium, C₅- or C₆-cycloalkyl-ammonium and di-(C₁-C₂-alkyl)-benzyl-ammonium salts.

2. Compounds according to Claim 1, **characterized in that**
A represents methylene, ethylidene (ethane-1,1-diyl) or dimethylene (ethane-1,2-diyl),
R¹ represents hydrogen, represents in each case optionally cyano-, carboxyl-, carbamoyl-, fluorine-, chlorine-, methoxy-, ethoxy-, n- or i-propoxy-, methylthio-, ethylthio-, n- or i-propylthio-, methylsulphinyl-, ethylsulphinyl-, n- or i-propylsulphinyl-, methylsulphonyl-, ethylsulphonyl-, n- or i-propylsulphonyl-substituted methyl, ethyl, nor i-propyl, n-, i-, s- or t-butyl, or represents in each case optionally cyano-, carboxyl-, carbamoyl-, fluorine-, chlorine-, methyl-, ethyl-, methoxycarbonyl- or ethoxycarbonyl-substituted cyclopropyl, cyclobutyl, cyclopentyl or cyclohexyl,
R² represents hydrogen, cyano, carbamoyl, fluorine, chlorine, bromine, represents in each case optionally cyano-, carbamoyl-, fluorine-, chlorine-, methoxy- or ethoxy-substituted methyl, ethyl, n- or i-propyl, methoxy, ethoxy, n- or i-propoxy, methoxycarbonyl, ethoxycarbonyl, n- or i-propoxycarbonyl, or represents in each case optionally fluorine-and/or chlorine-substituted methylthio, ethylthio, n- or i-propylthio, methylsulphinyl, ethylsulphinyl, n- or i-propylsulphinyl, methylsulphonyl, ethylsulphonyl, n- or i-propylsulphonyl,
R³ represents hydrogen, nitro, cyano, carboxyl, carbamoyl, thiocarbamoyl, fluorine, chlorine, bromine, iodine, represents in each case optionally fluorine- and/or chlorine-, methoxy-, ethoxy-, n- or i-propoxy-, methylthio-, ethylthio-, n- or i-propylthio-, methylsulphinyl-, ethylsulphinyl-, methylsulphonyl- or ethylsulphonyl-substituted methyl, ethyl, n- or i-propyl, n-, i-, s- or t-butyl, represents in each case optionally fluorine- and/or chlorine-, methoxy-, ethoxy-, n- or i-propoxy-substituted methoxy, ethoxy, n- or i-propoxy, represents in each case optionally fluorine- and/or chlorine-substituted methylthio, ethylthio, n- or i-propylthio, methylsulphinyl, ethylsulphinyl, n- or i-propylsulphinyl, methylsulphonyl, ethylsulphonyl, nor i-propylsulphonyl, or represents methylamino, ethylamino, n- or i-propylamino, dimethylamino, diethylamino, dimethylaminosulphonyl or diethylaminosulphonyl,
R⁴ represents nitro, cyano, carboxyl, carbamoyl, thiocarbamoyl, fluorine, chlorine, bromine, represents in each case optionally fluorine- and/or chlorine-, methoxy-, ethoxy-, n- or i-propoxy-, methylthio-, ethylthio-, n- or i-propylthio-, methylsulphinyl-, ethylsulphinyl-, methylsulphonyl- or ethylsulphonyl-substituted methyl, ethyl, n- or i-propyl, n-, i-, s- or t-butyl, represents in each case optionally fluorine- and/or chlorine-, methoxy-, ethoxy-, n- or i-propoxy-substituted methoxy, ethoxy, n- or i-propoxy, represents in each case optionally fluorine-and/or chlorine-substituted methylthio, ethylthio, n- or i-propylthio, methylsulphinyl, ethylsulphinyl, n- or i-propylsulphinyl, methylsulphonyl, ethylsulphonyl, n- or i-propylsulphonyl, or represents methylamino, ethylamino, n- or i-propylamino, dimethylamino, diethylamino, dimethylaminosulphonyl or diethylaminosulphonyl,
R⁵ represents hydrogen, hydroxyl, mercapto, cyano, fluorine, chlorine, bromine, iodine, represents in each case optionally fluorine-, chlorine-, methoxy-, ethoxy-, n- or i-propoxy-, n-, i-, s- or t-butoxy-, methylthio-, ethylthio-, n- or i-propylthio-, n-, i-, s- or t-butylthio-, methylsulphinyl-, ethylsulphinyl-, n- or i-propylsulphinyl-, methylsulphonyl-, ethylsulphonyl-, n- or i-propylsulphonyl-substituted methyl, ethyl, nor i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, represents methylamino, ethylamino, n- or i-propylamino, n-, i-, s- or t-butylamino, dimethylamino, diethylamino, di-n-propylamino or di-i-propylamino, represents in each case optionally fluorine- and/or chlorine-substituted ethenyl, propenyl, butenyl, ethinyl, propinyl, butinyl, propenyloxy, butenyloxy, propenylthio, butenylthio, propenylamino or butenylamino, represents in each case optionally fluorine- and/or chlorine-substituted cyclopropyl, cyclobutyl, cyclopentyl, cyclohexyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopropylmethylthio, cyclobutylmethylthio, cyclopentylmethylthio, cyclohexylmethylthio, cyclopropylmethylamino, cyclobutylmethylamino, cyclopentylmethylamino or cyclohexylmethylamino, or represents in each case optionally fluorine-, chlorine-, methyl-, ethyl-, n- or i-propyl-, n-, i-, s- or t-butyl-, methoxy-, ethoxy-, n- or i-propoxy-substituted phenyl, phenyloxy, phenylthio, phenylamino, benzyl, benzyloxy, benzylthio or benzylamino, or - if two adjacent radicals R⁵ and R⁵ are located at a double bond - together with the adjacent radical R⁵ also represents a benzo grouping,
R⁶ represents hydrogen, hydroxyl, amino, represents in each case optionally fluorine- and/or chlorine-, methoxy- or ethoxy-substituted methyl, ethyl, n- or i-propyl, n-, i- or s-butyl, methoxy, ethoxy, n- or i-propoxy, methylamino, ethylamino or dimethylamino, represents in each case optionally fluorine- and/or chlorine-substituted ethenyl, propenyl, ethinyl, propinyl or propenyloxy, represents in each case optionally fluorine- and/or chlorine-substituted cyclopropyl, cyclobutyl, cyclopentyl, cyclohexyl, cyclopropylmethyl, cyclobutylmethyl, cyclopentylmethyl or cyclohexylmethyl, or represents in each case optionally fluorine-, chlorine-, methyl-, ethyl-, n- or i-propyl-, n-, i-, s-or t-butyl-, methoxy-, ethoxy-, n- or i-propoxy-substituted phenyl or benzyl, or together with an adjacent radical R⁵ or R⁶ represents in each case optionally methyl- and/or ethyl-substituted propane-1,3-diyl (trimethylene), butane-1,4-diyl (tetramethylene) or pentane-1,5-diyl (pentamethylene), and
Z represents one of the heterocyclic groupings below

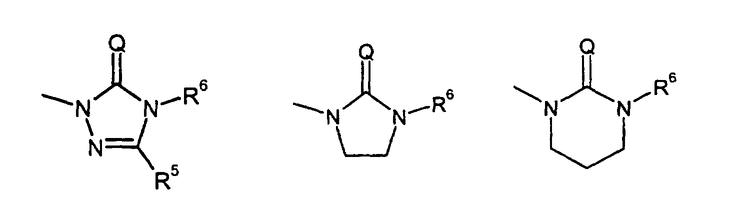

3. Compounds according to Claim 1 or 2, **characterized in that**
n represents 0 or 1,
A represents methylene,
R¹ represents in each case optionally cyano-, fluorine-, chlorine-, methoxy-, ethoxy-, n- or i-propoxy-, methylthio-, ethylthio-, n- or o-propylthio-, methylsulphinyl-, ethylsulphinyl-, n- or i-propylsulphinyl-, methylsulphonyl-, ethylsulphonyl-, n- or i-propylsulphonyl-substituted methyl, ethyl, n- or i-propyl, or represents in each case optionally cyano-, fluorine-, chlorine-, methylor ethyl-substituted cyclopropyl, cyclobutyl, cyclopentyl or cyclohexyl,
R² represents hydrogen, cyano, carbamoyl, fluorine, chlorine, bromine, represents in each case optionally cyano-, carbamoyl-, fluorine-, chlorine-, methoxy- or ethoxy-substituted methyl, ethyl, n- or i-propyl, or represents in each case optionally fluorine- and/or chlorine-substituted methylthio, ethylthio, n- or i-propylthio, methylsulphinyl, ethylsulphinyl, n- or i-propylsulphinyl, methylsulphonyl, ethylsulphonyl, n- or i-propylsulphonyl,
R³ represents hydrogen, nitro, cyano, carboxyl, carbamoyl, thiocarbamoyl, fluorine, chlorine, bromine, represents in each case optionally fluorine- and/or chlorine-, methoxy-, ethoxy-, n- or i-propoxy-, methylthio-, ethylthio-, n- or i-propylthio-, methylsulphinyl-, ethylsulphinyl-, methylsulphonyl- or ethylsulphonyl-substituted methyl, ethyl, n- or i-propyl, n-, i-, s- or t-butyl, represents in each case optionally fluorine- and/or chlorine-, methoxy-, ethoxy-, n- or i-propoxy-substituted methoxy, ethoxy, n- or i-propoxy, represents in each case optionally fluorine- and/or chlorine-substituted methylthio, ethylthio, n- or i-propylthio, methylsulphinyl, ethylsulphinyl, n- or i-propylsulphinyl, methylsulphonyl, ethylsulphonyl, nor i-propylsulphonyl, or represents methylamino, ethylamino, n- or i-propylamino, dimethylamino, diethylamino, dimethylaminosulphonyl or diethylaminosulphonyl,
R⁴ represents methylsulphonyl, chlorine, methoxy, nitro, trifluoromethyl or methyl,
R⁵ represents hydrogen, hydroxyl, chlorine, bromine, methyl, ethyl, n- or i-propyl, n-, i-, s- or t-butyl, difluoromethyl, dichloromethyl, trifluoromethyl, trichloromethyl, chlorodifluoromethyl, fluorodichloromethyl, fluoroethyl, chloroethyl, difluoroethyl, dichloroethyl, fluoro-n-propyl, fluoro-i-propyl, chloro-n-propyl, chloro-i-propyl, methoxymethyl, ethoxymethyl, methoxyethyl, ethoxyethyl, methoxy, ethoxy, n- or i-propoxy, n-, i-, s- or t-butoxy, fluoroethoxy, chloroethoxy, difluoroethoxy, dichloroethoxy, trifluoroethoxy, trichloroethoxy, chlorofluoroethoxy, chlorodifluoroethoxy, fluorodichloroethoxy, methylthio, ethylthio, n- or i-propylthio, fluoroethylthio, chloroethylthio, difluoroethylthio, dichloroethylthio, chlorofluoroethylthio, chlorodifluoroethylthio, fluorodichloroethylthio, methylsulphinyl, ethylsulphinyl, n- or i-propylsulphinyl, methylsulphonyl, ethylsulphonyl, n- or i-propylsulphonyl, methylamino, dimethylamino, propenylthio, butenylthio, propinylthio, butinylthio, cyclopropyl, cyclopropylmethyl, cyclopropylmethoxy, phenyl or phenoxy, and
R⁶ represents amino, methyl, ethyl, n- or i-propyl, n-, i-, s- or t-butyl, methoxy, ethoxy, methylamino, dimethylamino, cyclopropyl or cyclopropylmethyl, or together with R⁵ represents propane-1,3-diyl (trimethylene), butane-1,4-diyl (tetramethylene) or pentane-1,5-diyl (pentamethylene).

4. Compounds according to any of Claims 1 to 3, **characterized in that**
R³ represents hydrogen, nitro, cyano, fluorine, chlorine, bromine, methyl, trifluoromethyl or methoxy,
R⁵ represents hydrogen, bromine, chlorine, methyl, ethyl, trifluoromethyl, cyclopropyl, difluoroethyl, methylthio, ethylthio, methoxy, ethoxy, nor i-propoxy, trifluoroethoxy, methylamino or dimethylamino and
R⁶ represents hydrogen, amino, methyl, ethyl, methoxy, ethoxy, cyclopropyl or dimethylamino.

5. Compounds according to any of Claims 1 to 4, **characterized in that**
Q represents oxygen (O).

6. Compounds according to any of Claims 1 to 5, **characterized in that**
Z represents the heterocyclic grouping

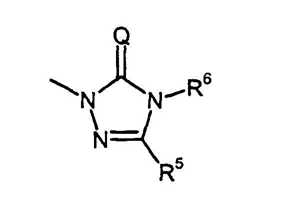

7. Compounds according to any of Claims 1 to 6, **characterized in that**
R¹ represents cyclopropyl.

8. Compounds according to any of Claims 1 to 7, **characterized in that**
R² represents hydrogen or cyano.

9. Compounds according to any of Claims 1 to 8, **characterized in that**
R⁵ represents bromine, methyl, ethyl, methoxy, methylthio, ethoxy, methylsulphonyl or dimethylamino, and
^{R6} represents amino, methyl, ethyl, cyclopropyl, dimethylamino, methoxy or ethoxy.

10. Compounds of the formula (IA) 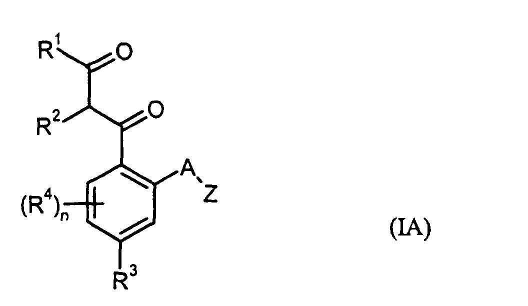 in which
n, A, R¹, R², R³, R⁴ and Z are each as defined in any of Claims 1 to 4 and 6 to 8.

11. Compounds of the formula (IB), 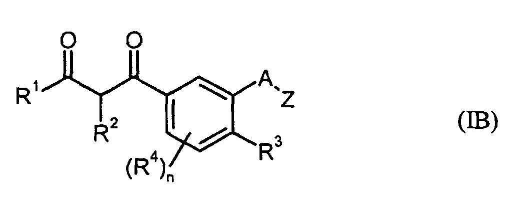 in which
n, A, R¹, R², R³, R⁴ and Z are each as defined in any of Claims 1 to 4 and 6 to 8.

12. Compounds of the formula (IC), 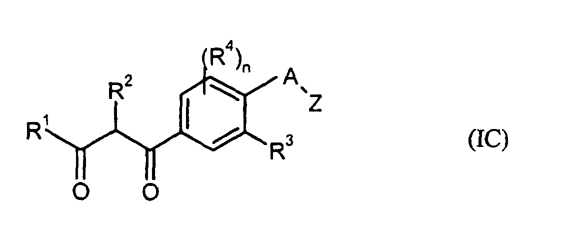 in which
n, A, R¹, R², R³, R⁴ and Z are each as defined in any of claims 1 to 4 and 6 to 8.

13. Process for preparing compounds according to any of Claims 1 to 12,
**characterized in that**
(a) ketones of the general formula (II) 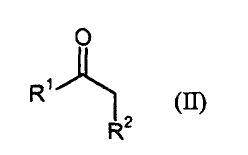
in which
R¹ and R² are each as defined in any of Claims 1 to 3 and 7 and 8, are reacted with substituted benzoic acids of the general formula (III) 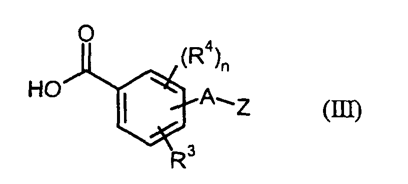 in which
n, A, R³, R⁴ and Z are each as defined in any of Claims 1 to 4 and 6,
if appropriate in the presence of one or more reaction auxiliaries and if appropriate in the presence of one or more diluents,
and electrophilic or nucleophilic substitutions and/or oxidations or reductions within the scope of the definition of the substituents are, if appropriate, subsequently, i.e. after the processes (a) according to the invention have been carried out, carried out in a customary manner on the resulting compounds of the formula (I), or the compounds of the formula (I) are converted in a customary manner into salts.

14. Compounds of the formula (III), 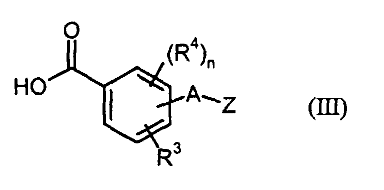 in which
n, A, R³, R⁴ and Z are each as defined in any of Claims 1 to 4 and 6,
except for 2-(5-carboxyl-2,4-dichlorophenyl)-4-difluoromethyl-5-methyl-2,4-dihydro-3H-1,2,4-triazol-3-one and 2-(5-carboxyl-2,4-dichlorophenyl)-4,5-dimethyl-2,4-dihydro-3H-1,2,4-triazol-3-one.

15. Herbicidal compositions, **characterized in that in that** they comprise at least one compound according to any of claims 1 to 12 and customary extenders.

16. Use of at least one compound according to any of claims 1 to 12 for controlling undesirable plants.

## Revendications

1. Benzoylcétones substituées de formule (I), 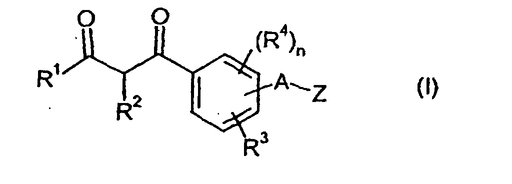 dans laquelle
n a la valeur 0, 1 ou 2,
A est un reste alcanediyle (alkylène) ayant 1 à 4 atomes de carbone,
R¹ représente l'hydrogène, un reste alkyle ayant 1 à 6 atomes de carbone, éventuellement substitué par un radical cyano, carboxy, carbamoyle, halogèno, alkoxy en C₁ à C₄, alkylthio en C₁ à C₄, alkylsulfinyle en C₁ à C₄ ou alkylsulfonyle en C₁ à C₄, ou bien un reste cycloalkyle ayant 3 à 6 atomes de carbone éventuellement substitué par un radical cyano, carboxy, carbamoyle, halogéno, alkyle en C₁ à C₄ ou (alkoxy en C₁ à C₄)-carbonyle,
R² représente l'hydrogène, un groupe cyano, carbamoyle, un halogène, un reste alkyle, alkoxy ou alkoxycarbonyle ayant chacun jusqu'à 6 atomes de carbone et chacun étant éventuellement substitué par un radical cyano, carbamoyle, halogèno ou alkoxy en C₁ à C₄, ou bien un reste alkylthio, alkylsulfinyle ou alkylsulfonyle ayant chacun 1 à 6 atomes de carbone et chacun étant éventuellement substitué par un halogène,
R³ représente l'hydrogène, un groupe nitro, cyano, carboxy, carbamoyle, thiocarbamoyle, un halogène, un reste alkyle, alkoxy, alkylthio, alkylsulfinyle ou alkylsulfonyle ayant chacun jusqu'à 4 atomes de carbone dans les groupes alkyle et chacun étant éventuellement substitué par un radical halogéno, alkoxy en C₁ à C₄, alkylthio en C₁ à C₄, alkylsulfinyle en C₁ à C₄ ou alkylsulfonyle en C₁ à C₄, ou bien un reste alkylamino, dialkylamino ou dialkylaminosulfonyle ayant chacun jusqu'à 4 atomes de carbone dans les groupes alkyle,
R⁴ est un groupe nitro, cyano, carboxy, carbamoyle, thiocarbamoyle, un halogène, un reste alkyle, alkoxy, alkylthio, alkylsulfinyle ou alkylsulfonyle ayant chacun jusqu'à 4 atomes de carbone dans les groupes alkyle et chacun étant éventuellement substitué par un radical halogèno, alkoxy en C₁ à C₄, alkylthio en C₁ à C₄, alkylsulfinyle en C₁ à C₄ ou alkylsulfonyle en C₁ à C₄, ou bien un reste alkylamino, dialkylamino ou dialkylaminosulfonyle ayant chacun jusqu'à 6 atomes de carbone dans les groupes alkyle, et
Z représente l'un des groupements hétérocycliques suivants : 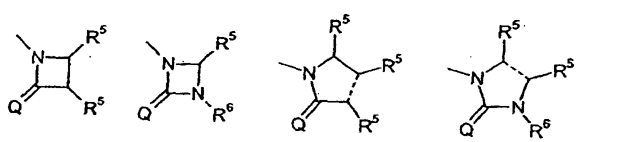 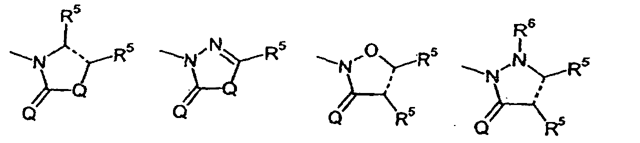 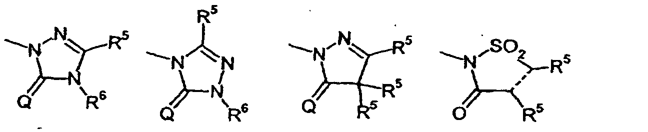 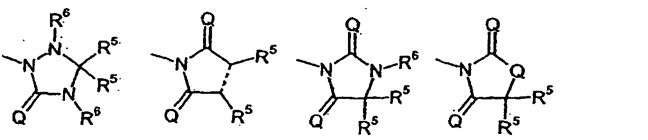 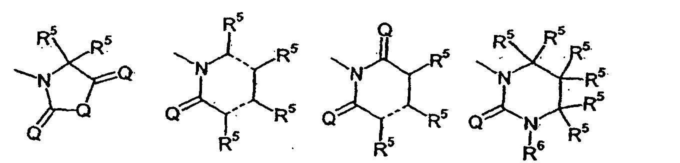 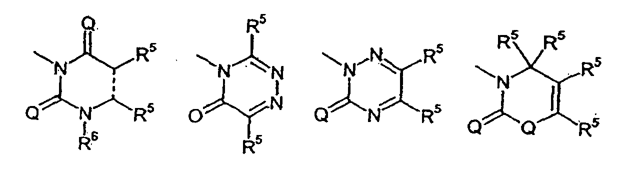 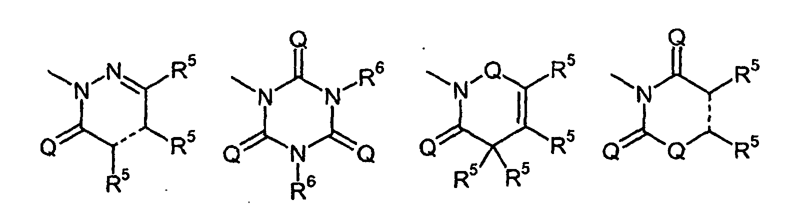 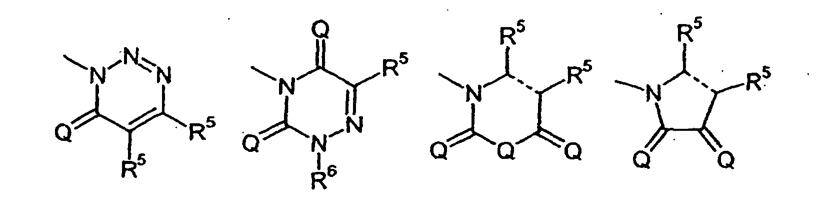 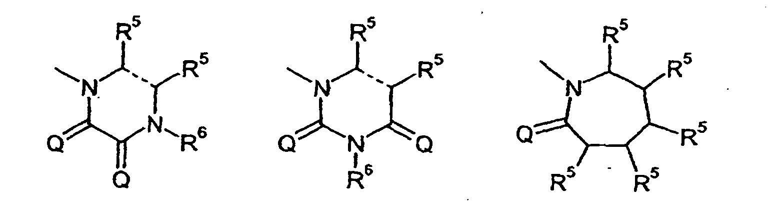 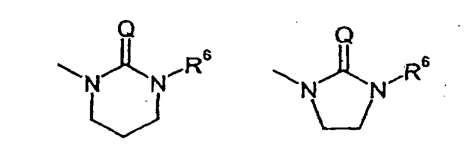 où la liaison en traits interrompus représente dans chaque cas une liaison simple ou une double liaison, et chaque groupement hétérocyclique ne porte, avantageusement, que deux substituants ayant la définition de R⁵ et/R⁶,
Q représente l'oxygène ou le soufre,
R⁵ représente l'hydrogène, un groupe hydroxy, mercapto, cyano, un halogène, un reste alkyle, alkylcarbonyle, alkoxy, alkoxycarbonyle, alkylthio, alkylsulfinyle ou alkylsulfonyle ayant chacun jusqu'à six atomes de carbone dans les groupes alkyle et chacun étant éventuellement substitué par un radical cyano, halogèno, alkoxy en C₁ à C₄, alkylthio en C₁ à C₄, alkylsulfinyle en C₁ à C₄ ou alkylsulfonyle en C₁ à C₄, un reste alkylamino ou dialkylamino ayant chacun jusqu'à 6 atomes de carbone dans les groupes alkyle et chacun étant éventuellement substitué par un halogène, un reste alcényle, alcynyle, alcényloxy, alcénylthio ou alcénylamino ayant chacun jusqu'à 6 atomes de carbone dans les groupes alcényle ou alcynyle et chacun étant éventuellement substitué par l'halogène, un reste cycloalkyle, cycloalkyloxy, cycloalkylthio, cycloalkylamino, cycloalkylalkyle, cycloalkylalkoxy, cycloalkylalkylthio ou cycloalkylalkylamino ayant chacun 3 à 6 atomes de carbone dans les groupes cycloalkyle et, le cas échéant, jusqu'à 4 atomes de carbone dans la partie alkyle et chacun étant éventuellement substitué par un halogène, ou bien un reste phényle, phényloxy, phénylthio, phénylamino, benzyle, benzyloxy, benzylthio ou benzylamino, chacun éventuellement substitué par un radical halogèno, alkyle en C₁ à C₄ ou alkoxy en C₁ à C₄, ou forme aussi un groupement benzo - au cas où deux restes R⁵ et R⁵ voisins se trouvent sur une double liaison - conjointement avec le reste R⁵ voisin, et
R⁶ représente l'hydrogène, un groupe hydroxy, amino, un reste alkylydénamino ayant jusqu'à 4 atomes de carbone, un reste alkyle, alkoxy, alkylamino, dialkylamino ou alcanoylamino ayant chacun jusqu'à 6 atomes de carbone dans les groupes alkyle et chacun étant éventuellement substitué par un radical halogéno ou alkoxy en C₁ à C₄, un reste alcényle, alcynyle ou alcényloxy ayant chacun jusqu'à 6 atomes de carbone dans les groupes alcényle ou alcynyle et chacun étant éventuellement substitué par un halogène, un reste cycloalkyle, cycloalkylalkyle ou cycloalkylamino ayant chacun 3 à 6 atomes de carbone dans les groupes cycloalkyle et, le cas échéant, jusqu'à 3 atomes de carbone dans la partie alkyle, chacun éventuellement substitué par un halogène, ou bien un reste phényle ou benzyle, chacun éventuellement substitué par un halogène, ou forme avec un reste R⁵ ou R⁶ voisin un reste alcanediyle de 3 à 5 atomes de carbone éventuellement substitué par un radical halogéno ou alkyle en C₁ à C₄,
les restes individuels R⁵ et R⁶ - dans la mesure où plusieurs de ces restes sont liés à de mêmes groupements hétérocycliques, pouvant avoir la même définition ou des définitions différentes dans le cadre des définitions données ci-dessus,
- y compris toutes les formes tautomères possibles et les sels de sodium, de potassium, de magnésium, de calcium, d'ammonium, de (alkyle en C₁ à C₄)-ammonium de di- (alkyle en C₁ à C₄)-ammonium, de tri-(alkyle en C₁ à C₄)-ammonium, de tétra-(alkyle en C₁ à C₄) - ammonium, de tri-(alkyle en C₁ à C₄)-sulfonium, de (cycloalkyle en C₅ ou C₆)-ammonium et de di-(alkyle en C₁ ou C₂)-benzylammonium.

2. Composés suivant la revendication 1, **caractérisés en ce que**
A représente un reste méthylène, éthylidène (éthane-1,1-diyle) ou diméthylène (éthane-1,2-diyle),
R¹ représente l'hydrogène, un reste méthyle, éthyle, n-propyle, isopropyle, n-butyle, isobutyle, sec.-butyle ou tertiobutyle, chacun éventuellement substitué par un radical cyano, carboxy, carbamoyle, fluoro, chloro, méthoxy, éthoxy, n-propoxy, isopropoxy, méthylthio, éthylthio, n-propylthio, isopropylthio, méthylsulfinyle, éthysulfinyle, n-propylsulfinyle, isopropylsulfinyle, méthylsulfonyle, éthylsulfonyle, n-propylsulfonyle ou isopropylsulfonyle, ou un reste cyclopropyle, cyclobutyle, cyclopentyle ou cyclohexyle, chacun éventuellement substitué par un radical cyano, carboxy, carbamoyle, fluoro, chloro, méthyle, éthyle, méthoxycarbonyle ou éthoxycarbonyle,
R² représente l'hydrogène, un groupe cyano, carbamoyle, le fluor, le chlore, le brome, un reste méthyle, éthyle, n-propyle, isopropyle, méthoxy, éthoxy, n-propoxy, isopropoxy, méthoxycarbonyle, éthoxycarbonyle, n-propoxycarbonyle ou isopropoxycarbonyle, chacun éventuellement substitué par un radical cyano, carbamoyle, fluoro, chloro, méthoxy ou éthoxy, ou bien un reste méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle ou isopropylsulfonyle, chacun éventuellement substitué par du fluor et/ou du chlore,
R³ représente l'hydrogène, un groupe nitro, cyano, carboxy, carbamoyle, thiocarbamoyle, le fluor, le chlore, le brome, l'iode, un reste méthyle, éthyle, n-propyle, isopropyle, n-butyle, isobutyle, sec.-butyle ou tertiobutyle, chacun éventuellement substitué par un radical fluoro et/ou chloro, méthoxy, éthoxy, n-propoxy, isopropoxy, méthylthio, éthylthio, n-propylthio, isopropylthio, méthylsulfinyle, éthylsulfinyle, méthylsulfonyle ou éthylsulfonyle, un reste méthylthio, éthylthio, n-propylthio, isopropylthio, méthylsulfinyle, éthylsulfinyle, n-propylsulfonyle, isopropylsulfinyle, méthylsulfonyle, éthylsulfonyle, n-propylsulfonyle, isopropylsulfonyle, chacun éventuellement substitué par du fluor et/ou du chlore, ou bien un reste méthylamino, éthylamino, n-propylamino, isopropylamino, diméthylamino, diéthylamino, diméthylaminosulfonyle ou diéthylaminosulfonyle,
R⁴ est un groupe nitro, cyano, carboxy, carbamoyle, thiocarbamoyle, le fluor, le chlore, le brome, un reste méthyle, éthyle, n-propyle, isopropyle, n-butyle, isobutyle, sec.-butyle ou tertiobutyle, chacun éventuellement substitué par du fluor et/ou du chlore ou un radical méthoxy, éthoxy, n-propoxy, isopropoxy, méthylthio, éthylthio, n-propylthio, isopropylthio, méthylsulfinyle, éthylsulfinyle, méthylsulfonyle ou éthylsulfonyle, un reste méthoxy, éthoxy, n-propoxy ou isopropoxy, chacun éventuellement substitué par du fluor et/ou du chlore, un radical méthoxy, éthoxy, n-propoxy ou isopropoxy, un reste méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle ou isopropylsulfonyle, chacun éventuellement substitué par du fluor et/ou du chlore, ou bien un reste méthylamino, éthylamino, n-propylamino, isopropylamino, diméthylamino, diéthylamino, diméthylaminosulfonyle ou diéthylaminosulfonyle,
R⁵ représente l'hydrogène, un groupe hydroxy, mercapto, cyano, le fluor, le chlore, le brome, l'iode, un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, méthylsulfinyle, éthylsulfinyle, n-propylsulfinyle, isopropylsulfinyle, méthylsulfonyle, éthylsulfonyle, n-propylsulfonyle ou isopropylsulfonyle, chacun éventuellement substitué par un radical fluoro, chloro, méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, méthylsulfinyle, éthylsulfinyle, n-propylsulfinyle, isopropylsulfinyle, méthylsulfonyle, éthylsulfonyle, n-propylsulfonyle ou isopropylsulfonyle, un groupe méthylamino, éthylamino, n-propylamino, isopropylamino, n-butylamino, isobutylamino, sec.-butylamino, tertiobutylamino, diméthylamino, diéthylamino, di-n-propylamino ou di-isopropylamino, un reste éthényle, propényle, butényle, éthynyle, propynyle, butynyle, propényloxy, butényloxy, propénylthio, buténylthio, propénylamino ou buténylamino, chacun éventuellement substitué par du fluor et/ou du chlore, un reste cyclopropyle, cyclobutyle, cyclopentyle, cyclohexyle, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino, cyclohexylamino, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopropylméthylthio, cyclobutylméthylthio, cyclopentylméthylthio, cyclohexylméthylthio, cyclopropylméthylamino, cyclobutylméthylamino, cyclopentylméthylamino ou cyclohexylméthylamino, chacun éventuellement substitué par du fluor et/ou du chlore, ou un reste phényle, phényloxy, phénylthio, phénylamino, benzyle, benzyloxy, benzylthio ou benzylamino, chacun éventuellement substitué par un radical fluoro, chloro, méthyle, éthyle, n-propyle, isopropyle, n-butyle, isobutyle, sec.-butyle, tertiobutyle, méthoxy, éthoxy, n-propoxy ou isopropoxy, ou bien forme aussi un groupement benzo - au cas où deux restes R⁵ et R⁵ voisins se trouvent sur une double liaison - conjointement avec le reste R⁵ voisin,
R⁶ représente l'hydrogène, un groupe hydroxy, amino, un reste méthyle, éthyle, n-propyle, isopropyle, n-butyle, isobutyle, sec.-butyle, méthoxy, éthoxy, n-propoxy, isopropoxy, méthylamino, éthylamino ou diméthylamino, chacun éventuellement substitué par du fluor et/ou du chlore, un radical méthoxy ou éthoxy, un reste éthényle, propényle, éthynyle, propynyle ou propényloxy, chacun éventuellement substitué par du fluor et/ou du chlore, un reste cyclopropyle, cyclobutyle, cyclopentyle, cyclohexyle, cyclopropylméthyle, cyclobutylméthyle, cyclopentylméthyle ou cyclohexylméthyle, chacun éventuellement substitué par du fluor et/ou du chlore, ou un reste phényle ou benzyle, chacun éventuellement substitué par un radical fluoro, chloro, méthyle, éthyle, n-propyle, isopropyle, n-butyle, isobutyle, sec.-butyle, tertiobutyle, méthoxy, éthoxy, n-propoxy ou isopropoxy, ou forme conjointement avec un reste R⁵ ou R⁶ voisin un reste propane-1,3-diyle (triméthylène), butane-1,4-diyle (tétraméthylène) ou pentane-1,5-diyle (pentaméthylène) chacun éventuellement substitué par un radical méthyle et/ou éthyle, et
Z représente l'un des groupements hétérocycliques suivants :

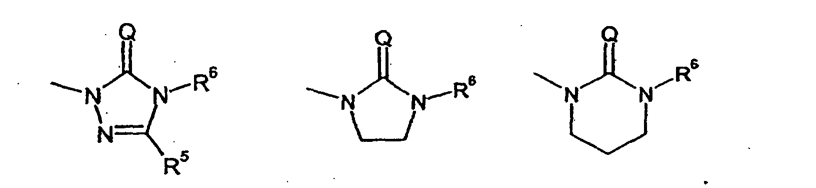

3. Composés suivant l'une des revendications 1. ou 3, **caractérisés en ce que**
n a la valeur 0 ou 1,
A est un groupe méthylène,
R¹ est un reste méthyle, éthyle, n-propyle, isopropyle, chacun éventuellement substitué par un radical cyano, fluoro, chloro, méthoxy, éthoxy, n-propoxy, isopropoxy, méthylthio, éthylthio, n-propylthio, o-propylthio, méthylsulfinyle, éthylsulfinyle, n-propylsulfinyle, isopropylsulfinyle, méthylsulfonyle, éthylsulfonyle, n-propylsulfonyle ou isopropylsulfonyle, un reste cyclopropyle, cyclobutyle, cyclopentyle ou cyclohexyle, chacun éventuellement substitué par un radical cyano, fluoro, chloro, méthyle ou éthyle,
R² représente l'hydrogène, un groupe cyano, carbamoyle, le fluor, le chlore, le brome, un reste méthyle, éthyle, n-propyle ou isopropyle, chacun éventuellement substitué par un radical cyano, carbamoyle, fluoro, chloro, méthoxy ou éthoxy, ou bien un reste méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle ou isopropylsulfonyle, chacun éventuellement substitué par un radical fluoro et/ou chloro,
R³ représente l'hydrogène, un groupe nitro, cyano, carboxy, carbamoyle, thiocarbamoyle, le fluor, le chlore, le brome, un reste méthyle, éthyle, n-propyle, isopropyle, n-butyle, isobutyle, sec.-butyle ou tertiobutyle, chacun éventuellement substitué par un radical fluoro et/ou chloro, méthoxy, éthoxy, n-propoxy, isopropoxy, méthylthio, éthylthio, n-propylthio ou isopropylthio, un reste méthoxy, éthoxy, n-propoxy ou isopropoxy chacun éventuellement substitué par un radical fluoro et/ou chloro, méthoxy, éthoxy, n-propoxy ou isopropoxy, un reste méthylthio, éthylthio, n-propylthio, isopropylthio, méthylsulfinyle, éthylsufinyle, n-propylsulfinyle, isopropopylsulfinyle, méthylsulfonyle, éthylsulfonyle, n-propylsulfonyle, isopropylsulfonyle, chacun éventuellement substitué par du fluor et/ou du chlore, ou bien un reste méthylamino, éthylamino, n-propylamino, isopropylamino, diméthylamino, diéthylamino, diméthylaminosulfonyle ou diéthylaminosulfonyle,
R⁴ est un reste méthylsulfonyle, chloro, méthoxy, nitro, trifluorométhyle ou méthyle,
R⁵ représente l'hydrogène, un groupe hydroxy, le chlore, le brome, un reste méthyle, éthyle, n-propyle, isopropyle, n-butyle, isobutyle, sec.-butyle, tertiobutyle, difluorométhyle, dichlorométhyle, trifluorométhyle, trichlorométhyle, chlorodifluorométhyle, fluorodichlorométhyle, fluoréthyle, chloréthyle, difluoréthyle, dichloréthyle, fluoro-n-propyle, fluorisopropyle, chloro-n-propyle, chlorisopropyle, méthoxyméthyle, éthoxyméthyle, méthoxyéthyle, éthoxyéthyle, méthoxy, éthoxy, n-propoxy, isopropoxy, n-butoxy, isobutoxy, sec.-butoxy, tertiobutoxy, fluoréthoxy, chloréthoxy, difluoréthoxy, dichloréthoxy, trifluoréthoxy, trichloréthoxy, chlorofluoréthoxy, chlorodifluoréthoxy, fluorodichloréthoxy, méthylthio, éthylthio, n-propylthio, isapropylthio, fluoréthylthio, chloréthylthio, difluoréthylthio, dichloréthylthio, chlorofluoréthylthio, chlorodifluoréthylthio, fluorodichloréthylthio, méthylsulfinyle, éthylsulfinyle, n-propylsulfinyle, isopropylsulfinyle, méthylsulfonyle, éthylsulfonyle, n-propylsulfonyle, isopropylsulfonyle, méthylamino, diméthylamino, propénylthio, buténylthio, propinylthio, butinylthio, cyclopropyle, cyclopropylméthyle, cyclopropylméthoxy, phényle ou phénoxy, et
R⁶ est un groupe amino, un reste méthyle, éthyle, n-propyle, isopropyle, n-butyle, isobutyle, sec.-butyle, tertiobutyle, méthoxy, éthoxy, méthylamino, diméthylamino, cyclopropyle ou cyclopropylméthyle, ou forme conjointement avec R⁵ un reste propane-1,3-diyle (triméthylène), butane-1,4-diyle (tétraméthylène) ou pentane-1,5-diyle (pentaméthylène).

4. Composés suivant l'une des revendications 1, 3 et 4, **caractérisés en ce que**
R³ représente l'hydrogène, un groupe nitro, cyano, le fluor, le chlore, le brome, un reste méthyle, trifluorométhyle ou méthoxy,
R⁵ représente l'hydrogène, le brome, le chlore, un reste méthyle, éthyle, trifluorométhyle, cyclopropyle, difluoréthyle, méthylthio, éthylthio, méthoxy, éthoxy, n-propoxy, isopropoxy, trifluoréthoxy, méthylamino ou diméthylamino, et
R⁶ représente l'hydrogène, un groupe amino, un reste méthyle, éthyle, méthoxy, éthoxy, cyclopropyle ou diméthylamino.

5. Composés suivant l'une des revendications 1, 3 à 5, **caractérisés en ce que**
Q représente l'oxygène (O).

6. Composés suivant l'une des revendications 1, 3 à 6, **caractérisés en ce que**
Z représente le groupement hétérocyclique

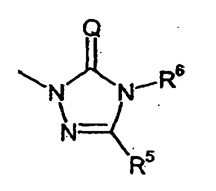

7. Composés suivant l'une des revendications 1, 3 à 7, **caractérisés en ce que**
R¹ est un reste cyclopropyle.

8. Composés suivant l'une des revendications 1, 3 à 8, **caractérisés en ce que**
R² représente l'hydrogène ou le groupe cyano.

9. Composés suivant l'une des revendications 1, 3 à 9, **caractérisés en ce que**
R⁵ représente le brome, un reste méthyle, éthyle, méthoxy, méthylthio, éthoxy, méthylsulfonyle ou diméthylamino et
R⁶ est un groupe amino, un reste méthyle, éthyle, cyclopropyle, diméthylamino, méthoxy ou éthoxy.

10. Composés de formule (IA), 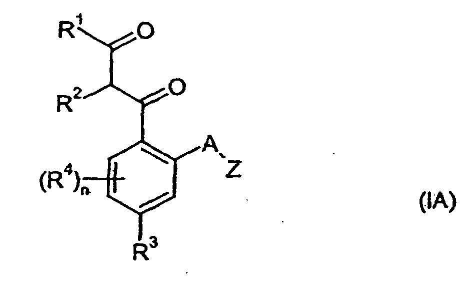 dans laquelle
n, A, R¹, R², R³, R⁴ et Z ont les définitions indiquées dans l'une des revendications 1 à 5 et 7 à 9.

11. Composés de formule (IB), 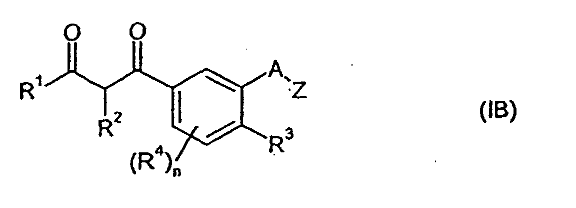 dans laquelle
n, A, R¹, R², R³, R⁴ et Z ont les définitions données dans l'une des revendications 1 à 5 et 7 à 9.

12. Composés de formule (IC), 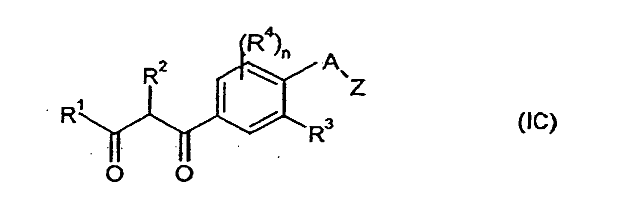 dans laquelle
n, A, R¹, R², R³, R⁴ et Z ont les définitions données dans l'une des revendications 1 à 5 et 7 à 9.

13. Procédé de production de composés suivant l'une des revendications 1 à 13, **caractérisé en ce que** :
(a) on fait réagir des cétones de formule générale (II) 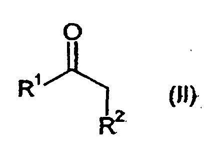
dans laquelle
R¹ et R² ont la définition indiquée dans l'une des revendications 1 à 4 ainsi que 8 et 9, avec des acides benzoïques substitués de formule générale (III) 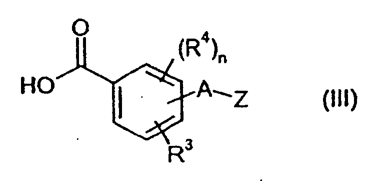 dans laquelle
n, A, R³, R⁴ et Z ont la définition indiquée dans l'une des revendications 1 à 5 ainsi que 7,
le cas échéant en présence d'un ou de plusieurs auxiliaires de réaction et en présence éventuelle d'un ou de plusieurs diluants,
après quoi, le cas échéant, c'est-à-dire après la conduite du procédé (a) conforme à l'invention, on conduit de manière classique des réactions électrophiles ou nucléophiles ou réactions d'oxydation ou de réduction sur les composés ainsi obtenus de formule (I) dans le cadre de la définition des substituants ou bien on transforme les composés de formule (I) en sels de manière usuelle.

14. Composés de formule (III), 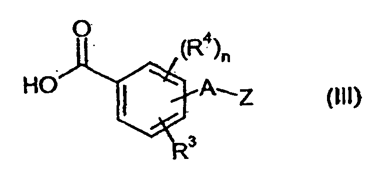 dans laquelle
n, A, R³, R⁴ et Z ont la définition indiquée dans l'une des revendications 1 à 5 ainsi que 7, à l'exception de la 2-(5-carboxy-2,4-dichlorophényl)-4-difluorométhyl-5-méthyl-2,4-dihydro-3H-1,2,4-triazole-3-one et de la 2-(5-carboxy-2,4-dichlorophényl)-4,5-diméthyl-2,4-dihydro-3H-1,2,4-triazole-3-one.

15. Compositions herbicides, **caractérisées en ce qu'**elles contiennent au moins un composé suivant l'une des revendications 1 à 13 et des diluants usuels.

16. Utilisation d'au moins un composé suivant l'une des revendications 1 à 13 pour combattre des plantes indésirables.
